(19) Europäisches Patentamt
European Patent Office
Office européen des brevets

(11) **EP 2 323 760 B1**

(12) **EUROPÄISCHE PATENTSCHRIFT**

(45) Veröffentlichungstag und Bekanntmachung des Hinweises auf die Patenterteilung:
**22.04.2020   Patentblatt 2020/17**

(21) Anmeldenummer: **09782500.4**

(22) Anmeldetag: **02.09.2009**

(51) Int Cl.:
*B01J 23/888* (2006.01)   *B01J 23/00* (2006.01)
*B01J 35/02* (2006.01)   *B01J 35/00* (2006.01)
*B01J 37/04* (2006.01)   *C07C 45/35* (2006.01)
*C07C 45/47* (2006.01)   *C07C 51/25* (2006.01)

(86) Internationale Anmeldenummer:
**PCT/EP2009/061326**

(87) Internationale Veröffentlichungsnummer:
**WO 2010/028991 (18.03.2010 Gazette 2010/11)**

(54) **VERFAHREN ZUR HERSTELLUNG VON GEOMETRISCHEN KATALYSATORFORMKÖRPERN**

METHOD FOR PRODUCING GEOMETRIC CATALYST MOULDED BODIES

PROCÉDÉ DE FABRICATION DE CORPS MOULÉS DE CATALYSEUR GÉOMÉTRIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **12.09.2008   DE 102008042061**
**12.09.2008   US 96553 P**

(43) Veröffentlichungstag der Anmeldung:
**25.05.2011   Patentblatt 2011/21**

(73) Patentinhaber: **BASF SE**
**67056 Ludwigshafen am Rhein (DE)**

(72) Erfinder:
• **RAICHLE, Andreas**
**01109 Dresden (DE)**
• **HORSTMANN, Catharina**
**67056 Ludwigshafen (DE)**
• **ROSOWSKI, Frank**
**68239 Mannheim (DE)**
• **MÜLLER-ENGEL, Klaus Joachim**
**76297 Stutensee (DE)**
• **PETZOLDT, Jochen**
**67273 Weisenheim am Berg (DE)**
• **CREMER, Ulrich**
**68161 Mannheim (DE)**

(74) Vertreter: **BASF IP Association**
**BASF SE**
**G-FLP-C006**
**67056 Ludwigshafen (DE)**

(56) Entgegenhaltungen:
**EP-A2- 1 005 908       WO-A1-2005/049200**
**WO-A2-02/49757       DE-A1-102008 042 060**

**Beschreibung**

Vorliegende Erfindung betrifft ein Verfahren zur Herstellung von geometrischen Katalysatorformkörpern $K^S$, die als Aktivmasse ein Multielementoxid $I^S$ der allgemeinen Stöchiometrie $I^S$,

$$[Bi_a Z^1_b O_x]_p [Mo_{12} Fe_d Z^2_e Z^3_f Z^4_g Z^5_h Z^6_i O_y]_1 \qquad (I^S),$$

mit

$Z^1$ = Wolfram oder Wolfram und Molybdän, mit der Maßgabe, dass wenigstens 10 mol-% der molaren Gesamtmenge an $Z^1$ Wolfram ist,

$Z^2$ = ein Element oder mehr als ein Element aus der Gruppe bestehend aus Nickel und Kobalt,

$Z^3$ = ein Element oder mehr als ein Element aus der Gruppe bestehend aus den Alkalimetallen, den Erdalkalimetallen und Thallium,

$Z^4$ = ein Element oder mehr als ein Element aus der Gruppe bestehend aus Zink, Phosphor, Arsen, Bor, Antimon, Zinn, Cer, Vanadium, Chrom und Wismut,

$Z^5$ = ein Element oder mehr als ein Element aus der Gruppe bestehend aus Silizium, Aluminium, Titan, Wolfram und Zirkonium,

$Z^6$ = ein Element oder mehr als ein Element aus der Gruppe bestehend aus Kupfer, Silber und Gold,

a = 0,1 bis 3,
b = 0,1 bis 10,
d = 0,01 bis 5,
e = 1 bis 10,
f = 0,01 bis 2,
g = 0 bis 5,
h = 0 bis 10,
i = 0 bis 1,
p = 0,05 bis 6, und
x, y = Zahlen, die durch die Wertigkeit und Häufigkeit der von Sauerstoff verschiedenen Elemente in $I^S$ bestimmt werden,

enthalten, bei dem man

- ein feinteiliges Mischoxid $Bi_a Z^1_b O_x$ mit einem Partikeldurchmesser $d_{50}^{A1}$ als Ausgangsmasse A1 mit der Maßgabe vorbildet, dass $1\ \mu m \leq d_{50}^{A1} \leq 100\ \mu m$ erfüllt ist;

- unter Verwendung von Quellen der von Sauerstoff verschiedenen Elemente des Anteils $T^S = [Mo_{12} Fe_d Z^2_e Z^3_f Z^4_g Z^5_h Z^6_j O_y]_1$ des Multielementoxids $I^S$ in wässrigem Medium mit der Maßgabe eine innige wässrige Mischung M erzeugt, dass

  - jede der verwendeten Quellen im Verlauf der Herstellung der wässrigen Mischung M einen Zerteilungsgrad Q durchläuft, der dadurch gekennzeichnet ist, dass sein Durchmesser $d_{90}^Q \leq 5\ \mu m$ µm beträgt, und

  - die wässrige Mischung M die Elemente Mo, Fe, $Z^2$, $Z^3$, $Z^4$, $Z^5$ und $Z^6$ in der Stöchiometrie $I^{S*}$,

$$Mo_{12} Fe_d Z^2_e Z^3_f Z^4_g Z^5_h Z^6_i \qquad (I^{S*}),$$

enthält;

- aus der wässrigen Mischung M durch Trocknen und Einstellen des Zerteilungsgrades $d_{90}^{A2}$ eine feinteilige Ausgangsmasse A2 mit einem Partikeldurchmesser $d_{90}^{A2}$ unter der Maßgabe erzeugt, dass

$$400 \text{ μm} \geq d_{90}^{A2} \geq 10 \text{ μm}$$ erfüllt ist;

- Ausgangsmasse A1 und Ausgangsmasse A2, oder Ausgangsmasse A1, Ausgangsmasse A2 und feinteilige Formgebungshilfsmittel zu einer feinteiligen Ausgangsmasse A3 mit der Maßgabe miteinander vermischt, dass die Ausgangsmasse A3 die über die Ausgangsmassen A1 und A2 in die Ausgangsmasse A3 eingebrachten, von Sauerstoff verschiedenen Elemente des Multielementoxids $I^S$ in der Stöchiometrie $I^{S**}$

$$[Bi_a Z^1_b]_p [Mo_{12} Fe_d Z^2_e Z^3_f Z^4_g Z^5_h Z^6_j]_1 \qquad (I^{S**}),$$

enthält,

- mit feinteiliger Ausgangsmasse A3 geometrische Formkörper V formt, und

- die Formkörper V bei erhöhter Temperatur unter Erhalt der geometrischen Katalysatorformkörper $K^S$ thermisch behandelt.

Außerdem betrifft vorliegende Erfindung die Verwendung von Katalysatorformkörpern $K^S$.

Geometrische Katalysatorformkörper $K^S$, die als Aktivmasse ein Multielementoxid $I^S$ enthalten, sowie Verfahren zur Herstellung solcher Katalysatorformkörper sind bekannt (vgl. z. B. die Deutsche Anmeldung mit dem Aktenzeichen 102007003778.5, EP-A 575 897, EP-A-1005908, WO 2007/017431, WO 02/24620, WO 2005/42459, WO 2005/47224, WO 2005/49200, WO 2005/113127, die Deutsche Anmeldung mit dem Aktenzeichen 102008040093.9, die Deutsche Anmeldung mit dem Aktenzeichen 102008040094.7 und die DE-A 102007005606).

Es ist ferner bekannt, dass Katalysatoren $K^S$ (geometrische Katalysatorformkörper $K^S$) zur Durchführung von heterogen katalysierten Partialoxidationen von 3 bis 6 C-Atome aufweisenden Alkanen, Alkanolen, Alkenen und/oder Alkenalen in der Gasphase geeignet sind.

Unter einer vollständigen Oxidation einer organischen Verbindung mit molekularem Sauerstoff wird in dieser Schrift verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden in dieser Schrift als Partialoxidationen einer organischen Verbindung zusammengefasst.

Im Besonderen sollen in dieser Schrift unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation.

Der Begriff der partiellen Oxidation soll in dieser Schrift aber auch die oxidative Dehydrierung und die partielle Ammoxidation, d. h., eine partielle Oxidation im Beisein von Ammoniak, umfassen.

Besonders geeignet sind Katalysatoren $K^S$ (geometrische Katalysatorformkörper $K^S$) zur Durchführung der heterogen katalysierten partiellen Gasphasenoxidation von Propen zu Acrolein und von iso-Buten zu Methacrolein, sowie zur Durchführung der heterogen katalysierten partiellen Gasphasenammoxidation von Propen zu Acrylnitril und von iso-Buten zu Methacrylnitril.

In der Regel bildet die heterogen katalysierte partielle Gasphasenoxidation von Propen (iso-Buten) zu Acrolein (Methacrolein) die erste Stufe einer zweistufigen heterogen katalysierten partiellen Gasphasenoxidation von Propen (iso-Buten) zu Acrylsäure (Methacrylsäure), wie sie beispielhaft in der WO 2005/42459 beschrieben ist.

Eine mit einer heterogen katalysierten partiellen Gasphasenoxidation von Propen (iso-Buten) zu Acrolein (Methacrolein) einhergehende Nebenproduktbildung an Acrylsäure (Methacrylsäure) ist daher in der Regel nicht unerwünscht und subsumiert normalerweise unter der erwünschten Wertproduktbildung.

Es ist weiterhin bekannt, dass die Performance (Aktivität und Selektivität der Zielproduktbildung (Wertproduktbildung)) von geometrischen Katalysatorformkörpern $K^S$ im Verlauf des kontinuierlichen Betriebs einer heterogen katalysierten partiellen Gasphasenoxidation von 3 bis 6 C-Atomen aufweisenden Alkanen, Alkanolen, Alkenen und/oder Alkenalen (z. B. zu den entsprechenden olefinisch ungesättigten Aldehyden und/oder Carbonsäuren) anfänglich mit zunehmender Betriebsdauer bis zu einem Maximalwert zunimmt (dies trifft vor allem auf den Fall einer durch geometrische Katalysatorformköper $K^S$ heterogen katalysierten partiellen Gasphasenoxidation von Propen zu Acrolein und/oder Acrylsäure sowie von iso-Buten zu Methacrolein und/oder Methacrylsäure zu; es gilt aber auch für den Fall einer heterogen katalysierten partiellen Gasphasenammoxidation von Propen zu Acrylnitril sowie von iso-Buten zu Methacrylnitril). Diese Betriebsphase mit einer zunehmenden Performance der geometrischen Katalysatorformkörper $K^S$ wird auch als Formierungsphase (oder verkürzt auch einfach "Formierung") der geometrischen Katalysatorformkörper $K^S$ bezeichnet. Sie erstreckt sich in der Regel über Zeiträume von mehreren Monaten und teilweise sogar über mehr als ein Jahr, wobei Aktivität und Selektivität der Zielproduktbildung ihren Maximalwert in der Regel zu unterschiedlichen Betriebszeitpunkten erreichen.

Ein Maß für die Aktivität geometrischer Katalysatorformkörper $K^S$ beziehungsweise eines diese enthaltenden Katalysatorbetts ist diejenige Temperatur, die erforderlich ist, um beim Durchgang des die partiell zu oxidierende organische Verbindung enthaltenden Reaktionsgasgemischs durch das Katalysatorbett einen bestimmten Umsatz der organischen Verbindung (z. B. des Propens oder des iso-Butens) zu erzielen. Befindet sich das Katalysatorbett z. B. in den von einem Salzbad umströmten Rohren eines Rohrbündelreaktors, kann mit zunehmender Aktivierung des Katalysatorbetts normalerweise, unter ansonsten unveränderten Betriebsbedingungen, die zur Erzielung des angestrebten, auf einen Einmaldurchgang des Reaktionsgasgemischs durch das Katalysatorbett bezogenen, Partialoxidationsumsatz erforderliche Eintrittstemperatur des Salzbads in den Rohrbündelreaktor sukzessive gesenkt werden. Das Betreiben des Katalysatorbetts bei einer tieferen Temperatur verlängert normalerweise dessen Lebensdauer.

Unter der Zielproduktselektivität (Wertproduktselektivität) wird das Verhältnis von beim einmaligen Durchgang des Reaktionsgasgemischs durch das Katalysatorbett gebildeter molarer Wertproduktmenge zu umgesetzter molarer Menge an partiell zu oxidierender organischer Ausgangsverbindung (• 100 in mol%) verstanden. D. h., eine erhöhte Anfangsselektivität der Wertproduktbildung mindert den für die Herstellung einer bestimmten Wertproduktmenge erforderlichen Rohstoffbedarf.

Insgesamt besteht somit eine generelles Interesse an geometrischen Katalysatorformkörpern $K^S$, die im kontinuierlichen Betrieb der heterogen katalysierten partiellen Gasphasenoxidation einerseits eine möglichst hohe Anfangsaktivität aufweisen und andererseits gleichzeitig eine möglichst hohe Anfangsselektivität der Wertproduktbildung gewährleisten.

Die Charakteristik der im Stand der Technik empfohlenen geometrischen Katalysatorformkörper $K^S$ ist dadurch gekennzeichnet, dass sie ein Beisein des Elements Bi im Anteil $T^S$ der Multielementoxid-I-Aktivmasse entweder ausschließen (vgl. z. B. EP-A 575 897, WO 02/24620) oder allenfalls unter ferner liefen als optional miteinbeziehen (vgl. z. B. DE-A 10 2007 004 961, WO 2007/017431 und die Deutsche Anmeldung mit dem Aktenzeichen 102008040093.9). Die experimentellen Ausführungsbeispiele des Standes der Technik schließen Bi in allen Fällen als Bestandteil des Anteils $T^S$ der jeweiligen Multielementoxid-$I^S$-Aktivmasse aus.

Angesichts dieses Sachverhalts bestand die Aufgabe der vorliegenden Erfindung darin, geometrische Formkörper $K^S$ und ein Verfahren zu deren Herstellung zur Verfügung zu stellen, die, insbesondere im Rahmen einer Verwendung für eine heterogen katalysierte Gasphasenoxidation von Propen zu Acrolein sowie von iso-Buten zu Methacrolein, im kontinuierlichen Partialoxidationsbetrieb sowohl eine erhöhte Anfangsselektivität der Wertproduktbildung gewährleisten, als auch eine erhöhte Anfangsaktivität aufweisen, und dies möglichst ohne signifikante Minderung ihrer Langzeitstabilität.

Als Lösung der Aufgabe wird ein Verfahren zur Herstellung von geometrischen Katalysatorformkörpern K, die als Aktivmasse ein Multielementoxid I der allgemeinen Stöchiometrie I,

$$[Bi_aZ^1_bO_x]_p[Bi_cMo_{12}Fe_dZ^2_eZ^3_fZ^4_gZ^5_hZ^6_iO_y]_1 \qquad (I),$$

mit

$Z^1 =$ Wolfram oder Wolfram und Molybdän, mit der Maßgabe, dass wenigstens 10 mol-% der molaren Gesamtmenge an $Z^1$ Wolfram ist,

$Z^2 =$ ein Element oder mehr als ein Element aus der Gruppe bestehend aus Nickel und Kobalt,

$Z^3 =$ ein Element oder mehr als ein Element aus der Gruppe bestehend aus den Alkalimetallen, den Erdalkalimetallen und Thallium,

$Z^4 =$ ein Element oder mehr als ein Element aus der Gruppe bestehend aus Zink, Phosphor, Arsen, Bor, Antimon, Zinn, Cer, Vanadium und Chrom,

$Z^5 =$ ein Element oder mehr als ein Element aus der Gruppe bestehend aus Silizium, Aluminium, Titan, Wolfram und Zirkonium,

$Z^6$ = ein Element oder mehr als ein Element aus der Gruppe bestehend aus Kupfer, Silber, Gold, Yttrium, Lanthan und den Lanthaniden,

a = 0,1 bis 3,
b = 0,1 bis 10,
d = 0,01 bis 5,
e = 1 bis 10,
f = 0,01 bis 2,
g = 0 bis 5,
h = 0 bis 10,
i = 0 bis 1,
p = 0,05 bis 6, und
x, y = Zahlen, die durch die Wertigkeit und Häufigkeit der von Sauerstoff verschiedenen Elemente in I bestimmt werden,

enthalten, bei dem man

- ein feinteiliges Mischoxid $Bi_a Z^1_b O_x$ mit einem Partikeldurchmesser $d^{A1}_{50}$ als Ausgangsmasse A1 mit der Maßgabe vorbildet, dass $1\ \mu m \leq d^{A1}_{50} \leq 100\ \mu m$ erfüllt ist;

- unter Verwendung von Quellen der von Sauerstoff verschiedenen Elemente des Anteils T = $[Bi_c Mo_{12} Fe_d Z^2_e Z^3_f Z^4_g Z^5_h Z^6_i O_y]_1$ des Multielementoxids I in wässrigem Medium mit der Maßgabe eine innige wässrige Mischung M erzeugt, dass

  - jede der verwendeten Quellen im Verlauf der Herstellung der wässrigen Mischung M einen Zerteilungsgrad Q durchläuft, der dadurch gekennzeichnet ist, dass sein Durchmesser $d^Q_{90} \leq 5\ \mu m$ beträgt, und
  - die wässrige Mischung M die Elemente Bi, Mo, Fe, $Z^2$, $Z^3$, $Z^4$, $Z^5$ und $Z^6$ in der Stöchiometrie I*,

$$Bi_c Mo_{12} Fe_d Z^2_e Z^3_f Z^4_g Z^5_h Z^6, \qquad (I^*),$$

  enthält;

- aus der wässrigen Mischung M durch Trocknen und Einstellen des Zerteilungsgrades $d^{A2}_{90}$ eine feinteilige Ausgangsmasse A2 mit einem Partikeldurchmesser $d^{A2}_{90}$ unter der Maßgabe erzeugt, dass $400\ \mu m \geq d^{A2}_{90} \geq 10\ \mu m$ erfüllt ist;

- Ausgangsmasse A1 und Ausgangsmasse A2, oder Ausgangsmasse A1, Ausgangsmasse A2 und feinteilige Formgebungshilfsmittel zu einer feinteiligen Ausgangsmasse A3 mit der Maßgabe miteinander vermischt, dass die Ausgangsmasse A3 die über die Ausgangsmassen A1 und A2 in die Ausgangsmasse A3 eingebrachten, von Sauerstoff verschiedenen, Elemente des Multielementoxids I in der Stöchiometrie I**

$$[Bi_a Z^1_b]_p [Bi_c Mo_{12} Fe_d Z^2_e Z^3_f Z^4_g Z^5_h Z^6_i]_1 \qquad (I^{**}),$$

  enthält,

- mit feinteiliger Ausgangsmasse A3 geometrische Formkörper V formt, und

- die Formkörper V bei erhöhter Temperatur unter Erhalt der geometrischen Katalysatorformkörper K thermisch behandelt,

zur Verfügung gestellt, das dadurch gekennzeichnet ist, dass der stöchiometrische Koeffizient c die Bedingung $0 < c \leq 0,8$ erfüllt.

Erfindungsgemäß bevorzugt ist $c \geq 0,001$, vorteilhaft $\geq 0,002$ und besonders bevorzugt ist $c \geq 0,003$ oder $\geq 0,005$.

Unter Einbezug des Gesichtspunktes der Langzeitstabilität der erfindungsgemäßen geometrischen Katalysatorformkörper K (insbesondere im Rahmen einer heterogen katalysierten partiellen Gasphasenoxidation von Propen zu Acrolein oder von iso-Buten zu Methacrolein) ist c anwendungstechnisch zweckmäßig $\leq 0{,}7$ oder $\leq 0{,}6$.

Erfindungsgemäß besonders günstige Werte für c erfüllen somit beispielsweise die Relationen $0{,}007 \leq c \leq 0{,}5$, oder $0{,}01 \leq c \leq 0{,}4$, oder $0{,}02 \leq c \leq 0{,}3$, oder $0{,}03 \leq c \leq 0{,}2$, oder $0{,}04 \leq c \leq 0{,}1$. Ein weiterer erfindungsgemäß vorteilhafter Wertebereich ist der Bereich $0{,}005 \leq c \leq 0{,}08$, oder $0{,}01 \leq c \leq 0{,}06$.

Darüber hinaus ist es erfindungsgemäß von Vorteil, wenn p 0,1 bis 4, oder 0,2 bis 3, oder 0,2 bis 2, oder 0,3 bis 1 beträgt.

Ferner ist es für die erfindungsgemäßen Zielsetzungen vorteilhaft, wen das Verhältnis der im Multielementoxid I insgesamt enthaltenen molaren Menge an Bi ($n_{Bi}$) zur im Multielementoxid I insgesamt enthaltenen molaren Menge an Mo ($n_{Mo}$), d. h., $n_{Bi}:n_{Mo}$, (0,3 bis 2) : 12, vorzugsweise (0,4 bis 1,8) : 12, und besonders bevorzugt (0,5 bis 1,6) : 12 beträgt.

Weiterhin ist es für die erfindungsgemäße Verfahrensweise günstig, wenn $1\ \mu m \leq d_{50}^{A1} \leq 75\ \mu m$, oder $1\ \mu m \leq d_{50}^{A1} \leq 50\ um$, oder $1\ \mu m \leq d_{50}^{A1} \leq 25\ \mu m$ beträgt. Weitere bevorzugt Wertebereiche für $d_{50}^{A1}$ sind die Bereiche $1\ \mu m \leq d_{50}^{A1} \leq 10\ \mu m$, $1{,}2\ \mu m \leq d_{50}^{A1} \leq 8\ \mu m$, $1{,}5\ \mu m \leq d_{50}^{A1} \leq 6\ \mu m$, $1{,}5\ \mu m \leq d_{50}^{A1} \leq 4\ \mu m$ und $2\ \mu m \leq d_{50}^{A1} \leq 3\ \mu m$ ist.

In entsprechender Weise ist es erfindungsgemäß von Vorteil, wenn 300 $300\ \mu m \geq d_{90}^{A2} \geq 10\ \mu m$, oder $200\ \mu m \geq d_{90}^{A2} \geq 20\ \mu m$, oder $170\ \mu m \geq d_{90}^{A2} \geq 30\ \mu m$, oder $150\ \mu m \geq d_{90}^{A2} \geq 40\ \mu m$, oder $130\ \mu m \geq d_{90}^{A2} \geq 40\ \mu m$ ist. Günstig ist auch $100\ \mu m \geq d_{90}^{A2} \geq 50\ \mu m$.

Als vorteilhaft für das erfindungsgemäße Verfahren erweist es sich ferner, wenn für die feinteilige Ausgangsmasse A2 auch $10\ \mu m \leq d_{50}^{A2} \leq 100\ \mu m$, vorzugsweise $20\ \mu m \leq d_{50}^{A2} \leq 60\ \mu m$ erfüllt ist.

Schließlich ist es für das erfindungsgemäße Verfahren zusätzlich von Vorteil, wenn das Verhältnis des Partikeldurchmessers $d_{90}^{A2}$ der feinteiligen Ausgangsmasse A2 zum Partikeldurchmesser $d_{10}^{A2}$ der feinteiligen Ausgangsmasse A2, das heißt $d_{90}^{A2} : d_{10}^{A2}$ (angegeben in derselben Längeneinheit) im Bereich von 5 bis 20, vorzugsweise im Bereich von 10 bis 15 liegt.

Zur Bestimmung von Partikeldurchmesserverteilungen in Trockenpulvern sowie den aus diesen entnommenen Partikeldurchmessern wie z. B. $d_{10}$, $d_{50}$ und $d_{90}$, wurde (soweit nicht ausdrücklich etwas anders explizit erwähnt wird) das jeweilige feinteilige Pulver über eine Dispergierrinne in den Trockendispergierer Sympatec RODOS (Sympatec GmbH, System-Partikel-Technik, Am Pulverhaus 1, D-38678 Clausthal-Zellerfeld) geführt, dort mit Druckluft trocken dispergiert und im Freistrahl in die Messzelle geblasen. In dieser wird dann nach ISO 13320 mit dem Laserbeugungsspektrometer Malvern Mastersizer S (Malvern Instruments, Worcestshire WR 14 1AT, United Kingdom) die volumenbezogene Partikeldurchmesserverteilung bestimmt. Die als Messergebnis angegebenen Partikeldurchmesser $d_x$ sind dabei so definiert, dass X % des Gesamtpartikelvolumens aus Partikeln mit diesem oder einem kleinerem Durchmesser bestehen. Das heißt, (100-X) % des Gesamtpartikelvolumens bestehen aus Partikeln mit einem Durchmesser > $d_x$. Wird in dieser Schrift nicht ausdrücklich etwas anderes erwähnt, beziehen sich Partikeldurchmesserbestimmungen und daraus entnommene $d_x$ wie z. B $d_{90}^{Q}$, $d_{50}^{A1}$ und $d_{90}^{A2}$ auf einen bei der Bestimmung angewandten (die Stärke der Dispergierung des Trockenpulvers während der Messung bestimmenden) Dispergierdruck von 2 bar absolut.

Alle in dieser Schrift auf ein Röntgendiffraktogramm bezogenen Angaben beziehen sich auf ein unter Anwendung von Cu-K$\alpha$-Strahlung als Röntgenstrahlung erzeugtes Röntgendiffraktogramm (Diffraktometer Theta-Theta Bruker D8 Advance, Röhrenspannung: 40 kV, Röhrenstrom: 40 mA, Aperturblende V20 (variabel), Streustrahlblende V20 (variabel), Detektorblende (0,1 mm), Meßintervall (2$\Theta$ = 2 Theta): 0,02°, Meßzeit je Schritt: 2,4 s, Detektor: Si-Halbleiterdetektor).

Die Definition der Intensität eines Beugungsreflexes im Röntgendiffraktogramm bezieht sich in dieser Schrift auf die in der DE-A 198 35 247, sowie die in der DE-A 100 51 419 und DE-A 100 46 672 niedergelegte Definition.

D.h., bezeichnet $A^1$ den Scheitelpunkt eines Reflexes 1 und bezeichnet $B^1$ in der Linie des Röntgendiffraktogramms bei Betrachtung entlang der zur 2$\Theta$-Achse senkrecht stehenden Intensitätsachse das nächstliegende ausgeprägte Minimum (Reflexschultern ausweisende Minima bleiben unberücksichtigt) links vom Scheitelpunkt $A^1$ und $B^2$ in entsprechender Weise das nächstliegende ausgeprägte Minimum rechts vom Scheitelpunkt $A^1$ und bezeichnet $C^1$ den Punkt, an dem eine vom Scheitelpunkt $A^1$ senkrecht zur 2$\Theta$-Achse gezogene Gerade eine die Punkte $B^1$ und $B^2$ ver-

bindende Gerade schneidet, dann ist die Intensität des Reflexes 1 die Länge des Geradenabschnitts $A^1C^1$, der sich vom Scheitelpunkt $A^1$ zum Punkt $C^1$ erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der $2\Theta$-Achse und der Intensitätsachse herangezogen werden. Eine beispielhafte Durchführung einer Intensitätsbestimmung zeigt die Fig. 6 in der DE-A 100 46 672. Eingehende Ausführungen zur Intensitätsbestimmung von Röntgenbeugungsreflexen finden sich auch in der DE-A 101 22 027. Aussagen zu Halbwertsbreiten von Beugungslinien beziehen sich in dieser Schrift in entsprechender Weise auf die Länge des Geradenabschnitts, der sich zwischen den beiden Schnittpunkten H1 und H2 ergibt, wenn man in der Mitte des Geradenabschnitts $A^1C^1$ eine Parallele zur $2\Theta$-Achse zieht, wobei $H^1$, $H^2$ den jeweils ersten Schnittpunkt dieser Parallelen mit der wie vorstehend definierten Linie des Röntgendiffraktogramms links und rechts von $A^1$ meinen. In der Regel betragen die Halbwertsbreiten von Röntgenbeugungsreflexen der Multielementoxid-I-Aktivmassen $\leq 1°$, sowie meist $\leq 0,5°$.

Alle Angaben in dieser Schrift zu spezifischen Oberflächen von Feststoffen beziehen sich auf Bestimmungen nach DIN 66131 (Bestimmung der spezifischen Oberfläche von Feststoffen durch Gasadsorption ($N_2$) nach Brunauer-Emmet-Teller (BET)), soweit nicht ausdrücklich etwas anderes erwähnt wird.

Das erfindungsgemäße Erfordernis, dass jede Quelle der von Sauerstoff verschiedenen Elemente des Anteils $T = [Bi_cMo_{12}Fe_dZ^2{}_eZ^3{}_fZ^4{}_gZ^5{}_hZ^6{}_jO_y]_1$ des Multielementoxids I im Verlauf der Herstellung der wässrigen Mischung M einen Zerteilungsgrad Q durchlaufen muss, der dadurch gekennzeichnet ist, dass sein Durchmesser $d^Q_{90} \leq 5$ μm beträgt, bringt zum Ausdruck, dass durchaus von einer grobkörnigeren Quelle (von einem grobkörnigeren Ausgangsmaterial) ausgegangen werden kann. Auf dem Weg der Einarbeitung einer solchen Quelle in die wässrige Mischung M, muss diese Quelle jedoch wenigstens einmal das Erfordernis $d^Q_{90} \leq 5$ μm erfüllen (selbstredend ist $d^Q_{90}$ stets $> 0$ μm).

Das Erfordernis $d^Q_{90} \leq 5$ μm ist grundsätzlich dann erfüllt, wenn man eine Quelle in einem Lösungsmittel auflöst (z. B. in wässrigem Medium; der Begriff "Lösen" ist dabei im Sinne einer molekularen bzw. ionischen Lösung gemeint) und die dabei resultierende Lösung zur Herstellung der wässrigen Mischung M verwendet. Dies ist dadurch bedingt, dass beim Lösen einer Quelle (Ausgangsverbindung, Ausgangssubstanz) in einem Lösungsmittel die Quelle im Lösungsmittel molekular bzw. ionisch zerteilt wird. Das heißt, die in der Lösung befindliche größte geometrische Einheit der gelösten Ausgangssubstanz (Quelle) weist unabdingbar "molekulare" Ausmaße auf, die damit in notwendiger Weise wesentlich kleiner als 5 μm sind. Selbstverständlich kann in ein- und derselben Lösung aber auch mehr als eine Quelle (wobei eine Quelle aber auch mehr als ein Element des Anteils T enthalten und damit gleichzeitig Quelle für mehr als ein Element sein kann) eines Elements des Anteils T gelöst und die dabei resultierende Lösung zur Herstellung der wässrigen Mischung M verwendet werden.

Das Erfordernis $d^Q_{90} \leq 5$ μm ist aber auch dann erfüllt, wenn sich eine Quelle eines Elements des Anteils T in einem Lösungsmittel in kolloidaler Lösung befindet. Kolloidale Lösungen stellen eine Verbindung zwischen echten (molekularen oder ionischen) Lösungen und Suspensionen dar. In diesen kolloiddispersen Systemen befinden sich kleinere Anhäufungen von Molekülen oder Atomen, welche jedoch weder mit dem bloßen Auge, noch mit dem Mikroskop erkennbar sind.

Die kolloidale Lösung erscheint optisch völlig klar (wenn auch oft gefärbt), da die in ihr enthaltenen Teilchen nur einen Durchmesser von 1 bis 250 nm (vorzugsweise bis 150 nm und besonders bevorzugt bis 100 nm) haben, weshalb der zugehörige $d^Q_{90}$ in notwendiger Weise $\leq 5$ μm beträgt. Aufgrund der geringen Größe ist eine Abtrennung der kolloidal gelösten Partikel durch konventionelle Filtration nicht möglich. Sie können jedoch durch Ultrafiltration mit Membranen pflanzlichen, tierischen oder künstlichen Ursprungs (z. B. Pergament, Schweinsblase oder Cellophan) von ihrem "Lösungsmittel" abgetrennt werden. Im Gegensatz zu den "optisch leeren" echten (molekularen oder ionischen) Lösungen, kann ein Lichtstrahl nicht ohne Ablenkung durch eine kolloidale Lösung hindurchtreten. Der Lichtstrahl wird von den kolloidal gelösten Partikeln gestreut und abgelenkt. Um kolloidale Lösungen stabil zu halten und weitergehende Partikelagglomerationen zu verhindern, enthalten sie häufig Netz- und Dispergierhilfsmittel sowie andere Additive zugesetzt.

Beispielsweise kann das Element Silizium beim erfindungsgemäßen Verfahren in Form eines Kieselsols zur Herstellung der wässrigen Mischung M eingebracht werden. Kieselsole sind kolloidale Lösungen von amorphem Siliciumdioxid in Wasser. Sie sind wasserflüssig und enthalten keine sedimentierbaren Bestandteile. Ihr $SiO_2$-Gehalt kann bei oft jahrelanger Haltbarkeit (ohne Sedimentation) bis zu 50 Gew.-% und mehr betragen.

Das Erfordernis $d^Q_{90} \leq 5$ μm ist aber auch dann erfüllt, wenn eine Quelle z. B. trocken auf diese Partikelgröße zerkleinert (z. B. durch Mahlen) wird.

Grundsätzlich kann ein solches Pulver unmittelbar als solches zur Herstellung der innigen wässrigen Mischung M verwendet werden. Selbstverständlich kann es aber auch in einem flüssigen Medium suspendiert und dann in Form dieser Suspension zur Herstellung der wässrigen Mischung M verwendet werden.

Erfindungsgemäß bevorzugt beträgt $d_{90}^Q$ für alle zur Herstellung der wässrigen Mischung M verwendeten Quellen (Ausgangsverbindungen, Ausgangssubstanzen) $\leq 4\ \mu m$ oder $\leq 3\ \mu m$, besonders bevorzugt $\leq 2\ \mu m$ oder $\leq 1\ \mu m$ und ganz besonders bevorzugt $\leq 0,8\ \mu m$ oder $\leq 0,5\ \mu m$. Noch besser beträgt $d_{90}^Q$ für alle zur Herstellung der wässrigen Mischung M verwendeten Quellen (Ausgangsverbindungen, Ausgangssubstanzen) $\leq 0,3\ \mu m$ oder $\leq 0,2\ \mu m$.

Besonders bevorzugt sind solche erfindungsgemäße Verfahren, bei denen im Verlauf der Herstellung der wässrigen Mischung M alle mitverwendeten Quellen der Elemente des Anteils T den Zustand einer kolloidalen oder einer echten (molekularen bzw. ionischen) Lösung durchlaufen (die dabei resultierenden wässrigen Mischungen M sollen in dieser Schrift als wässrige Mischungen $M^L$ bezeichnet werden).

Ganz besonders bevorzugt sind solche erfindungsgemäße Verfahren, bei denen im Verlauf der Herstellung der wässrigen Mischung M alle mitverwendeten Quellen der von Silizium verschiedenen Elemente des Anteils T den Zustand einer echten (molekularen bzw. ionischen) Lösung durchlaufen (die dabei resultierenden wässrigen Mischungen M sollen in dieser Schrift als wässrige Mischung $M^{L*}$ bezeichnet werden). Enthält die wässrige Mischung M darüber hinaus eine Quelle des Elementes Silizium, handelt es sich dabei mit Vorteil um eine kolloidale Lösung derselben (besonders bevorzugt um ein Kieselsol). Solche wässrigen Mischungen M sollen in dieser Schrift als wässrige Mischungen $M^{L**}$ bezeichnet werden.

Unter einer innigen wässrigen Mischung M sollen in dieser Schrift solche Mischungen M verstanden werden, deren beim Übergang von der wässrigen Mischung M in die feinteilige Ausgangsmasse A2 gasförmig entweichender Stoffanteil zu wenigstens 50 % seines Gewichtes, vorteilhaft zu wenigstens 60 % seines Gewichtes, besonders vorteilhaft zu wenigstens 70 % seines Gewichtes, ganz besonders vorteilhaft zu wenigstens 80 % seines Gewichtes und noch besser zu wenigstens 90 % seines Gewichtes aus Wasserdampf besteht. Neben Wasser kann der vorgenannte gasförmig entweichende Stoffanteil noch Verbindungen wie HCl, $HNO_3$, Kohlendioxid, Ammoniak, Alkohole (z. B. Methanol, Ethanol, Glykol und Glyzerin), Ketone wie z. B. Aceton oder andere in Wasser bei Normalbedingungen (1 atm, 25 °C) lösliche organische Verbindungen enthalten.

Als Quellen für die Elemente des Anteils T der gewünschten erfindungsgemäßen Multielementoxidaktivmasse I kommen prinzipiell solche Verbindungen in Betracht, bei denen es sich bereits um Oxide handelt und/oder um solche Verbindungen, die durch Erhitzen, wenigstens in Anwesenheit von molekularem Sauerstoff, in Oxide überführbar sind.

Neben den Oxiden kommen als solche Ausgangsverbindungen (Quellen) vor allem Halogenide, Nitrate, Formiate, Oxalate, Citrate, Acetate, Carbonate, Aminkomplexe, Ammoniumsalze und/oder Hydroxide (sowie die Hydrate der vorgenannten Salze) in Betracht.

Eine günstige Mo-Quelle ist Ammoniumheptamolybdattetrahydrat. Grundsätzlich ist aber auch z. B. Molybdäntrioxid einsetzbar. Erfindungsgemäß günstige $Z^2$-Quellen sind die Nitrate bzw. Nitrathydrate der $Z^2$-Elemente. Erfindungsgemäß vorteilhafte $Z^3$-Quellen sind die Hydroxide und Nitrate der $Z^3$-Elemente bzw. deren Hydrate. Für das Element Eisen wird beim erfindungsgemäßen Verfahren mit Vorteil ein Eisennitrathydrat verwendet. Als Wismutquelle wird man zur Herstellung der wässrigen Mischung M vorzugsweise wasserlösliche Salze des Wismuts wie Nitrate, Carbonate, Hydroxide und/oder Acetate oder unmittelbar deren wässrige Lösungen verwenden.

Kieselsol bildet die erfindungsgemäß bevorzugte Si-Quelle. Erfindungsgemäß bevorzugte Lanthaniden sind Er, Tb, Ho, Eu, Tm, Nd, Lu, Dy, Gd, Ce und Sm. Als ihre Quelle werden vorzugsweise ebenso wie im Fall von La und Y die entsprechenden Nitrathydrate verwendet.

Neben den relevanten Quellen der Elemente des Anteils T des Multielementoxids I können in die jeweilige wässrige Mischung M auch noch Substanzen eingearbeitet werden, die wenigstens unter den Bedingungen der thermischen Behandlung der geometrischen Formkörper V unter Ausbildung der geometrischen Katalysatorformkörper K zu gasförmig entweichenden Verbindungen zerfallen und/oder zersetzt (chemisch umgesetzt) werden. Derartige Substanzen können beispielsweise als Porenbildner fungieren und zum Zweck der Einstellung der aktiven inneren Oberfläche einbezogen werden. Als solche (Hilfs)Substanzen kommen beispielsweise $NH_4OH$, $(NH_4)_2CO_3$, $NH_4HCO_3$, $NH_4NO_3$, Harnstoff, $NH_4CHO_2$, $H_2CO_3$, $HNO_3$, $H_2SO_4$, $NH_4CH_3CO_2$, $NH_4Cl$, HCl, $NH_4HSO_4$, $(NH_4)_2SO_4$, Ammoniumoxalat, Hydrate der vorgenannten Verbindungen sowie organische Substanzen wie z. B. Stearinsäure, Malonsäure, Ammoniumsalze der vorgenannten Säuren, Stärken (z. B. Kartoffelstärke und Maisstärke), Cellulose, gemahlene Nussschale, feinteiliges Kunststoffmehl (z. B. aus Polyethylen, Polypropylen), etc. in Betracht.

Erfindungsgemäß bevorzugt erfolgt die Erzeugung der feinteiligen Ausgangsmasse A2 aus der wässrigen Mischung M (insbesondere im Fall einer wässrigen Mischung $M^L$, oder $M^{L*}$, oder $M^{L**}$) durch Sprühtrocknung derselben. Das heißt, die wässrige Mischung M wird in diesem Fall zunächst in feinteilige Tröpfchen zerteilt und selbige daran anschließend getrocknet. Erfindungsgemäß bevorzugt erfolgt die Trocknung im Heißluftstrom. Grundsätzlich können zur vorgenannten Sprühtrocknung aber auch andere heiße Gase verwendet werden (z. B. Stickstoff, oder mit Stickstoff verdünnte Luft sowie sonstige inerte Gase).

Die Sprühtrocknung kann dabei grundsätzlich sowohl im Gleichstrom als auch im Gegenstrom der Tröpfchen zum heißen Gas erfolgen. Vorzugsweise erfolgt sie im Gegenstrom der Tröpfchen zum heißen Gas. Besonders bevorzugt im Heißluftgegenstrom. Typisch Gaseintrittstemperaturen liegen dabei im Bereich von 250 bis 450, vorzugsweise 270 bis 370 °C. Typische Gasaustrittstemperaturen liegen dabei im Bereich von 100 bis 160 °C.

Erfindungsgemäß bevorzugt wird die Sprühtrocknung dabei so durchgeführt, dass sich der für die feinteilige Ausgangsmasse A2 erwünschte Partikeldurchmesser $d_{90}^{A2}$ als Ergebnis der Sprühtrocknung unmittelbar einstellt (das heißt, der entsprechende $d_{90}$ des resultierenden Sprühpulvers ist), so dass das resultierende Sprühpulver unmittelbar die erfindungsgemäß einzusetzende Ausgangsmasse A2 bilden kann.

Ist der Zerteilungsgrad des resultierenden Sprühpulvers im Vergleich zum erwünschten $d_{90}^{A2}$ zu klein, so kann selbiges z. B. durch nachfolgendes Kompaktieren auf den für die Ausgangsmasse A2 erwünschten Zerteilungsgrad zielgerichtet vergröbert werden. Umgekehrt kann das bei der Sprühtrocknung unmittelbar resultierende Sprühpulver durch Mahlen bei Bedarf auch auf den für die Ausgangsmasse A2 erwünschten Zerteilungsgrad verfeinert werden.

Selbstverständlich kann die innige wässrige Mischung M aber auch zunächst durch konventionelles Eindampfen (vorzugsweise bei vermindertem Druck; die Trocknungstemperatur sollte im Regelfall 150 °C nicht überschreiten) getrocknet und die dabei resultierende Trockenmasse durch nachfolgendes Zerkleinern auf den erfindungsgemäß erforderlichen Zerteilungsgrad $d_{90}^{A2}$ eingestellt werden. Grundsätzlich kann die Trocknung der wässrigen Mischung M beim erfindungsgemäßen Verfahren aber auch durch Gefriertrocknung erfolgen.

$Z^2$ ist beim erfindungsgemäßen Verfahren bevorzugt ausschließlich Co.
$Z^3$ ist beim erfindungsgemäßen Verfahren vorzugsweise K, Cs und/oder Sr, besonders bevorzugt K.
$Z^5$ ist beim erfindungsgemäßen Verfahren vorzugsweise Si.

Der stöchiometrische Koeffizient a beträgt vorteilhaft 0,3 bis 3, vorzugsweise 0,5 bis 3 und besonders bevorzugt 1,5 bis 2,5.

Der stöchiometrische Koeffizient b beträgt vorteilhaft 1 bis 6, besonders vorteilhaft 2 bis 5 und ganz besonders vorteilhaft 3 bis 5.

Erfindungsgemäß bevorzugt sind wenigstens 20 mol-%, besser wenigstens 40 mol-% oder wenigstens 50 mol-%, und noch besser wenigstens 60 mol-% oder wenigstens 80 mol-% sowie am Besten wenigstens 90 mol-% oder 100 mol-% der molaren Gesamtmenge an $Z^1$ Wolfram

Der stöchiometrische Koeffizient e beträgt vorzugsweise 3 bis 8, besonders vorteilhaft 4 bis 7 und ganz besonders vorteilhaft 5 bis 6.

Der stöchiometrische Koeffizient f beträgt vorteilhaft 0,02 bis 2 und besonders vorteilhaft 0,03 bis 1 bzw. 0,05 bis 0,5.

Der stöchiometrische Koeffizient d beträgt vorteilhaft 0,1 bis 4,5, vorzugsweise 0,5 bis 4 und besonders bevorzugt 1 bis 4 oder 2 bis 4.

Der stöchiometrische Koeffizient h beträgt vorzugsweise > 0 bis 10, besonders bevorzugt 0,1 bis 8 oder 0,2 bis 7, ganz besonders bevorzugt 0,3 bis 6 oder 0,4 bis 5, und am vorteilhaftesten 0,5 bis 3 oder 1 bis 3.

Die stöchiometrischen Koeffizienten g und i können beide gleichzeitig 0 betragen, aber auch unabhängig voneinander von 0 verschiedene Werte annehmen.

Das heißt, die Ausführungsbeispiele B1 bis B13 und die Vergleichsbeispiele V1 bis V15 (aus diesen werden dadurch Beispiele) können unter ansonsten unveränderten Bedingungen (einschließlich der nachfolgenden Verwendung als Katalysatoren für die Propenpartialoxidation) auch unter Verwendung feinteiliger Ausgangsmasse A2 durchgeführt werden, deren zugehörige Stöchiometrie I* $Mo_{12}Ni_{3,0}Co_{2,5}Fe_{3,0}Bi_{0,01}Si_{1,6}K_{0,08}$, oder $Mo_{12}Ni_{3,0}Co_4Fe_{3,0}Bi_{0,02}Si_{1,6}K_{0,08}$, oder $Mo_{12}Sb_{0,2}Co_{4,2}Fe_14Zn_{0,2}Bi_{0,03}W_{0,1}K_{0,06}$, oder $Mo_{12}Sb_{0,2}Co_{4,2}Fe_{1,4}Zn_{0,2}Bi_{0,8}W_{0,1}K_{0,06}$, oder $Mo_{12}Ni_{2,8}Co_{5,2}Fe_{1,8}Bi_{0,05}K_{0,1}$, oder $Mo_{12}Ni_{2,8}Co_{5,2}Fe_{1,8}Bi_{0,7}K_{0,1}$, oder $Mo_{12}Co_5Fe_1Ni_3W_{0,5}Bi_{0,1}K_{0,1}$, oder $Mo_{12}Co_5Fe_1Ni_3W_{0,5}Bi_{0,8}K_{0,1}$, oder $Mo_{12}Co_7Fe_{3,0}Bi_{0,02}Si_{1,6}K_{0,08}$, oder $Mo_{12}Co_7Fe_{3,0}Bi_{0,05}Si_{1,6}K_{0,08}$, oder $Mo_{12}Co_7Fe_{3,0}Bi_{0,1}Si_{1,6}K_{0,08}$, oder $Mo_{12}Co_7Fe_{3,0}Bi_{0,2}Si_{1,6}K_{0,08}$, oder $Mo_{12}Co_7Fe_{3,0}Bi_{0,5}Si_{1,6}K_{0,08}$, oder $Mo_{12}Co_7Fe_{3,0}Bi_{0,01}Si_{1,6}K_{0,08}$, oder $Mo_{12}Co_{5,5}Fe_{3,0}Bi_{0,02}Gd_{0,05}Si_{1,6}K_{0,08}$, oder $Mo_{12}Co_{5,5}Fe_{3,0}Bi_{0,03}Y_{0,05}Si_{1,6}K_{0,08}$, oder $Mo_{12}Co_{5,5}Fe_{3,0}Bi_{0,01}Er_{0,05}Si_{1,6}K_{0,08}$, oder $Mo_{12}Co_{5,5}Fe_{3,0}Bi_{0,02}Sm_{0,05}Si_{1,6}K_{0,08}$, oder $Mo_{12}Co_{5,5}Fe_{3,0}Bi_{0,06}Eu_{0,05}Si_{1,6}K_{0,08}$, oder $Mo_{12}Co_{5,5}Fe_{3,0}Bi_{0,03}Dy_{0,05}Si_{1,6}K_{0,08}$, oder $Mo_{12}Co_{5,5}Fe_{3,0}Bi_{0,02}Yb_{0,05}Si_{1,6}K_{0,08}$, oder $Mo_{12}Co_{5,5}Fe_{3,0}Bi_{0,03}Tb_{0,05}Si_{1,6}K_{0,08}$, oder $Mo_{12}Co_{5,5}Fe_{3,0}Bi_{0,05}Ho_{0,05}Si_{1,6}K_{0,08}$, oder $Mo_{12}Co_{5,5}Fe_{3,0}Bi_{0,04}Ce_{0,05}Si_{1,6}K_{0,08}$, oder $Mo_{12}Co_{5,5}Fe_{3,0}Bi_{0,06}La_{0,05}Si_{1,6}K_{0,08}$ ist. In der feinteiligen Ausgangsmasse A2 von Beispiel B1 nicht enthaltene, von den vorstehenden Stöchiometrien aber umfasste Elemente werden dabei unter Verwendung ihrer Nitrathydrate als Quelle in der Lösung B aufgelöst. Abweichend hiervon wird W als Ammoniumparawolframat zur Lösung A zugesetzt.

Gleichzeitig kann in allen vorgenannten Fallgestaltungen sowie in den Ausführungsbeispielen B1 bis B13 $d_{50}^{A1}$ auch 2,2 μm, oder 2,4 μm, oder 2,6 μm, oder 2,8 μm, oder 3,0 μm, oder 3,5 μm, oder 4,0 μm, oder 4,5 μm, oder 5,0 μm, oder 5,5 μm, oder 6,0 μm, oder 6,5 μm, oder 7,0 μm betragen.

Ferner kann in allen vorstehend genannten Fällen, einschließlich aller Ausführungsbeispiele B1 bis B13, $d_{90}^{A2}$ auch 50 μm, oder 52 μm, oder 54 μm, oder 56 μm, oder 58 μm, oder 60 μm, oder 62 μm, oder 64 μm, oder 66 μm, oder 68 μm, oder 70 μm, oder 72 μm, oder 74 μm, oder 76 μm, oder 78 μm, oder 80 μm, oder 82 μm, oder 84 μm, oder 86 μm, oder 88 μm oder 90 μm betragen. Beispielhaft kann in allen vorgenannten Fällen $d_{50}^{A1}$ = 2,4 μm μm und $d_{90}^{A2}$ = 68 μm μm sein. Der stöchiometrische Koeffizient p kann dabei auch 0,25, oder 0,35, oder 0,4 sein.

Die Vorbildung des feinteiligen Mischoxids $Bi_aZ^1_b$ kann in an sich bekannter Weise erfolgen (vgl. z. B. EP-A 575 897, DE-A 3338380, EP-A 835, WO 02/24620, WO 2007/017431, die Deutsche Anmeldung mit dem Aktenzeichen 102007003778.5, WO 2005/030393 und die Deutsche Anmeldung mit dem Aktenzeichen 102008040093.9).

In der Regel wird man dabei wenigstens eine Quelle des Elementes Bi und wenigstens eine Quelle des Elements W sowie gegebenenfalls eine Quelle des Elementes Mo (d. h., wenigstens eine das Element Bi enthaltende Ausgangsverbindung und wenigstens eine das Element W enthaltende Ausgangsverbindung sowie gegebenenfalls eine das Element Mo enthaltende Ausgangsverbindung) in wässrigem Medium miteinander innig vermischen, das wässrige Gemisch trocknen und die dabei resultierende Trockenmasse bei Temperaturen im Bereich von 400 bis 900 °C (vorzugsweise 600 bis 900 °C und besonders bevorzugt 700 bis 900 °C) kalzinieren (thermisch behandeln) sowie durch Zerteilen des dabei resultierenden Kalzinats unter Erhalt der feinteiligen Ausgangsmasse A1 den erfindungsgemäß erforderlichen Partikeldurchmesser $d_{50}^{A1}$ einstellen. Als Quellen des Bi, W und Mo kommen grundsätzlich solche Verbindungen in Betracht, bei denen es sich bereits um Oxide dieser Elemente handelt, oder solche Verbindungen, die durch Erhitzen, wenigstens in Anwesenheit von molekularem Sauerstoff, in Oxide überführbar sind.

Vorzugsweise wird man wasserlösliche Salze des Wismuts wie Nitrate, Carbonate, Hydroxide und/oder Acetate mit Wolframsäure (die in Wasser im wesentlichen unlösliche Wolframsäure wird dabei vorzugsweise als feinteiliges Pulver eingesetzt, dessen $d_{90}$ anwendungstechnisch zweckmäßig ≤ 5 μm oder ≤ 2 μm, vorzugsweise 0,1 bis 1 μm beträgt) und/oder deren Ammoniumsalze in Wasser mischen, die wässrige Mischung trocknen (vorzugsweise sprühtrocknen) und die getrocknete Masse anschließend wie beschrieben thermisch behandeln. Wird Mo mitverwendet, kann z. B. Molybdäntrioxid oder Ammoniumheptamolybdattetrahydrat als Mo-Quelle eingesetzt werden.

Erfolgte die Trocknung durch Sprühtrocknung, wird man das resultierende Sprühpulver vorab der Kalzination vorteilhaft vergröbern (z. B. anwendungstechnisch vorteilhaft unter Zusatz von bis zu 20 Gew.-% Wasser anteigen und z. B. mittels eines Extruders zu für Kalzinationszwecke einfacher handhabbaren Stränglingen extrudieren; diese werden nachfolgend getrocknet und danach kalziniert). Üblicherweise erfolgt die thermische Behandlung im Luftstrom (z. B. im Fall der vorgenannten Stränglinge in einem Drehrohrofen, wie er in der DE-A 103 25 487 beschrieben ist). Das Zerteilen des resultierenden kalzinierten Mischoxids auf den erfindungsgemäß wesentlichen Partikeldurchmesser $d_{50}^{A1}$ wird man normalerweise durch Mahlen in Mühlen bewirken. Bei Bedarf wird das Mahlgut nachfolgend auf den gewünschten Zerteilungsgrad klassiert.

Bevorzugte im Rahmen des erfindungsgemäßen Verfahrens vorab gebildete Mischoxide $Bi_aZ^1_b$ sind die Mischoxide $Bi_2W_4O_{15}$ ($Bi_2W_2O_9 \cdot 2\ WO_3$), $Bi_2W_2O_9$, $Bi_2W_{2,5}O_{10,5}$ ($Bi_2W_2O_9 \cdot ½\ WO_3$), $Bi_2W_3O_{12}$ ($Bi_2W_2O_9 \cdot WO_3$), $Bi_2W_5O_{18}$ ($Bi_2W_2O_9 \cdot 3\ WO_3$), $Bi_2W_6O_{21}$ ($Bi_2W_2O_9 \cdot 4\ WO_3$), $Bi_2W_8O_{27}$ ($Bi_2W_2O_9 \cdot 6\ WO_3$) und $Bi_2W_1O_6$, unter denen das $Bi_2W_4O_{15}$ erfindungsgemäß ganz besonders bevorzugt wird (die Ausführungsbeispiele B1 bis B13 sowie die Vergleichsbeispiele V1 bis V15 (einschließlich ihrer Verwendung für die Propenpartialoxidation) können daher auch unter Verwendung von $Bi_2W_3O_{12}$, oder $Bi_2W_5O_{18}$, oder $Bi_2W_6O_{21}$, oder $Bi_2W_8O_{27}$, oder $Bi_2W_1O_6$, oder $Bi_2W_2O_9$ als feinteilige Ausgangsmasse A1 entsprechender Körnung wie das verwendete feinteilige $Bi_2W_4O_{15}$ ausgeführt werden).

Ebenso wie in die wässrige Mischung M können auch in das wässrige Gemisch der wenigstens einen Bi- sowie der wenigstens einen W-Quelle (sowie gegebenenfalls zusätzlichen Mo-Quelle) im Rahmen der Herstellung des Mischoxids $Bi_aZ^1_bO_x$ zusätzlich Substanzen eingearbeitet werden, die unter den Bedingungen der zur Ausbildung des Mischoxids $Bi_aZ^1_bO_x$ angewandten thermischen Behandlung zu gasförmig entweichenden Verbindungen zerfallen und/oder zersetzt (chemisch umgesetzt) werden. Derartige Substanzen können beispielsweise als Porenbildner fungieren und zum Zweck der Beeinflussung der aktiven inneren Oberfläche des Mischoxids $Bi_aZ^1_bO_x$ einbezogen werden.

Als solche (Hilfs)Substanzen kommen beispielsweise $NH_4OH$, $(NH_4)_2CO_3$, $NH_4HCO_3$, $NH_4NO_3$, Harnstoff, $NH_4CHO_2$, $H_2CO_3$, $HNO_3$, $H_2SO_4$, $NH_4CH_3CO_2$, $NH_4HSO_4$, $NH_4Cl$, HCl, $(NH_4)_2SO_4$, Ammoniumoxalat, Hydrate der vorgenannten Verbindungen sowie organische Substanzen wie z. B. Stearinsäure, Malonsäure, Ammoniumsalze der vorgenannten Säuren, Stärken (z. B. Kartoffelstärke und Maisstärke), Cellulose, gemahlene Nussschale, feinteiliges Kunststoffmehl (z. B. Polyethylen, Polypropylen), etc. in Betracht.

Bei der Herstellung der feinteiligen Ausgangsmasse A3 aus den feinteiligen Ausgangsmassen 1 und 2 werden anwendungstechnisch zweckmäßig, aber nicht in notwendiger Weise, feinteilige Formgebungshilfsmittel mitverwendet.

Diese können bereits vorab einer Vermischung der feinteiligen Ausgangsmassen A1 und A2 in beide dieser feinteiligen Ausgangsmassen oder in nur eine der beiden feinteiligen Ausgangsmassen A1, A2 eingemischt werden.

Selbstverständlich können die feinteiligen Formgebungshilfsmittel aber auch oder nur (erst) in das feinteilige Gemisch aus feinteiliger Ausgangsmasse A1 und feinteiliger Ausgangsmasse A2 eingemischt werden.

Zur Gruppe der feinteiligen Formgebungshilfsmittel (insbesondere dann, wenn diese Elemente $Z^5$ enthalten, werden die Formgebungshilfsmittel mit einem $d_{90} > 5 \mu m$ eingesetzt; andere von Sauerstoff verschiedene Elemente des Multielementoxids I enthalten sie üblicherweise nicht) gehören zunächst die sogenannten Antibackmittel.

Hierbei handelt es sich um feinteilige Materialien, die anwendungstechnisch vorteilhaft mitverwendet werden, um im Rahmen des Vermischens z. B. eine Reagglomeration (ein "Zusammenbacken") von Partikeln innerhalb der Ausgangsmasse A1 und/oder innerhalb der Ausgangsmasse A2 weitestgehend zu unterdrücken, da eine solche Reagglomeration den wirksamen Partikeldurchmesser beeinflussen könnte. Eine erfindungsgemäß bevorzugte Gruppe an feinteiligen Antibackmitteln bilden feinteilige hydrophobisierte Kieselsäuren, insbesondere feinteilige hydrophobisierte synthetische Kieselsäuren (Siliziumdioxide). Synthetische Kieselsäuren können einerseits unmittelbar pyrogen aus Sand und andererseits durch Fällungsreaktionen aus Wasserglas erzeugt werden. Insbesondere synthetische Kieselsäuren sind auf Grund ihrer oberflächenständigen OH-Gruppen hydrophil, d. h., sie werden durch Wasser benetzt. Zum Beispiel durch Reaktion dieser oberflächenständigen OH-Gruppen mit Chlorsilanen lassen sich sowohl aus den pyrogenen als auch aus den Fällungskieselsäuren hydrophobisierte Produkte herstellen. Beispielsweise kann die Hydrophobisierung durch Umsetzung mit Dimethyldichlorsilan im Beisein von Wasserdampf bei ca. 400 °C in einem Fließbettreaktor erfolgen (wird vorzugsweise bei pyrogenen Kieselsäuren angewendet).

Insbesondere bei Fällungskieselsäuren wird das Chlorsilan der Fällsuspension bei einer Temperatur von 50 bis 90°C unter gründlichem Rühren zugegeben. Anschließend folgen Filtration, Neutralwaschen mit Wasser, Trocknen der Filterkuchen und Tempern bei 300 bis 400°C. In H. Brunner, D. Schutte, Chem. Ing. Techn. 89, 437 (1965) sowie in DT 2435860 und DT 1117245 wird die Herstellung hydrophobisierter feinteiliger Kieselsäuren näher beschrieben. Handelsprodukte von hydrophobisierten Fällungskieselsäuren bilden z.B. die SIPERNAT®-Marken.

Erfindungsgemäß bevorzugt wird als feinteiliges Antibackmittel Sipernat® D17 der Firma Degussa bzw. der Fa. EVONIK Industries mitverwendet. Sipernat® D17 enthält auf sein Gewicht bezogen etwa 2 Gew.-% an chemisch gebundenem Kohlenstoff und wird von Wasser nicht benetzt. Sein Rüttelgewicht (gemäß ISO 787-11) beträgt 150 g/l. Sein $d_{50}$-Wert beträgt 10 $\mu m$ (Laserbeugung nach ISO 13320-1) und die spezifische Oberfläche (Stickstoffadsorption nach ISO 5794-1, Annex D) beträgt 100 $m^2/g$.

Mit Vorteil wird feinteiliges Antibackmittel wie z.B. Sipernat® D17 in die feinteilige Ausgangsmasse A1 eingemischt, bevor diese mit der feinteiligen Ausgangsmasse A2 zur feinteiligen Ausgangsmasse A3 vermischt wird. In der Regel liegt die Zusatzmenge an feinteiligem Antibackmittel dabei bei 0,1 bis 3 Gew.-%, bezogen auf das Gewicht der feinteiligen Ausgangsmasse A1.

Der Zusatz von Antibackmittel mindert auch den für eine homogene Vermischung der beiden Ausgangsmassen A1 und A2 erforderlichen Energieeintrag, was sich insbesondere auf den Erhalt der Partikelgröße der feinteiligen Ausgangsmasse A2 beim Vermischen vorteilhaft auswirkt.

Erfolgt die erfindungsgemäße Formung der feinteiligen Ausgangsmasse A3 zu den geometrischen Formkörpern V erfindungsgemäß vorteilhaft durch Verdichten (Komprimieren oder Kompaktieren), ist es anwendungstechnisch zweckmäßig, der feinteiligen Ausgangsmasse A3 als weitere feinteilige Formgebungshilfsmittel Gleitmittel wie z.B. Graphit, Ruß, Polyethylenglykol, Polyacrylsäure, Stearinsäure, Stärke, Mineralöl, Pflanzenöl, Wasser, Bortrifluorid und/oder Bornitrid zuzugeben. Eine Mitverwendung von Gleitmitteln im Rahmen einer entsprechenden Formgebung findet sich z.B. in den Schriften DE-A 102007004961, WO 2005/030393, US-A 2005/0131253, WO 2007/017431, DE-A 102007005606 und in der deutschen Anmeldung Nr. 102008040093.9 beschrieben. Erfindungsgemäß bevorzugt wird ausschließlich feinteiliger Graphit als Gleitmittel mitverwendet. Bevorzugt zugesetzte Graphite sind Asbury 3160 und Asbury 4012 der Firma Asbury Graphite Mills, Inc. New Jersey 08802, USA und Timrex® T44 der Firma Timcal Ltd., 6743 Bodio, Schweiz.

Mit Vorteil wird der feinteilige Graphit (typische $d_{90}$-Werte erfindungsgemäß geeigneter Graphite betragen 30 bis 300 $\mu m$) erst dem Gemisch aus feinteiliger Ausgangsmasse A1 und feinteiliger Ausgangsmasse A2 zugesetzt. Er kann jedoch auch vorab der Vermischung der beiden feinteiligen Ausgangsmassen A1, A2 in jede derselben (oder in nur eine der beiden) eingemischt werden. Bezogen auf das Gewicht der feinteiligen Ausgangsmasse A3 kann diese z. B. bis zu 15 Gew.-% an feinteiligem Gleitmittel enthalten. Meist liegt der Gleitmittelgehalt in der feinteiligen Ausgangsmasse A3 jedoch bei ≤ 9 Gew.-%, vielfach bei ≤ 5 Gew.-%, oft bei ≤ 4 Gew.-%; dies insbesondere dann, wenn das feinteilige Gleitmittel Graphit ist. In der Regel beträgt die vorgenannte Zusatzmenge ≥ 0,5 Gew.-%, meist ≥ 2,5 Gew.-%.

Bei Bedarf können der feinteiligen Ausgangsmasse A3 als weitere Formgebungshilfsmittel noch feinteilige Verstärkungsmittel wie Mikrofasern aus Glas, Asbest, Siliciumcarbid oder Kaliumtitanat zugesetzt werden, die sich nach Beendigung der Formgebung durch Verdichten förderlich auf den Zusammenhalt des erhaltenen Komprimats (des resultierenden Formkörpers V) auswirken.

Im Rahmen der erfindungsgemäßen thermischen Behandlung der Formkörper V, bei der die Katalysatorformkörper K erwachsen, können mitverwendete Formgebungshilfsmittel sowohl im resultierenden Katalysatorformkörper K erhalten bleiben, als auch durch thermische und/oder chemische Zersetzung zu gasförmigen Verbindungen (z.B. CO, $CO_2$) wenigstens teilweise gasförmig aus diesen entweichen. Im Katalysatorformkörper K verbleibende Formgebungshilfsmittel wirken im Rahmen einer katalytischen Verwendung desselben in selbigem im Wesentlichen ausschließlich als die Multielementoxid-I-Aktivmasse verdünnend.

Im Regelfall erfolgt die Verdichtung der feinteiligen Ausgangsmasse A3 zur gewünschten Geometrie des Formkörpers V (des geometrischen Katalysatorvorläuferformkörpers) durch Einwirkung äußerer Kräfte (Druck) auf das feinteilige Vorläufergemisch. Der dabei anzuwendende Formgebungsapparat bzw. die dabei anzuwendende Formgebungsmethode unterliegt keiner Beschränkung.

Beispielsweise kann die verdichtende Formgebung durch Strangpressen, Tablettieren oder Extrudieren erfolgen. Dabei wird die feinteilige Ausgangsmasse A3 vorzugsweise anfasstrocken eingesetzt. Sie kann jedoch z. B. bis zu 10 % ihres Gesamtgewichts Substanzen zugesetzt enthalten, die bei Normalbedingungen (25°C, 1 atm) flüssig sind. Auch kann die feinteilige Ausgangsmasse A3 feste Solvate (z.B. Hydrate) enthalten, die solche flüssigen Substanzen in chemisch und/oder physikalisch gebundener Form aufweisen. Selbstverständlich kann die feinteilige Ausgangsmasse A3 aber auch völlig frei von solchen Substanzen sein.

Erfindungsgemäß bevorzugtes Formgebungsverfahren durch Verdichten der feinteiligen Ausgangsmasse A3 ist die Tablettierung. Die Grundzüge des Tablettierens sind z.B. in "Die Tablette", Handbuch der Entwicklung, Herstellung und Qualitätssicherung, W.A. Ritschel und A. Bauer-Brandl, 2. Auflage, Edition Verlag Aulendorf, 2002 beschrieben und in völlig entsprechender Weise auf ein erfindungsgemäßes Tablettierverfahren übertragbar.

Mit Vorteil wird eine erfindungsgemäße Tablettierung wie in den Schriften WO 2005/030393, Deutsche Anmeldung Nr. 102008040093.9, Deutsche Anmeldung Nr. 102008040094.7 und WO 2007/017431 beschrieben durchgeführt.

Anstatt die feinteilige Ausgangsmasse A3 als solche unmittelbar zur gewünschten Geometrie des Formkörpers V zu verdichten (in einem einzigen Verdichtungsschritt), ist es erfindungsgemäß häufig zweckmäßig, als einen ersten Formgebungsschritt zunächst eine Zwischenkompaktierung durchzuführen, um die feinteilige Ausgangsmasse A3 zu vergröbern (in der Regel auf Partikeldurchmesser von 100 bis 2000 $\mu$m, bevorzugt 150 bis 1500 $\mu$m, besonders bevorzugt 400 bis 1250 $\mu$m, oder 400 bis 1000 $\mu$m, oder 400 bis 800 $\mu$m).

Dabei kann bereits vor der Zwischenkompaktierung z.B. feinteiliges Gleitmittel (z.B. Graphit) zugesetzt werden. Anschließend erfolgt auf der Grundlage des vergröberten Pulvers die endgültige Formgebung, wobei bei Bedarf zuvor nochmals z.B. feinteiliges Gleitmittel (z.B. Graphit) sowie gegebenenfalls weiteres Formgebungs- und/oder Verstärkungshilfsmittel zugegeben werden kann.

Ebenso wie der zur Verdichtung der feinteiligen Ausgangsmasse A3 zu verwendende Formgebungsapparat bzw. die dabei anzuwendende Formgebungsmethode, unterliegt auch die angestrebte Geometrie der resultierenden Formkörper V beim erfindungsgemäßen Verfahren keiner Beschränkung. D.h., die Katalysatorvorläuferformkörper (die Formkörper V) können sowohl regelmäßig als auch unregelmäßig geformt sein, wobei regelmäßig geformte Formkörper V in der Regel erfindungsgemäß bevorzugt sind.

Beispielsweise kann der Formkörper V beim erfindungsgemäßen Verfahren Kugelgeometrie aufweisen. Dabei kann der Kugeldurchmesser z.B. 2 bis 10 mm, oder 4 bis 8 mm betragen. Die Geometrie des Katalysatorvorläuferformkörpers (des Formkörpers V) kann aber auch vollzylindrisch oder hohlzylindrisch (ringförmig) sein. In beiden Fällen können Außendurchmesser (A) und Höhe (H) z.B. 2 bis 10 mm, oder 2 bzw. 3 bis 8 mm betragen. Im Fall von Hohlzylindern (Ringen) ist in der Regel eine Wandstärke von 1 bis 3 mm zweckmäßig. Selbstverständlich kommen als Katalysatorvorläufergeometrie aber auch alle diejenigen Geometrien in Betracht, die in der WO 02/062737 offenbart und empfohlen werden. Im Fall von Vollzylindern kann der Außendurchmesser auch 1 bis 10 mm betragen.

Die im Rahmen einer Verdichtung von feinteiliger Ausgangsmasse A3 angewandten Formgebungsdrucke werden beim erfindungsgemäßen Verfahren im Allgemeinen 50 kg/cm² bis 5000 kg/cm² betragen. Vorzugsweise betragen die Formgebungsdrucke 200 bis 3500 kg/cm², besonders bevorzugt 600 bis 25000 kg/cm².

Insbesondere im Fall von ringförmigen Formkörpern V soll die formgebende Verdichtung beim erfindungsgemäßen Verfahren der Lehre der Schriften Deutsche Anmeldung Nr. 102008040093.9, Deutsche Anmeldung Nr. 102008040094.7 und WO 2005/030393 folgend so durchgeführt werden, dass die Seitendruckfestigkeit SD des resultierenden ringförmigen Formkörpers V 12 N ≤ SD ≤ 25 N beträgt. Vorzugsweise beträgt SD ≥ 13 N und ≤ 24 N, bzw. ≥ 14 N und ≤ 22 N sowie ganz besonders bevorzugt ≥ 15 N und ≤ 20 N.

Die experimentelle Bestimmung der Seitendruckfestigkeit wird dabei wie in den Schriften WO 2005/030393 sowie WO 2007/017431 beschrieben durchgeführt. Selbstverständlich sind ringähnliche Formkörper V, wie sie die Deutsche Anmeldung Nr. 102008040093.9 empfiehlt, erfindungsgemäß ganz besonders bevorzugt. Die Stirnfläche von ringförmigen oder ringähnlichen Formkörpern V kann beim erfindungsgemäßen Verfahren sowohl gekrümmt als auch nicht gekrümmt sein (vgl. insbesondere DE-A 102007004961, EP-A 184 790 und die deutsche Anmeldung Nr.

102008040093.9). Bei der Ermittlung der Höhe solcher geometrischer Formkörper V wird eine solche Krümmung nicht berücksichtigt.

Erfindungsgemäß erhältliche Katalysatorformkörper K, die durch thermische Behandlung von Formkörpern V hergestellt wurden, welche durch Verdichtung von feinteiliger Ausgangsmasse A3 erhalten worden sind, werden als Vollkatalysatoren (Vollkatalysatorformkörper K) bezeichnet.

Erfindungsgemäß besonders vorteilhafte Ringgeometrien von durch Verdichten von feinteiliger Ausgangsmasse A3 erhältlichen Formkörpern V erfüllen die Bedingung H/A = 0,3 bis 0,7. Besonders bevorzugt ist H/A = 0,4 bis 0,6. Weiterhin ist es für die vorgenannten ringförmigen Formkörper V erfindungsgemäß günstig, wenn das Verhältnis I/A (wobei I der Innendurchmesser der Ringgeometrie ist) 0,3 bis 0,7, vorzugsweise 0,4 bis 0,7 beträgt.

Besonders vorteilhaft sind vorgenannte Ringgeometrien, wenn sie gleichzeitig eines der vorteilhaften H/A-Verhältnisse und eines der vorteilhaften I/A-Verhältnisse aufweisen. Solche möglichen Kombinationen sind z.B. H/A = 0,3 bis 0,7 und I/A = 0,3 bis 0,8 oder 0,4 bis 0,7. Alternativ kann H/A 0,4 bis 0,6 und I/A gleichzeitig 0,3 bis 0,8 oder 0,4 bis 0,7 betragen. Ferner ist es für die relevanten Ringgeometrien günstig, wenn H 2 bis 6 mm und vorzugsweise 2 bis 4 mm beträgt. Weiterhin ist es vorteilhaft, wenn A bei den Ringen 4 bis 8 mm, vorzugsweise 4 bis 6 mm beträgt. Die Wandstärke erfindungsgemäß bevorzugter Ringgeometrien beträgt 1 bis 1,5 mm.

Mögliche Ringgeometrien für vorgenannte ringförmige Formkörper V sind somit (A x H x I) 5 mm x 2 mm x 2 mm, oder 5 mm x 3 mm x 2 mm, oder 5 mm x 3 mm x 3 mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm, oder 7 mm x 7 mm x 3 mm, oder 7 mm x 7 mm x 4 mm.

Die thermische Behandlung von erfindungsgemäßen Formkörpern V (insbesondere ringförmiger Formkörper V; alles Nachfolgende gilt insbesondere für ihre thermische Behandlung) unter Erhalt der geometrischen Katalysatorformkörper K erfolgt im Rahmen des erfindungsgemäßen Verfahrens in der Regel bei Temperaturen (damit ist in dieser Schrift die Temperatur innerhalb des Kalzinationsgutes gemeint), die 350°C überschreiten. Normalerweise wird im Rahmen der thermischen Behandlung die Temperatur von 650°C jedoch nicht überschritten. Erfindungsgemäß vorteilhaft wird im Rahmen der thermischen Behandlung die Temperatur von 600°C, bevorzugt die Temperatur von 550°C und besonders bevorzugt die Temperatur von 500°C nicht überschritten.

Ferner wird im Rahmen der thermischen Behandlung der Formkörper V vorzugsweise die Temperatur von 380°C, mit Vorteil die Temperatur von 400°C, mit besonderem Vorteil die Temperatur von 420°C und ganz besonders bevorzugt die Temperatur von 440°C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur (Phase 1) von 150 bis 350°C, vorzugsweise 220 bis 290°C, und daran anschließend eine thermische Behandlung bei einer Temperatur (Phase 2) von 400 bis 600°C, vorzugsweise 430 bis 550°C durchgeführt werden.

Normalerweise nimmt die thermische Behandlung der Formkörper V mehrere Stunden (häufig mehr als 5 h) in Anspruch. Vielfach erstreckt sich die Gesamtdauer der thermischen Behandlung auf mehr als 10 h. Meist werden im Rahmen der thermischen Behandlung der Formkörper V Behandlungsdauern von 45 h bzw. 25 h nicht überschritten. Oft liegt die Gesamtbehandlungsdauer unterhalb von 20 h. Grundsätzlich kann die thermische Behandlung bei höheren Temperaturen über eine kürzere Behandlungsdauer oder bei nicht zu hohen Temperaturen während einer längeren Behandlungsdauer durchgeführt werden. In einer erfindungsgemäß vorteilhaften Ausführungsform der thermischen Behandlung der Formkörper V werden 465°C nicht überschritten und die Behandlungsdauer im Temperaturfenster $\geq$ 440°C erstreckt sich auf > 10 bis 20 h.

In allen Beispielen und Vergleichsbeispielen erfolgt die Endkalzination bei Endkalzinationstemperaturen $\leq$ 490 °C, insbesondere $\leq$ 475 °C. Dies ist unter anderem darauf zurückzuführen, dass im kommerziellen Maßstab im höher liegenden Endkalzinationstemperaturbereich der Energieaufwand, und, bei einer Endkalzination im Beisein von Luft, die Gefahr von Graphitbränden überproportional zunehmen. Ferner kann die Langzeitstabilität der resultierenden geometrischen Katalysatorformkörper K unter einer zu hohen Endkalzinationstemperatur und/oder Endkalzinationsdauer leiden. In einer weiteren erfindungsgemäß vorteilhaften Ausführungsform der thermischen Behandlung der Formkörper V werden 465 °C (nicht jedoch 490 °C) überschritten und die Behandlungsdauer im Temperaturfenster von $\geq$ 465 °C erstreckt sich auf 2 bis 10 h.

Grundsätzlich können alle in dieser Schrift angesprochenen oder ausgeführten Beispiele und Vergleichsbeispiele unter ansonsten unveränderten Bedingungen aber auch bei einer Endkalzinationstemperatur von 430 °C, oder 432 °C, oder 434 °C, oder 436 °C, oder 438 °C, oder 440 °C, oder 442 °C, oder 444 °C, oder 446 °C, oder 448 °C, oder 450 °C, oder 452 °C, oder 454 °C, oder 456 °C, oder 458 °C, oder 460 °C, oder 462 °C, oder 464 °C, oder 466 °C, oder 468 °C, oder 470 °C, oder 472 °C, oder 474 °C oder 475 °C durchgeführt werden.

Die Endkalzination in allen Ausführungsbeispielen B1 bis B13 sowie in allen Vergleichsbeispielen V1 bis V16 (einschließlich der nachfolgenden Verwendung als Katalysatoren für die Propenpartialoxidation) kann aber auch unter ansonsten unveränderten Bedingungen während einer auf 9, oder 8, oder 7, oder 6, oder 5, oder 4, oder 3, oder 2, oder 1 h verkürzten Endkalzinationsdauer bei einer jeweils um 2, oder 4, oder 6, oder 8, oder 10, oder 12, oder 14, oder 16, oder 20 °C erhöhten Endkalzinationstemperatur durchgeführt werden.

Die thermische Behandlung (auch die Phase 1 (auch Zersetzungsphase genannt)) der Formkörper V kann sowohl unter Inertgas als auch unter einer oxidativen Atmosphäre wie z.B. Luft (oder ein sonstiges Gemisch aus Inertgas und Sauerstoff) sowie unter reduzierender Atmosphäre (z. B. ein Gemisch aus Inertgas, $NH_3$, CO und/oder $H_2$ oder unter Methan, Acrolein, Methacrolein) erfolgen. Selbstredend kann die thermische Behandlung auch unter Vakuum ausgeführt werden. Auch kann die Kalzinationsatmosphäre über die Kalzinationsdauer variabel gestaltet werden. Erfindungsgemäß bevorzugt erfolgt die thermische Behandlung der Formkörper V in einer oxidierenden Atmosphäre. Anwendungstechnisch zweckmäßig besteht sie überwiegend aus stehender oder bewegter Luft. Sie kann jedoch ebenso (z. B. im Fall aller Beispiele und Vergleichsbeispiele) aus einem stehenden oder bewegten Gemisch aus 25 Vol.-% $N_2$ und 75 Vol.-% Luft, oder 50 Vol.-% $N_2$ und 50 Vol.-% Luft, oder 75 Vol.-% $N_2$ und 25 Vol.-% Luft, oder 100 Vol.-% $N_2$ bestehen (insbesondere im Fall der in dieser Schrift angesprochenen oder ausgeführten Beispiele und Vergleichsbeispiele).

Prinzipiell kann die thermische Behandlung der Formkörper V in den unterschiedlichsten Ofentypen wie z.B. beheizbare Umluftkammern (Umluftöfen), Hordenöfen, Drehrohröfen, Bandkalzinierern oder Schachtöfen durchgeführt werden. Erfindungsgemäß vorteilhaft erfolgt die thermische Behandlung der Formkörper V in einer Bandkalzinierungsvorrichtung, wie sie die DE-A 10046957 und die WO 02/24620 empfehlen. Eine Heißpunktausbildung innerhalb des Kalzinationsgutes wird dabei weitestgehend dadurch vermieden, dass mit Hilfe von Ventilatoren durch ein das Kalzinationsgut tragendes gasdurchlässiges Förderband erhöhte Volumenströme an Kalzinationsatmosphäre durch das Kalzinationsgut gefördert werden.

Die thermische Behandlung der Formkörper V unterhalb von 350°C verfolgt in der Regel das Ziel der thermischen Zersetzung der in den Formkörpern V enthaltenen Quellen der Elemente (der elementaren Konstituenten) der angestrebten Multielementoxid-I-Aktivmasse der Katalysatorformkörper K sowie von gegebenenfalls mitverwendeten Formgebungshilfsmitteln. Häufig erfolgt diese Zersetzungsphase im Rahmen des Aufheizens des Kalzinationsgutes auf Temperaturen $\geq$ 350°C.

Grundsätzlich kann die thermische Behandlung wie in der US 2005/0131253 beschrieben erfolgen.

In typischer Weise betragen die Seitendruckfestigkeiten von wie beschrieben erfindungsgemäß erhältlichen ringförmigen Vollkatalysatorformkörpern K 5 bis 13 N, häufig 8 bis 11 N.

Erfindungsgemäß hergestellte Vollkatalysatorformkörper K müssen nicht in notwendiger Weise als solche als Katalysatoren für heterogen katalysierte partielle Gasphasenoxidationen von 3 bis 6 C-Atome aufweisenden Alkanen, Alkanolen, Alkenen und/oder Alkenalen eingesetzt werden. Vielmehr können sie auch einer Mahlung unterworfen werden und von dem dabei resultierenden feinteiligen Material (gegebenenfalls nach Klassieren des dabei resultierenden feinteiligen Materials) mit Hilfe eines geeigneten flüssigen Bindemittels (z.B. Wasser) auf die Oberfläche eines geeigneten, z.B. kugel- oder ringförmigen, Trägerkörpers (geometrischen Trägerformkörpers) aufgebracht werden (z. B. unter Anwendung des in der DE-A 2909671, sowie DE-A 100 51 419 offenbarten Verfahrensprinzips). Nach Trocknung oder unmittelbar nach Auftragung der Aktivmassenschale auf den Trägerkörper kann der resultierende Schalenkatalysator als Katalysator für vorgenannte heterogen katalysierte Gasphasenpartialoxidationen eingesetzt werden, wie es z.B. die WO 02/49757 und die DE-A 10122027 für ähnliche Aktivmassen beschreiben.

Als Trägermaterialien können bei vorstehender Verfahrensweise übliche poröse oder unporöse Aluminiumoxide, Siliciumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit (z.B. die bereits erwähnten Kugeln oder Ringe) bevorzugt werden. Von besonderem Vorteil ist die Verwendung von im Wesentlichen unporösen, oberflächenrauhen Ringen aus Steatit, deren Längstausdehnung (längste direkte geradlinige Verbindungslinie zweier auf der Oberfläche des Trägerformkörpers befindlicher Punkte) typisch 2 bis 12 mm, häufig 4 bis 10 mm beträgt (vgl. auch die DE-A 4442346). Vorgenannte Längstausdehnungen kommen auch für sonstige Trägerformkörper wie z. B. Kugeln, Vollzylinder und sonstige Ringe in Betracht.

Die Schichtdicke der auf den Trägerformkörper aufgebrachten Aktivmassenschale (Pulvermasse) wird zweckmäßigerweise im Bereich 10 bis 1000 $\mu$m, bevorzugt im Bereich 100 bis 700 $\mu$m und besonders bevorzugt im Bereich 300 bis 500 $\mu$m liegend gewählt. Mögliche Schalendicken sind auch 10 bis 500 $\mu$m oder 200 bis 300 $\mu$m. Häufig liegt die Oberflächenrauhigkeit $R_z$ des Trägerformkörpers im Bereich von 40 bis 200 $\mu$m, vielfach im Bereich von 40 bis 100 $\mu$m (bestimmt gemäß DIN 4768 Blatt 1 mit einem "Hommel Tester für DIN-ISO Oberflächenmaßgrößen" der Fa. Hommelwerke, DE). Zweckmäßigerweise ist das Trägermaterial unporös (Gesamtvolumen der Poren auf das Volumen des Trägerkörpers bezogen $\leq$ 1 Vol.-%).

Grundsätzlich kann die Formung (Verdichtung) der feinteiligen Ausgangsmasse A3 zu einem Formkörper V auch dadurch erfolgen, dass man die feinteilige Ausgangsmasse A3 mit Hilfe eines geeigneten flüssigen Bindemittels auf die Oberfläche eines wie vorstehend beschriebenen geometrischen Trägerformkörpers aufbringt. Nach Trocknung können die dabei resultierenden Vorläuferformkörper V in erfindungsgemäßer Weise unter Erhalt von erfindungsgemäßen Schalenkatalysatorformkörpern K thermisch behandelt werden.

Auch kann durch Mahlen von erfindungsgemäß hergestellten Vollkatalysatorformkörpern K erzeugtes Aktivmassenpulver als solches im Wirbel- bzw. Fließbett für die in diesen Schriften angesprochenen heterogen katalysierten partiellen Gasphasenoxidationen eingesetzt werden.

Ferner ist es im Hinblick auf eine möglichst ausgeprägte Langzeitstabilität erfindungsgemäßer geometrischer Katalysatorformkörper K (insbesondere bei ihrer Verwendung als Katalysatoren für die heterogen katalysierte partielle Gasphasenoxidation von Propen zu Acrolein oder von iso-Buten zu Methacrolein) vorteilhaft, wenn zusätzlich zur erfindungsgemäßen Bedingung für den stöchiometrischen Koeffizienten c der Betrag F (der Stabilitätswert F des geometrischen Katalysatorformkörpers K) des Produktes

$$( d_{50}^{A1} )^{0,7} \quad \bullet \quad ( d_{90}^{A2} )^{1,5} \quad \bullet \quad (p/2)^{-1}$$

$\geq 820$ beträgt, wobei die beiden Durchmesser $d_{50}^{A1}$, $d_{90}^{A2}$ zur Produktbildung in der Längeneinheit $\mu$m einzusetzen sind.

Erfindungsgemäß bevorzugt beträgt F $\geq$ 830, vorteilhaft $\geq$ 840 und noch besser $\geq$ 850.

Besonders vorteilhaft beträgt F $\geq$ 870 oder $\geq$ 900, und besonders vorteilhaft beträgt F $\geq$ 950 oder $\geq$ 1000. Ganz besonders vorteilhaft beträgt F $\geq$ 1050, oder $\geq$ 1100 bzw. $\geq$ 1500, oder $\geq$ 2000.

Unter Einbezug der Zielvorgabe einer befriedigenden Anfangsselektivität der Zielproduktbildung bereits bei Inbetriebnahme des Katalysatorbetts beträgt F mit Vorzug $\leq$ 5000 oder $\leq$ 4000, teilweise $\leq$ 2500, oder $\leq$ 2400 oder $\leq$ 2200. Günstige Werte für F sind auch solche, die $\leq$ 2000, oder $\leq$ 1800, oder $\leq$ 1600 bzw. $\leq$ 1500 betragen.

D. h., erfindungsgemäß vorteilhafte Werte für F betragen 5000 $\geq$ F $\geq$ 1000, oder 4000 $\geq$ F $\geq$ 2000, oder 2500 $\geq$ F $\geq$ 850, oder 2450 $\geq$ F $\geq$ 900, oder 2400 $\geq$ F $\geq$ 950.

Erfindungsgemäß besonders vorteilhafte Werte für F betragen 1900 $\geq$ F $\geq$ 1000, oder 1800 $\geq$ F $\geq$ 1050.

Erfindungsgemäß ganz besonders vorteilhafte Werte für F betragen 1700 $\geq$ F $\geq$ 1100, oder 1500 $\geq$ F $\geq$ 1150.

Die Langzeitstabilität erfindungsgemäßer geometrischer Katalysatorformkörper K oder eines diese enthaltenden Katalysatorbetts ist dann besonders ausgeprägt, wenn die im Rahmen der Formierung resultierende Aktivität im weitergehenden Betrieb der Partialoxidation sich möglichst langsam verringert. Periodisch wiederholte Regenerierverfahren wie sie z. B. die Schriften WO 2005/42459 und WO 2005/49200 empfehlen können die Katalysatorlebensdauer zusätzlich verlängern.

Erhöhte stöchiometrische Koeffizienten c können die Langzeitstabilität erfindungsgemäßer geometrischer Katalysatorformkörper K mindern. Erhöhte Werte für $d_{50}^{A1}$, $d_{50}^{A2}$ sowie $d_{90}^{A2}$ und verminderte stöchiometrische Koeffizienten p wirken entgegengesetzt.

Durch die erfindungsgemäße Bi-Dotierung können $d_{50}^{A1}$, $d_{50}^{A2}$ sowie $d_{90}^{A2}$ vergleichsweise größere und der stöchiometrische Koeffizient p vergleichsweise kleinere Werte annehmen, ohne nennenswerte Einbußen bei Anfangsaktivität und Anfangsselektivität zu bedingen. Insgesamt sind so bei sowohl befriedigender Anfangsaktivität als auch bei befriedigender Anfangsselektivität vergleichsweise erhöhte Werte für F und F* realisierbar.

Ist beim erfindungsgemäßen Verfahren, zusätzlich zur Bedingung für den Betrag F, für den Betrag F* des Produktes (die Partikeldurchmesser $d_{50}^{A1}$ (der feinteiligen Ausgangsmasse A1), $d_{50}^{A2}$ (der feinteiligen Ausgangsmasse A2) sind dabei wieder in der Längeneinheit $\mu$m angegeben)

$$( d_{50}^{A1} )^{0,7} \quad \bullet \quad ( d_{50}^{A2} )^{0,7} \quad \bullet \quad (p/2)^{-1}$$

die Bedingung F* $\geq$ 15 (vorzugsweise $\geq$ 18), besonders bevorzugt 35 bzw. 25 $\geq$ F* $\geq$ 18 erfüllt, so ist dies über das bisher bereits Gesagte hinaus von zusätzlichem Vorteil.

Die erfindungsgemäß erhältlichen Katalysatorformkörper K eignen sich als Katalysatoren für alle heterogen katalysierten partiellen Gasphasenoxidationen, für die in dieser Schrift bereits die geometrischen Katalysatorformkörper K[S] als geeignet erwähnt worden sind. Besonders geeignet sind erfindungsgemäß erhältliche geometrische Katalysatorformkörper K jedoch als Katalysatoren für die Partialoxidationen von Propen zu Acrolein und von iso-Buten und/oder tert. Butanol zu Methacrolein. Dies gilt insbesondere für erfindungsgemäße ringförmige Vollkatalysatorformkörper K. Die Partialoxidation kann dabei z.B. wie in den Schriften DE-A 102007004961, WO 02/49757, WO 02/24620, Deutsche Anmeldung Nr. 102008040093.9, WO 2005/030393, EP-A 575 897, WO 2007/082827, WO 2005/113127, WO 2005/047224, WO 2005/042459 und WO 2007/017431 beschrieben durchgeführt werden.

Dabei erweisen sich die in dieser Schrift individualisiert hervorgehobenen Ringgeometrien der wie beschrieben erhältlichen ringförmigen Vollkatalysatoren insbesondere auch dann als vorteilhaft, wenn die Belastung der Katalysatorbeschickung mit im Reaktionsgasausgangsgemisch enthaltenem Propen, iso-Buten und/oder tert. Butanol (bzw. dessen Methylether) $\geq$ 130 Nl/l Katalysatorbeschickung $\cdot$ h beträgt (Vor- und/oder Nachschüttungen aus reinem Inertmaterial werden bei Belastungsbetrachtungen in dieser Schrift nicht als zur Katalysatorbeschickung gehörig betrachtet).

Die Vorteilhaftigkeit von wie beschrieben erhältlichen ringförmigen Vollkatalysatorformkörpern K liegt aber auch dann vor, wenn die vorgenannte Belastung der Katalysatorbeschickung ≥ 140 Nl/l·h, oder ≥ 150 Nl/l·h, oder ≥ 160 Nl/l·h beträgt. Im Normalfall wird die vorgenannte Belastung der Katalysatorbeschickung ≤ 600 Nl/l·h, häufig ≤ 500 Nl/l·h, vielfach ≤ 400 Nl/l·h oder ≤ 350 Nl/l·h betragen. Belastungen im Bereich von 160 Nl/l·h bis 300 bzw. 250 oder 200 Nl/l·h sind besonders zweckmäßig. Selbstverständlich können die wie beschrieben erhältlichen ringförmigen Vollkatalysatorformkörper K als Katalysatoren für die Partialoxidation von Propen zu Acrolein bzw. von iso-Buten und/oder tert. Butanol (bzw. dessen Methylether) zu Methacrolein auch bei Belastungen der Katalysatorbeschickung mit der partiell zu oxidierenden Ausgangsverbindung von ≤ 130 Nl/l·h, oder ≤ 120 Nl/l·h, oder ≤ 110 Nl/l·h, in erfindungsgemäß vorteilhafter Weise betrieben werden. In der Regel wird diese Belastung jedoch bei Werten ≥ 60 Nl/l·h, oder ≥ 70 Nl/l·h, oder ≥ 80 Nl/l·h liegen.

Prinzipiell kann die Belastung der Katalysatorbeschickung mit der partiell zu oxidierenden Ausgangsverbindung (Propen, iso-Buten und/oder tert. Butanol (bzw. dessen Methylether)) über zwei Stellschrauben eingestellt werden:

a) die Belastung der Katalysatorbeschickung mit Reaktionsgasausgangsgemisch (das Reaktionsgasgemisch, das dem Katalysatorfestbett zugeführt wird), und/oder
b) den Gehalt des Reaktionsgasausgangsgemischs mit der partiell zu oxidierenden Ausgangsverbindung.

Die erfindungsgemäß erhältlichen z. B. ringförmigen Vollkatalysatorformkörper K eignen sich insbesondere auch dann, wenn bei oberhalb von 130 Nl/l·h liegenden Belastungen der Katalysatorbeschickung mit der partiell zu oxidierenden organischen Verbindung die Belastungseinstellung vor allem über die vorgenannte Stellschraube a) erfolgt.

Der Propenanteil (iso-Butenanteil bzw. tert. Butanolanteil (bzw. der Methyletheranteil)) im Reaktionsgasausgangsgemisch wird im Regelfall (d.h. im wesentlichen unabhängig von der Belastung) 4 bis 20 Vol.-%, häufig 5 bis 15 Vol.-%, oder 5 bis 12 Vol.-%, oder 5 bis 8 Vol.-% betragen (jeweils bezogen auf das Gesamtvolumen des Reaktionsgasausgangsgemischs).

Häufig wird man das Gasphasenpartialoxidationsverfahren der mit den wie beschrieben erhältlichen z. B. ringförmigen Vollkatalysatorformkörper K (oder sonstigen geometrischen Katalysatorformkörpern K) katalysierten Partialoxidation (im wesentlichen unabhängig von der Belastung) bei einem partiell zu oxidierende (organische) Verbindung (z.B. Propen): Sauerstoff: indifferente Gase (einschließlich Wasserdampf) Volumenverhältnis im Reaktionsgasausgangsgemisch von 1:(1,0 bis 3,0):(5 bis 25), vorzugsweise 1:(1,5 bis 2,3):(10 bis 20), durchführen.

Unter indifferenten Gasen (oder auch Inertgasen) werden dabei solche Gase verstanden, die im Verlauf der Partialoxidation zu wenigstens 95 mol-%, vorzugsweise zu wenigstens 98 mol-% chemisch unverändert erhalten bleiben.

Bei den vorstehend beschriebenen Reaktionsgasausgangsgemischen kann das indifferente Gas zu ≥ 20 Vol.-%, oder zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Bei Belastungen der Katalysatorbeschickung mit der partiell zu oxidierenden organischen Verbindung von ≥ 150 Nl/l·h ist jedoch die Mitverwendung von inerten Verdünnungsgasen wie Propan, Ethan, Methan, Pentan, Butan, $CO_2$, CO, Wasserdampf und/oder Edelgasen für das Rektionsgasausgangsgemisch empfehlenswert. Generell können diese inerten Gase und ihre Gemische aber auch bereits bei geringeren Belastungen der Katalysatorbeschickung mit der partiell zu oxidierenden organischen Verbindung eingesetzt werden. Auch kann Kreisgas als Verdünnungsgas mitverwendet werden. Unter Kreisgas wird das Restgas verstanden, das verbleibt, wenn man aus dem Produktgasgemisch der Partialoxidation die Zielverbindung im Wesentlichen selektiv abtrennt. Dabei ist zu berücksichtigen, dass die Partialoxidationen zu Acrolein oder Methacrolein mit den erfindungsgemäß erhältlichen z. B. ringförmigen Katalysatorformkörpern K nur die erste Stufe einer zweistufigen Partialoxidation zu Acrylsäure oder Methacrylsäure als den eigentlichen Zielverbindungen sein können, so dass die Kreisgasbildung dann meist erst nach der zweiten Stufe erfolgt. Im Rahmen einer solchen zweistufigen Partialoxidation wird in der Regel das Produktgasgemisch der ersten Stufe als solches, gegebenenfalls nach Abkühlung und/oder Sekundärsauerstoffzugabe, der zweiten Partialoxidationsstufe zugeführt.

Bei der Partialoxidation von Propen zu Acrolein, unter Anwendung der wie beschrieben erhältlichen z. B. ringförmigen Katalysatorformkörper K, kann eine typische Zusammensetzung des Reaktionsgasausgangsgemischs gemessen am Reaktoreingang (unabhängig von der gewählten Belastung) z.B. die nachfolgenden Komponenten enthalten:

| | |
|---|---|
| 6 bis 6,5 Vol.-% | Propen, |
| 1 bis 3,5 Vol.-% | $H_2O$, |
| 0,2 bis 0,5 Vol.-% | CO, |
| 0,6 bis 1,2 Vol.-% | $CO_2$, |
| 0,015 bis 0,04 Vol.-% | Acrolein, |
| 10,4 bis 11,3 Vol.-% | $O_2$, und |

als Restmenge ad 100 Vol.-% molekularer Stickstoff;
oder:

|  |  |
|---|---|
| 5,6 Vol.-% | Propen, |
| 10,2 Vol.-% | Sauerstoff, |
| 1,2 Vol.-% | $CO_x$, |
| 81,3 Vol.-% | $N_2$, und |
| 1,4 Vol.-% | $H_2O$. |

Erstere Zusammensetzungen eignen sich insbesondere bei Propenbelastungen von $\geq$ 130 Nl/l·h und letztere Zusammensetzung insbesondere bei Propenbelastungen < 130 Nl/l·h, insbesondere $\leq$ 100 Nl/l·h des Katalysatorfestbetts.

Als alternative Zusammensetzungen des Reaktionsgasausgangsgemischs kommen (unabhängig von der gewählten Belastung) solche in Betracht, die nachfolgende Komponentengehalte aufweisen:

|  |  |
|---|---|
| 4 bis 25 Vol.-% | Propen, |
| 6 bis 70 Vol.-% | Propan, |
| 5 bis 60 Vol.-% | $H_2O$, |
| 8 bis 65 Vol.-% | $O_2$, und |
| 0,3 bis 20 Vol.-% | $H_2$; |

oder

|  |  |
|---|---|
| 4 bis 25 Vol.-% | Propen, |
| 6 bis 70 Vol.-% | Propan, |
| 0 bis 60 Vol.-% | $H_2O$, |
| 8 bis 16 Vol.-% | $O_2$, |
| 0 bis 20 Vol.-% | $H_2$, |
| 0 bis 0,5 Vol.-% | CO, |
| 0 bis 1,2 Vol.-% | $CO_2$, |
| 0 bis 0,04 Vol.-% | Acrolein, |

und als Restmenge ad 100 Vol.-% im Wesentlichen $N_2$;
oder

|  |  |
|---|---|
| 50 bis 80 Vol.-% | Propan, |
| 0,1 bis 20 Vol.-% | Propen, |
| 0 bis 10 Vol.-% | $H_2$, |
| 0 bis 20 Vol.-% | $N_2$, |
| 5 bis 15 Vol.-% | $H_2O$, und |

soviel molekularer Sauerstoff, dass das molare Verhältnis von Sauerstoffgehalt zu Propengehalt 1,5 bis 2,5 beträgt.
oder

|  |  |
|---|---|
| 6 bis 9 Vol.-% | Propen, |
| 8 bis 18 Vol.-% | molekularer Sauerstoff, |
| 6 bis 30 Vol.-% | Propan, und |
| 32 bis 72 Vol.-% | molekularer Stickstoff. |

Das Reaktionsgasausgangsgemisch kann aber auch wie folgt zusammengesetzt sein:

|  |  |
|---|---|
| 4 bis 15 Vol.-% | Propen, |

(fortgesetzt)

| 1,5 bis 30 Vol.-% | (häufig 6 bis 15 Vol.-%) Wasser, |
| ≥ 0 bis 10 Vol.-% | (vorzugsweise ≥ 0 bis 5 Vol.-%) |
| | von Propen, Wasser, Sauerstoff und Stickstoff verschiedene Bestandteile, soviel molekularer Sauerstoff, dass das molare Verhältnis von enthaltenem molekularem Sauerstoff zu enthaltenem molekularem Propen 1,5 bis 2,5 beträgt, und als Restmenge bis zur 100 Vol.-% Gesamtmenge molekularer Stickstoff. |

Eine andere mögliche Reaktionsgasausgangsgemischzusammensetzung kann enthalten:

| 6,0 Vol.-% | Propen, |
| 60 Vol.-% | Luft, und |
| 34 Vol.-% | $H_2O$. |

Alternativ können auch Reaktionsgasausgangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 verwendet werden.

Auch eignen sich die wie beschrieben erhältlichen z. B. ringförmigen Katalysatorformkörper K für die Verfahren der DE-A 10246119 bzw. DE-A 10245585.

Weitere erfindungsgemäß geeignete Reaktionsgasausgangsgemische können im nachfolgenden Zusammensetzungsraster liegen:

| 7 bis 11 Vol.-% | Propen, |
| 6 bis 12 Vol.-% | Wasser, |
| ≥ 0 bis 5 Vol.-% | von Propen, Wasser, Sauerstoff und Stickstoff verschiedener Bestandteile, soviel molekularer Sauerstoff, dass das molare Verhältnis von enthaltenem molekularem Sauerstoff zu enthaltenem Propen 1,6 bis 2,2 beträgt, und |
| | als Restmenge bis zur 100 Vol.-% Gesamtmenge molekularer Stickstoff. |

Im Fall von Methacrolein als Zielverbindung kann das Reaktionsgasausgangsgemisch insbesondere auch wie in der DE-A 44 07 020 beschrieben zusammengesetzt sein.

Die Reaktionstemperatur für die Propenpartialoxidation liegt bei Verwendung der wie beschrieben erhältlichen z. B. ringförmigen Katalysatorformkörper K häufig bei 300 bis 380 °C. Das gleiche trifft im Fall von Methacrolein als Zielverbindung zu.

Der Reaktionsdruck liegt für die vorgenannten Partialoxidationen in der Regel bei 0,5 bzw. 1,5 bis 3 bzw. 4 bar (gemeint sind in dieser Schrift, soweit nicht ausdrücklich anders erwähnt ist, stets Absolutdrucke).

Die Gesamtbelastung der Katalysatorbeschickung mit Reaktionsgasausgangsgemisch beläuft sich bei den vorgenannten Partialoxidationen typisch auf 1000 bis 10000 Nl/l·h, meist auf 1500 bis 5000 Nl/l·h und oft auf 2000 bis 4000 Nl/l·h.

Als im Reaktionsgasausgangsgemisch zu verwendendes Propen kommen vor allem polymer grade Propen und chemical grade Propen in Betracht, wie es z. B. die DE-A 102 32 748 beschreibt.

Als Sauerstoffquelle wird normalerweise Luft eingesetzt.

Die Partialoxidation in Anwendung der wie beschrieben erhältlichen z. B. ringförmigen Katalysatorformkörper K kann im einfachsten Fall z. B. in einem Einzonen-Vielkontaktrohr-Festbettreaktor durchgeführt werden, wie ihn die DE-A 44 31 957, die EP-A 700 714 und die EP-A 700 893 beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Eine typische Kontaktrohrlänge beläuft sich z. B. auf 3,20 m. Anwendungstechnisch zweckmäßig beläuft sich die im Rohrbündelbehälter untergebrachte Anzahl an Kontaktrohren auf wenigstens 1000, vorzugsweise auf wenigstens 5000. Häufig beträgt die Anzahl der im Reaktionsbehälter untergebrachten Kontaktrohre 15000 bis 35000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Die Partialoxidation kann aber auch in einem Mehrzonen (z. B. "Zwei-Zonen")-Vielkontaktrohr-Festbettreaktor durchgeführt werden, wie es die DE-A 199 10 506, die DE-A 103 13 213, die DE-A 103 13 208 und die EP-A 1 106 598 insbesondere bei erhöhten Belastungen der Katalysatorbeschickung mit der partiell zu oxidierenden organischen Verbindung empfehlen. Eine typische Kontaktrohrlänge im Fall eines Zweizonen-Vielkontaktrohr-Festbettreaktors beträgt 3,50 m. Alles andere gilt im Wesentlichen wie beim Einzonen-Vielkontaktrohr-Festbettreaktor beschrieben. Um die Kontaktrohre, innerhalb derer sich die Katalysatorbeschickung befindet, wird in jeder Temperierzone ein Wärmeaustauschmittel geführt. Als solche eignen sich z. B. Schmelzen von Salzen wie Kaliumnitrat, Kaliumnitrit, Natriumnitrit und/oder Natriumnitrat, oder von niedriger schmelzenden Metallen wie Natrium, Quecksilber sowie Legierungen verschiedener Metalle. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperaturzone bis zur Austrittsstelle aus der Temperaturzone um 0 bis 15 °C, häufig 1 bis 10 °C, oder 2 bis 8 °C, oder 3 bis 6 °C ansteigt.

Die Eingangstemperatur des Wärmeaustauschmittels, das, über die jeweilige Temperierzone betrachtet, im Gleichstrom oder im Gegenstrom zum Reaktionsgasgemisch geführt werden kann, wird vorzugsweise wie in den Schriften EP-A 1 106 598, DE-A 199 48 523, DE-A 199 48 248, DE-A 103 13 209, EP-A 700 714, DE-A 103 13 208, DE-A 103 13 213, WO 00/53557, WO 00/53558, WO 01/36364, WO 00/53557 sowie den anderen in dieser Schrift als Stand der Technik zitierten Schriften empfohlen gewählt. Innerhalb der Temperierzone wird das Wärmeaustauschmittel bevorzugt mäanderförmig geführt. In der Regel verfügt der Vielkontaktrohr-Festbettreaktor zusätzlich über Thermorohre zur Bestimmung der Gastemperatur im Katalysatorbett. In zweckmäßiger Weise wird der Innendurchmesser der Thermorohre und der Durchmesser der innenliegenden Aufnahmehülse für das Thermoelement so gewählt, dass das Verhältnis von Reaktionswärme entwickelndem Volumen zu Wärme abführender Oberfläche bei Thermorohren und Arbeitsrohren gleich oder nur geringfügig unterschiedlich ist.

Der Druckverlust sollte bei Arbeitsrohren und Thermorohren, bezogen auf gleiche GHSV, gleich sein. Ein Druckverlustausgleich beim Thermorohr kann durch Zusatz von versplittetem Katalysator zu den Katalysatorformkörpern erfolgen. Dieser Ausgleich erfolgt zweckmäßig über die gesamte Thermorohrlänge homogen.

Zur Bereitung der Katalysatorbeschickung in den Kontaktrohren können, wie bereits erwähnt, nur wie beschrieben erhältliche z. B. ringförmige Katalysatorformkörper K oder z. B. auch weitgehend homogene Gemische aus wie beschrieben erhältlichen z. B. ringförmigen Katalysatorformkörpern K und keine Aktivmasse aufweisenden, sich bezüglich der heterogen katalysierten partiellen Gasphasenoxidation im wesentlichen inert verhaltenden Formkörpern verwendet werden. Als Materialien für solche inerten Formkörper kommen z. B. poröse oder unporöse Aluminiumoxide, Siliciumdioxid, Zirkondioxid, Siliciumcarbid, Silikate wie Magnesium- oder Aluminiumsilikat und/oder Steatit (z.B. vom Typ C220 der Fa. CeramTec, DE) in Betracht.

Die Geometrie solcher inerter Verdünnungsformkörper kann im Prinzip beliebig sein. D.h., es können beispielsweise Kugeln, Polygone, Vollzylinder oder auch, wie z. B. im Fall ringförmiger Katalysatorformkörper K, Ringe sein. Häufig wird man als inerte Verdünnungsformkörper solche wählen, deren Geometrie derjenigen der mit ihnen zu verdünnenden Katalysatorformkörper K entspricht. Längs der Katalysatorbeschickung können aber auch die Geometrie des Katalysatorformkörpers K gewechselt oder Katalysatorformkörper K unterschiedlicher Geometrie in weitgehend homogener Abmischung eingesetzt werden. In einer weniger bevorzugten Vorgehensweise kann auch die Aktivmasse des Katalysatorformkörpers K längs der Katalysatorbeschickung verändert werden.

Ganz generell wird, wie bereits erwähnt, die Katalysatorbeschickung mit Vorteil so gestaltet, dass die volumenspezifische (d. h., die auf die Einheit des Volumens normierte) Aktivität in Strömungsrichtung des Reaktionsgasgemischs entweder konstant bleibt oder zunimmt (kontinuierlich, sprunghaft oder stufenförmig).

Eine Verringerung der volumenspezifischen Aktivität kann in einfacher Weise z. B. dadurch erzielt werden, dass man eine Grundmenge von erfindungsgemäß einheitlich hergestellten z. B. ringförmigen Katalysatorformkörpern K mit inerten Verdünnungsformkörpern homogen verdünnt. Je höher der Anteil der Verdünnungsformkörper gewählt wird, desto geringer ist die in einem bestimmten Volumen der Beschickung enthaltene Aktivmasse bzw. Katalysatoraktivität. Eine Verringerung kann aber auch dadurch erzielt werden, dass man die Geometrie der erfindungsgemäß erhältlichen Katalysatorformkörper K so verändert, dass die in der Einheit des Reaktionsrohrinnenvolumens enthaltene Aktivmassenmenge kleiner wird.

Für die heterogen katalysierten Gasphasenpartialoxidationen mit wie beschrieben erhältlichen ringförmigen Vollkatalysatorformkörpern K wird die Katalysatorbeschickung vorzugsweise entweder auf der gesamten Länge einheitlich mit nur einem Typ Vollkatalysatorringformkörper K gestaltet oder wie folgt strukturiert. Am Reaktoreingang wird auf einer Länge von 10 bis 60 %, bevorzugt 10 bis 50 %, besonders bevorzugt 20 bis 40 % und ganz besonders bevorzugt 25 bis 35 % (d. h., z. B. auf einer Länge von 0,70 bis 1,50 m, bevorzugt 0,90 bis 1,20 m), jeweils der Gesamtlänge der Katalysatorbeschickung, ein im Wesentlichen homogenes Gemisch aus erfindungsgemäß erhältlichem ringförmigem Vollkatalysatorformkörper K und inertem Verdünnungsformkörper (wobei beide vorzugsweise im Wesentlichen die gleiche Geometrie aufweisen) platziert, wobei der Gewichtsanteil der Verdünnungsformkörper (die Massendichten von Katalysatorformkörpern K und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d. h., z. B. auf einer Länge von 1,00 bis 3,00 m oder von 1,00 bis 2,70 m, bevorzugt 1,40 bis 3,00 m, oder 2,00 bis 3,00 m) entweder eine nur in geringerem Umfang (als im ersten Abschnitt) verdünnte Schüttung des wie beschrieben erhältlichen ringförmigen Vollkatalysatorformkörpers K, oder, ganz besonders bevorzugt, eine alleinige (unverdünnte) Schüttung desselben ringförmigen Vollkatalysatorformkörper K, der auch im ersten Abschnitt verwendet worden ist. Natürlich kann über die gesamte Beschickung auch eine konstante Verdünnung gewählt werden. Auch kann im ersten Abschnitt nur mit einem erfindungsgemäß erhältlichen ringförmigen Vollkatalysatorformkörper K von geringer, auf seinen Raumbedarf bezogener, Aktivmassendichte und im zweiten Abschnitt mit einem erfindungsgemäß erhältlichen ringförmigen Vollkatalysatorformkörper K mit hoher, auf seinen Raumbedarf bezogener, Aktivmassendichte beschickt werden (z.B. 6,5 mm x 3 mm x 4,5 mm [A x H x I] im ersten Abschnitt, und 5 x 2 x 2 mm im zweiten Abschnitt).

Insgesamt werden bei einer mit den wie beschrieben erhältlichen z. B. ringförmigen Katalysatorformkörpern K als Katalysatoren durchgeführten Partialoxidation zur Herstellung von Acrolein oder Methacrolein die Katalysatorbeschickung, das Reaktionsgasausgangsgemisch, die Belastung und die Reaktionstemperatur in der Regel so gewählt, dass beim einmaligen Durchgang des Reaktionsgasgemischs durch die Katalysatorbeschickung ein Umsatz der partiell zu oxidierenden organischen Verbindung (Propen, iso-Buten, tert.-Butanol bzw. dessen Methylether) von wenigstens 90 mol-%, oder wenigstens 92 mol-%, vorzugsweise von wenigstens 94 mol-% resultiert. Die Selektivität der Acrolein- bzw. Methacroleinbildung wird dabei regelmäßig $\geq$ 80 mol-%, bzw. $\geq$ 85 mol-% betragen. In natürlicher Weise werden dabei möglichst geringe Heißpunkttemperaturen angestrebt.

Abschließend sei festgehalten, dass wie beschrieben erhältliche ringförmige Vollkatalysatorformkörper K auch ein vorteilhaftes Bruchverhalten bei der Reaktorbefüllung aufweisen.

Die Inbetriebnahme einer(s) frischen, erfindungsgemäß erhältliche geometrische Katalysatorformkörper K enthaltenden, Katalysatorbeschickung (Katalysatorfestbetts) kann z. B. wie in der DE-A 103 37 788 beschrieben erfolgen.

Die Formierung erfindungsgemäßer geometrischer Katalysatorformkörper K kann dadurch beschleunigt werden, dass man sie bei im wesentlich gleichbleibendem Umsatz unter erhöhter Belastung der Katalysatorbeschickung mit Reaktionsgasausgangsgemisch durchführt.

Im Übrigen sind erfindungsgemäß erhältliche geometrische Katalysatorformkörper K ganz generell als Katalysatoren mit erhöhter Anfangsaktivität und erhöhter Zielproduktbildungsselektivität für gasphasenkatalytische Partialoxidationen 3 bis 6 (d. h., 3, 4, 5 oder 6) C-Atome enthaltender Alkane (insbesondere Propan), Alkanole, Alkanale, Alkene und Alkenale zu z. B. olefinisch ungesättigten Aldehyden und/oder Carbonsäuren, sowie den entsprechenden Nitrilen (Ammoxidation, vor allem von Propen zu Acrylnitril und von 2-Methylpropen bzw. tert.-Butanol (bzw. dessen Methylether) zu Methacrylnitril) sowie für gasphasenkatalytische oxidative Dehydrierungen der vorgenannten 3, 4, 5 oder 6 C-Atome enthaltenden organischen Verbindungen geeignet.

Die großtechnische Herstellung von erfindungsgemäßen Katalysatorformkörpern K (sowie aller Ausführungsbeispiele B1 bis B13 und Vergleichsbeispiele V1 bis V16) erfolgt anwendungstechnisch zweckmäßig wie in den Deutschen Anmeldungen Nr. 102008040093.9 und 102008040094.7 beschrieben (besonders vorteilhaft gemäß Beispiel I.3. der Anmeldung Nr. 102008040094.7).

Beispiele und Vergleichsbeispiele

I) Herstellung von ringförmigen Vollkatalysatorformkörpern B1 bis B13 sowie von ringförmigen Vergleichskatalysatorformkörpern V1 bis V16 mit der nachfolgenden Stöchiometrie der Aktivmasse:
$$[Bi_2W_4O_{15}]_p[Bi_cMo_{12}Fe_{3,0}Co_eSi_{1,6}K_{0,08}O_x]_1$$

1) Herstellung des ringförmigen Vergleichsvollkatalysatorformkörpers V1 ($[Bi_2W_4O_{15}]_{0,50}[Mo_{12}Fe_{3,0}Co_{5,5}Si_{1,6}K_{0,08}O_x]_1$)

a) Herstellung der feinteiligen Ausgangsmasse A1 $Bi_2W_4O_{15}$ ($Bi_2W_2O_9 \cdot 2WO_3$)

In einem 1,75-m³-Doppelmantelbehälter (der Zwischenraum wurde von Temperierwasser durchströmt) aus Edelstahl mit Balkenrührer wurden in 773 kg einer 25 °C aufweisenden wässrigen salpetersauren Wismutnitratlösung (11,3 Gew.-% Bi; freie Salpetersäure 3 bis 5 Gew.-%; hergestellt mit Salpetersäure aus Wismutmetall der Firma Sidech S.A., 1495 Tilly, Belgien, Reinheit: > 99,997 Gew.-% Bi, < 7 mg/kg Pb, je < 5 mg/kg Ni, Ag, Fe, je < 3 mg/kg Cu, Sb und je < 1 mg/kg Cd, Zn) bei 25 °C innerhalb von 20 min portionsweise 214,7 kg 25 °C aufweisende Wolframsäure (74,1 Gew.-% W, mittlere Korngröße (laut Hersteller bestimmt gemäß ASTM B330) 0,4 bis 0,8 $\mu$m, Glühverlust (2 h bei 750 °C an Luft) 6 - 8 Gew.-%, Schüttgewicht 5 - 8 g/inch³, H.C. Starck, D-38615 Goslar) eingerührt (70 U/min). Das resultierende wässrige Gemisch wurde anschließend noch 3 h bei 25 °C gerührt und dann sprühgetrocknet. Die Sprühtrocknung erfolgte in einem Drehscheibensprühturm vom Typ FS 15 der Firma Niro im Heißluftgleichstrom bei einer Gaseintrittstemperatur von 300 ± 10 °C, einer Gasaustrittstemperatur von 100 ± 10°C, einer Scheibendrehzahl von 18000 U/min, einem Durchsatz von 200 l/h und einer Luftmenge von 1800 Nm³/h. Das resultierende Sprühpulver wies einen Glühverlust von 12,8 Gew.-% (3 h bei 600 °C im Porzellantiegel (der bei 900 °C bis zur Gewichtskonstanz geglüht worden war) unter Luft glühen) und (bei einem Dispergierdruck von 1,1 bar absolut) einen $d_{50}$ von 28,0 μm ($d_{10}$ = 9,1 μm, $d_{90}$ = 55,2 μm) auf. Die nachfolgende Tabelle 1 gibt einen Überblick über repräsentative $d_x$-Werte in Abhängigkeit vom angewandten absoluten Dispergierdruck:

Tabelle 1

|  | 2 bar | 1,5 bar | 1,2 bar | 1,1 bar |
|---|---|---|---|---|
| $d_{10}$ (μm) | 0,91 | 1,17 | 3,4 | 9,1 |
| $d_{50}$ (μm) | 5,8 | 8,5 | 19,7 | 28,0 |
| $d_{90}$ (μm) | 27,5 | 34,3 | 47,2 | 55,2 |

Das erhaltene Sprühpulver wurde anschließend mit 16,7 Gew.-% (bezogen auf das Gewicht des Sprühpulvers) an 25 °C aufweisendem Wasser in einem Auspresskneter für 30 min angeteigt und mittels eines Extruders zu Strängen des Durchmessers 6 mm extrudiert. Diese wurden in Abschnitte von 6 cm geschnitten, auf einem 3-zonigen Bandtrockner bei einer Verweilzeit von 120 min je Zone bei Temperaturen von 90-95°C (Zone 1), 115 °C (Zone 2) und 125 °C (Zone 3) an Luft getrocknet und dann bei einer Temperatur im Bereich um 830 °C thermisch behandelt (kalziniert; im luftdurchströmten Drehrohrofen (0,3 mbar Unterdruck, 200 Nm³/h Luft, 50 kg/h Extrudat, Drehzahl: 1 U/min)). Wesentlich bei der genauen Einstellung der Kalzinationstemperatur ist, dass sie an der angestrebten Phasenzusammensetzung des Kalzinationsprodukts orientiert zu erfolgen hat, das kalzinierte Material aber andererseits eine BET-Oberfläche ≥ 0,2 m²/g aufweist. Gewünscht sind die Phasen $WO_3$ (monoklin) und $Bi_2W_2O_9$ (orthorhombisch), unerwünscht ist hier das Vorhandensein von γ-$Bi_2WO_6$ (Russellit). Sollte daher nach der Kalzination der Gehalt der Verbindung γ-$Bi_2WO_6$ mehr als 5 Intensitäts-% betragen (berechnet als Verhältnis (Int.-V) der Intensität des Reflexes von γ-$Bi_2WO_6$ im Röntgenpulverdiffraktogramm bei 2Θ = 28,4 ° (CuKα-Strahlung) zur Intensität des Reflexes von $Bi_2W_2O_9$ bei 2Θ = 30,0 °), so ist die Präparation zu wiederholen und die Kalzinationstemperatur oder die Verweilzeit bei gleichbleibender Kalzinationstemperatur zu erhöhen, bis der vorgenannte Grenzwert erreicht oder unterschritten wird. Das so erhaltene vorgebildete kalzinierte Mischoxid wurde mit einer Biplex-Querstromsichtmühle Typ 500 BQ der Firma Hosokawa Alpine AG, Augsburg mit 2500 U/min gemahlen, so dass der $d_{50}$-Wert (bei einem Dispergierdruck von 2,0 bar absolut gemessen) der anfallenden feinteiligen Ausgangsmasse A1 $d_{50}^{A1}$ = 2,45 μm ( $d_{10}^{A1}$ = 1,05 μm, $d_{90}^{A1}$ = 5,9 μm ), die BET-Oberfläche 0,8 m²/g und der γ-$Bi_2WO_6$-Gehalt 3 Intensitäts-% (= 100-% • Int.-V) betrug. Die feinteilige Ausgangsmasse A1 wurde dann in Portionen von 20 kg in einem Schräglagen-Mischer mit Misch- und Schneidflügel (Drehzahl Mischflügel: 60 U/min, Drehzahl Schneidflügel: 3000 U/min) innerhalb von 5 min homogen mit 0,5 Gew.-% (bezogen auf die feinteilige Ausgangsmasse A1) feinteiligem hydrophobisiertem $SiO_2$ der Fa. Degussa vom Typ Sipernat® D17 (Rüttelgewicht 150 g/l; $d_{50}$-Wert der $SiO_2$-Partikel (Laserbeugung nach ISO 13320-1) = 10 μm, die spezifische Oberfläche (Stickstoffadsorption nach ISO 5794-1, Annex D) = 100 m²/g) vermischt.

b) Herstellung der feinteiligen Ausgangsmasse A2 ($Mo_{12}Fe_{3,0}Co_{5,5}Si_{1,6}K_{0,08}$)

Eine Lösung A wurde hergestellt, indem man in einem wassertemperierten 1,75-m³-Doppelmantelbehälter aus Edelstahl mit einem Balkenrührer bei 60 °C unter Rühren (70 U/min) zu 660 l eine Temperatur von 60 °C aufweisendem Wasser innerhalb einer Minute 1,075 kg einer eine Temperatur von 60 °C aufweisenden wässrigen Kaliumhydroxidlösung (47,5 Gew.-% KOH) und anschließend über eine Differenzialdosierwaage mit einer Dosiergeschwindigkeit von 600 kg/h 237,1 kg Ammoniumheptamolybdattetrahydrat der Temperatur 25 °C (weiße Kristalle mit einer Körnung d < 1 mm, 81,5 Gew.-% $MoO_3$, 7,0-8,5 Gew.-% $NH_3$, max. 150 mg/kg Alkalimetalle, H.C. Starck, D-38642 Goslar) dosierte und die resultierende leicht trübe Lösung bei 60 °C 60 min rührte (70 U/min).

Eine Lösung B wurde hergestellt, indem man in einem wassertemperierten 1,75-m³-Doppelmantelbehälter aus Edelstahl mit einem Balkenrührer bei 60 °C in 282,0 kg einer eine Temperatur von 60 °C aufweisenden wässrigen Kobalt(-II)-nitratlösung (12,5 Gew.-% Co, hergestellt mit Salpetersäure aus Kobaltmetall der Firma MFT Metals & Ferro-Alloys Trading GmbH, D-41747 Viersen, Reinheit, >99,6 Gew.-%, < 0,3 Gew.-% Ni, < 100 mg/kg Fe, < 50 mg/kg Cu) vorlegte und zu dieser unter Rühren (70 U/min) 142,0 kg einer 60 °C warmen Eisen-(III)-nitrat-nonahydrat-Schmelze (13,8 Gew.-% Fe, < 0,4 Gew.-% Alkalimetalle, < 0,01 Gew.-% Chlorid, < 0,02 Gew.-% Sulfat, Dr. Paul Lohmann GmbH, D-81857 Emmerthal) dosierte. Anschließend wurde unter Aufrechterhaltung der 60 °C 30 Minuten nachgerührt. Dann wurde unter Beibehalt der 60 °C die Lösung B in die vorgelegte Lösung A abgelassen und weitere 15 Minuten mit 70 U/min bei 60 °C gerührt. Anschließend wurden dem resultierenden wässrigen Gemisch 19,9 kg eines Kieselgels der Fa. Grace vom Typ Ludox TM 50 (50,1 Gew.-% $SiO_2$, Dichte: 1,29 g/ml, pH 8,5 bis 9,5, Alkaligehalt max. 0,5 Gew.-%) zugegeben und danach noch weitere 15 Minuten mit 70 U/min bei 60°C gerührt.

Anschließend wurde in einem Drehscheibensprühturm vom Typ FS-15 der Firma Niro im Heißluftgegenstrom sprühgetrocknet (Gaseintrittstemperatur: 350 ± 10°C, Gasaustrittstemperatur: 140 ± 5°C, Scheibendrehzahl: 15000 U/min, Durchsatz: 270 kg/h, Luftmenge: 2100 $Nm^3$/h). Das resultierende Sprühpulver (= die feinteilige Ausgangsmasse A2) wies einen Glühverlust von 30,0 Gew.-% (3 h bei 600°C unter Luft glühen) und einen $d_{90}^{A2}$ -Wert (bei einem Dispergierdruck von 2,0 bar absolut gemessen) von 59,6 $\mu$m ($d_{10}^{A2}$ = 4,5 µm, $d_{50}^{A2}$ = 26,3 µm) auf.

c) Herstellung des ringförmigen Vergleichsvollkatalysatorformkörpers V1

Die Sipermat® D17 zugesetzt enthaltende feinteilige Ausgangsmasse A1 wurde mit der feinteiligen Ausgangsmasse A2 in den für eine Multimetalloxidaktivmasse der Stöchiometrie:

$$[Bi_2W_4O_{15}]_{0,50}[Mo_{12}Fe_{3,0}Co_{5,5}Si_{1,6}K_{0,08}O_x]_1$$

erforderlichen Mengen (Gesamtmenge: 3 kg) in einem Eirich-Intensivmischer (Typ R02, Füllvolumen: 3-5 l, Leistung: 1,9 kW, Maschinenfabrik Gustav Eirich GmbH & Co KG, D-74736 Hardheim) mit gegenläufig zum Teller rotierenden Messerköpfen (Drehzahl Teller: 44 U/min, Drehzahl Messerköpfe: 2500 U/min) innerhalb von 5 min homogen vermischt. Bezogen auf die vorgenannte Gesamtmasse wurden zu dieser in einem Rhönradmischer (Raddurchmesser: 650 mm, Trommelvolumen: 5 l) zusätzlich 1 Gew.-% Graphit TIMREX® T44 der Firma Timcal AG ($d_{50}$ = 20,8 $\mu$m) innerhalb von 30 min bei einer Drehzahl von ca. 30 U/min homogen untergemischt. Das resultierende Gemisch wurde dann in einem Laborkalander mit 2 gegenläufigen Stahlwalzen bei einem Pressdruck von 9 bar kompaktiert und durch ein Sieb mit einer Maschenweite von 0,8 mm gedrückt. Das Kompaktat wurde anschließend in obigem Rhönradmischer bei einer Drehzahl von ca. 30 U/min innerhalb von 30 Minuten mit, bezogen auf sein Gewicht, weiteren 2,5 Gew.-% des genannten Graphits vermischt und anschließend wie in der Deutschen Anmeldung mit dem Aktenzeichen 10 2008 040 093.9 beschrieben in einem Kilian Rundläufer (9-fach-Tablettiermaschine) vom Typ S100 (Fa. Kilian, D-50735 Köln) unter Stickstoffatmosphäre zu ringförmigen Vollkatalysatorvorläuferformkörpern (Formkörpern V) der Geometrie 5 x 3 x 2 mm (A (Außendurchmesser) x H (Höhe) x I (Innendurchmesser)) mit einer Seitendruckfestigkeit von 20 ± 1 N und einer Masse von 125 mg verdichtet (Füllhöhe: 7,5-9 mm, Presskraft: 3,0-3,5 kN).

Zur abschließenden thermischen Behandlung wurden jeweils 1000 g der jeweils hergestellten ringförmigen Vollkatalysatorvorläuferformkörper V gleichmäßig aufgeteilt auf nebeneinander angeordnete 4 Gitternetze mit einer quadratischen Grundfläche von jeweils 150 mm x 150 mm (Schütthöhe: ca. 15 mm) in einem mit 650 Nl/h auf anfänglich 140 °C temperierter Luft (anstelle von Luft kann auch ein Gasstrom aus 25 Vol.-% $N_2$ und 75 Vol.-% Luft, oder aus 50 Vol.-% $N_2$ und 50 Vol.-% Luft, oder aus 75 Vol.-% $N_2$ und 25 Vol.-% Luft, oder aus 100 Vol.-% $N_2$ verwendet werden) durchströmten Umluftofen (Firma Heraeus Instruments GmbH, D-63450 Hanau, Typ: K 750/2) zunächst innerhalb von 120 min von Raumtemperatur (25°C) auf 185 °C aufgeheizt. Diese Temperatur wurde für 1 h aufrechterhalten und dann innerhalb von 50 min auf 225°C erhöht. Die 225°C wurden für 2 h gehalten, bevor die Temperatur innerhalb von 23 min weiter auf 270°C erhöht wurde. Diese Temperatur wurde ebenfalls 1 h aufrechterhalten, bevor sie, innerhalb von 177 min im Wesentlichen linear auf 462 ± 1 °C erhöht wurde. Diese Endkalzinationstemperatur wurde für 10 Stunden gehalten. Danach wurde innerhalb von ca. 12 h auf 25 °C abgekühlt.

Der resultierende ringförmige Vergleichsvollkatalysatorformkörper V1 wies ein Verhältnis von insgesamt enthaltener molarer Menge an Wismut zu insgesamt enthaltener molarer Menge an Molybdän, $n_{Bi}/n_{Mo}$, von 1/12 auf.

2) Herstellung des ringförmigen Vergleichsvollkatalysatorformkörpers V2 ($[Bi_2W_4O_{15}]_{0,60}[Mo_{12}Fe_{3,0}Co_{5,5}Si_{1,6}K_{0,08}O_x]_1$)

Die Herstellung von V2 erfolgte wie unter 1) für V1 beschrieben, mit dem Unterschied, dass im Verfahrensschritt 1c) das Mischungsverhältnis von Ausgangsmasse A1 zu Ausgangsmasse A2 so angepasst wurde, dass bei der Kalzinierung die Stöchiometrie

$$[Bi_2W_4O_{15}]_{0,60}[Mi_{12}Fe_{3,0}Co_{5,5}Si_{1,6}K_{0,08}O_x]_1$$

resultierte. Das Verhältnis $n_{Bi}/n_{Mo}$ war 1,2/12.

3) Herstellung des nicht erfindungsgemäßen Vollkatalysatorformkörpers V3 ([Bi$_2$W$_4$O$_{15}$]$_{0,45}$[Mo$_{12}$Fe$_{3,0}$Co$_{5,5}$Si$_{1,6}$K$_{0,08}$O$_x$]$_1$)

Die Herstellung von V3 erfolgte wie unter 1) für V1 beschrieben, mit dem Unterschied, dass im Verfahrensschritt 1c) das Mischungsverhältnis von Ausgangsmasse A1 zu Ausgangsmasse A2 so angepasst wurde, dass bei der Kalzinierung die Stöchiometrie

$$[Bi_2W_4O_{15}]_{0,45}[Mo_{12}Fe_{3,0}Co_{5,5}Si_{1,6}K_{0,08}O_x]_1$$

resultierte. n$_{Bi}$/n$_{Mo}$ war 0,9/12.

4) Herstellung des ringförmigen Vergleichsvollkatalysatorformkörpers V4 ([Bi$_2$W$_4$O$_{15}$]$_{0,35}$[Mo$_{12}$Fe$_{3,0}$Co$_{5,5}$Si$_{1,6}$K$_{0,08}$O$_x$]$_1$)

Die Herstellung von V4 erfolgte wie unter 1) für V1 beschrieben, mit dem Unterschied, dass im Verfahrensschritt 1c) das Mischungsverhältnis von Ausgangsmasse A1 zu Ausgangsmasse A2 so angepasst wurde, dass bei der Kalzinierung die Stöchiometrie

$$[Bi_2W_4O_{15}]_{0,35}[Mo_{12}Fe_{3,0}Co_{5,5}Si_{1,6}K_{0,08}O_x]_1$$

resultierte. n$_{Bi}$/n$_{Mo}$ war 0,7/12.

5) Herstellung des ringförmigen Vergleichsvollkatalysatorformkörpers V5 ([Bi$_2$W$_4$O$_{15}$]$_{0,25}$[Mo$_{12}$Fe$_{3,0}$Co$_{5,5}$Si$_{1,6}$K$_{0,08}$O$_x$]$_1$)

Die Herstellung von V5 erfolgte wie unter 1) für V1 beschrieben, mit dem Unterschied, dass im Verfahrensschritt 1c) das Mischungsverhältnis von Ausgangsmasse A1 zu Ausgangsmasse A2 so angepasst wurde, dass bei der Kalzinierung die Stöchiometrie

$$[Bi_2W_4O_{15}]_{0,25}[Mo_{12}Fe_{3,0}Co_{5,5}Si_{1,6}K_{0,08}O_x]_1$$

resultiere. n$_{Bi}$/n$_{Mo}$ war 0,5/12.

6) Herstellung des ringförmigen Vollkatalysatorformkörpers V6 ([Bi$_2$W$_4$O$_{15}$]$_{0,50}$[Mo$_{12}$Fe$_{3,0}$Co$_{5,5}$Si$_{1,6}$K$_{0,08}$O$_x$]$_1$)

Die Herstellung von V6 erfolgte wie unter 1) für V1 beschrieben, mit dem Unterschied, dass bei der Herstellung der Ausgangsmasse A2 im Verfahrensschritt 1b) die Drehzahl der Zerstäuberscheibe im Sprühturm auf 18000 U/min erhöht wurde. Das so resultierende Sprühpulver wies einen Glühverlust von 30,0 Gew.-% (3 h bei 600°C unter Luft glühen) und einen $d_{90}^{A2}$-Wert (bei einem Dispergierdruck von 2,0 bar absolut gemessen) von 47,1 μm $\left( d_{10}^{A2} = 3{,}9 \text{ μm}, \ d_{50}^{A2} = 20{,}3 \text{ μm} \right)$ auf. n$_{Bi}$/n$_{Mo}$ war 1/12.

7) Herstellung des ringförmigen Vollkatalysatorformkörpers V7 ([Bi$_2$W$_4$O$_{15}$]$_{0,50}$[Mo$_{12}$Fe$_{3,0}$Co$_{5,5}$Si$_{1,6}$K$_{0,08}$O$_x$]$_1$)

Die Herstellung von V7 erfolgte wie unter 6) für V6 beschrieben, mit dem Unterschied, dass bei der Herstellung der Ausgangsmasse A1 im Verfahrensschritt 1a) der Durchsatz so erhöht wurde, dass sich ein $d_{50}^{A1}$-Wert (bei einem Dispergierdruck von 2,0 bar absolut gemessen) von 3,0 μm $\left( d_{10}^{A1} = 1{,}10 \text{ μm}, \ d_{90}^{A1} = 13{,}9 \text{ μm} \right)$ ergab. Die BET-Oberfläche betrug 0,5 m$^2$/g und der γ-Bi$_2$WO$_6$-Gehalt 4 Intensitäts-%. n$_{Bi}$/n$_{Mo}$ war 1/12.

8) Herstellung des ringförmigen Vergleichsvollkatalysatorformkörpers V8 ([Bi$_2$W$_4$O$_{15}$]$_{0,50}$[Mo$_{12}$Fe$_{3,0}$Co$_{5,5}$Si$_{1,6}$K$_{0,08}$O$_x$]$_1$)

Die Herstellung von V8 erfolgte wie unter 6) für V6 beschrieben, mit dem Unterschied, dass bei der Herstellung der Ausgangsmasse A1 im Verfahrensschritt 1a) der Durchsatz so reduziert wurde, dass sich ein $d_{50}^{A1}$-Wert (bei einem Dispergierdruck von 2,0 bar absolut gemessen) von 2,10 μm $\left( d_{10}^{A1} = 0{,}92 \text{ μm}, \ d_{90}^{A1} = 4{,}7 \text{ μm} \right)$ ergab. Die BET-Oberfläche betrug 1,2 m$^2$/g und der γ-Bi$_2$WO$_6$-Gehalt 4 Intensitäts-%. n$_{Bi}$/n$_{Mo}$ war 1/12.

9) Herstellung des ringförmigen Vergleichsvollkatalysatorformkörpers V9 ($[Bi_2W_4O_{15}]_{0,50}[Mo_{12}Fe_{3,0}Co_{5,5}Si_{1,6}K_{0,08}O_x]_1$)

Die Herstellung von V9 erfolgte wie unter 6) für V6 beschrieben, mit dem Unterschied, dass bei der Herstellung der Ausgangsmasse A1 im Verfahrensschritt 1a) nach der Mahlung in der Querstromsichtmühle eine weitere Mahlung in einer Spiralstrahlmühle (Injektionsdruck: 8 bar) erfolgte, so dass sich ein $d_{50}^{A1}$-Wert (bei einem Dispergierdruck von 2,0 bar absolut gemessen) von 1,60 μm ($d_{10}$ = 0,78 μm, $d_{90}$ = 3,3 μm) ergab. Die BET-Oberfläche betrug 1,9 m2/g und der γ-$Bi_2WO_6$-Gehalt 4 Intensitäts-%. $n_{Bi}/n_{Mo}$ war 1/12.

10) Herstellung des ringförmigen Vergleichsvollkatalysatorformkörpers V10 ($[Bi_2W_4O_{15}]_{0,50}[Mo_{12}Fe_{3,0}Co_{5,5}Si_{1,6}K_{0,08}O_x]_1$)

Die Herstellung von V10 erfolgte wie unter 9) für V9 beschrieben, mit dem Unterschied, dass bei der Kalzination im Verfahrensschritt 1c) eine Endkalzinationstemperatur von 457 ± 1 °C eingestellt wurde. $n_{Bi}/n_{Mo}$ war 1/12.

11) Herstellung des ringförmigen Vergleichsvollkatalysatorformkörpers V11 ($[Bi_2W_4O_{15}]_{0,50}[Mo_{12}Fe_{3,0}Co_{5,5}Si_{1,6}K_{0,08}O_x]_1$)

Die Herstellung von V11 erfolgte wie unter 10) für V10 beschrieben, mit dem Unterschied, dass bei der Herstellung der Ausgangsmasse A2 im Verfahrensschritt 1b) die Drehzahl der Zerstäuberscheibe im Sprühturm auf 12500 U/min erniedrigt wurde. Das so resultierende Sprühpulver wies einen Glühverlust von 30,0 Gew.-% (3 h bei 600°C unter Luft glühen) und einen $d_{90}^{A2}$-Wert (bei einem Dispergierdruck von 2,0 bar absolut gemessen) von 80,3 μm ($d_{10}^{A2}$ = 7,0 μm, $d_{50}^{A2}$ = 39,4 μm) auf. $n_{Bi}/n_{Mo}$ war 1/12.

12) Herstellung des ringförmigen Vergleichsvollkatalysatorformkörpers V12 ($[Bi_2W_4O_{15}]_{0,10}[Mo_{12}Fe_{3,0}Co_{5,5}Si_{1,6}K_{0,08}O_x]_1$)

Die Herstellung von V12 erfolgte wie unter 11) für V11 beschrieben, mit dem Unterschied, dass im Verfahrensschritt 1c) das Mischungsverhältnis von Ausgangsmasse A1 zu Ausgangsmasse A2 so angepasst wurde, dass bei der Kalzinierung die Stöchiometrie

$$[Bi_2W_4O_{15}]_{0,10}[Mo_{12}Fe_{3,0}Co_{5,5}Si_{1,6}K_{0,08}O_x]_1$$

resultierte. $n_{Bi}/n_{Mo}$ war 0,2/12.

13) Herstellung des ringförmigen Vergleichsvollkatalysatorformkörpers V13 ($[Bi_2W_4O_{15}]_{0,10}[Mo_{12}Fe_{3,0}Co_{5,5}Si_{1,6}K_{0,08}O_x]_1$)

Die Herstellung von V13 erfolgte wie unter 12) für V12 beschrieben, mit dem Unterschied, dass bei der Kalzination im Verfahrensschritt 1c) eine Endkalzinationstemperatur von 470 ± 1 °C eingestellt wurde. $n_{Bi}/n_{Mo}$ war 0,2/12.

14) Herstellung des ringförmigen Vollkatalysatorformkörpers V14 ($[Bi_2W_4O_{15}]_{0,50}[Mo_{12}Fe_{3,0}Co_{5,5}Si_{1,6}K_{0,08}O_x]_1$)

Die Herstellung von V14 erfolgte wie unter 8) für V8 beschrieben, mit dem Unterschied, dass bei der Herstellung der Ausgangsmasse A2 im Verfahrensschritt 1b) die Drehzahl der Zerstäuberscheibe im Sprühturm auf 12500 U/min erniedrigt wurde. Das so resultierende Sprühpulver wies einen Glühverlust von 30,0 Gew.-% (3 h bei 600°C unter Luft glühen) und einen $d_{90}^{A2}$-Wert (bei einem Dispergierdruck von 2,0 bar absolut gemessen) von 80,3 μm ($d_{10}^{A2}$ = 7,0 μm, $d_{50}^{A2}$ = 39,4 μm) auf. $n_{Bi}/n_{Mo}$ war 1,0/12.

15) Herstellung des ringförmigen Vollkatalysatorformkörpers V15 ($[Bi_2W_4O_{15}]_{0,50}[Bi_{1,0}Mo_{12}Fe_{3,0}Co_{5,5}Si_{1,6}K_{0,08}O_x]_1$)

Die Herstellung von V15 erfolgte wie unter 1) für V1 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 205,7 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von V15 gemäß Verfahrensschritt 1c) wurde das Mischungsverhältnis von Ausgangsmasse A1 zu Ausgangsmasse A2 so angepasst, dass bei der Kalzinierung die Stöchiometrie

$$[Bi_2W_4O_{15}]_{0,50}[Bi_{1,0}Mo_{12}Fe_{3,0}Co_{5,5}Si_{1,6}K_{0,08}O_x]_1$$

resultierte. $n_{Bi}/n_{Mo}$ war 2/12. $d_{90}^{A2}$ war zu 59,9 μm.

16) Herstellung des ringförmigen Vollkatalysatorformkörpers V16 ($[Bi_{0,6}Mo_{12}Fe_{3,0}Co_{7,0}Si_{1,6}K_{0,08}O_x]_1$)

Die Herstellung von V16 erfolgte wie unter 1) für V1 beschrieben, mit den folgenden Unterschieden:

i) Bei der Herstellung der Lösung B für die Ausgangsmasse A2 wurde die Menge der wässrigen Kobalt(-II)-nitratlösung auf 358,9 kg erhöht.

ii) Bei der Herstellung der Ausgangsmasse A2 wurden vor dem Ablassen der Lösung B in die Lösung A unter Rühren (70 U/min) bei 60 °C in die Lösung B zusätzlich 123,4 kg der eine Temperatur von 60 °C aufweisenden wässrigem salpetersaure Wismutnitratlösung aus Verfahrensschritt 1a) eindosiert und anschließend für weitere 30 Minuten bei 60 °C gerührt.

iii) Bei der Herstellung von V16 gemäß Verfahrensschritt 1c) wurde auf das Abmischen mit Ausgangsmasse A1 im Eirich-Intensivmischer verzichtet. $n_{Bi}/n_{Mo}$ war damit 0,6/12. $d_{90}^{A2}$ war 59,4 μm.

17) Herstellung des ringförmigen Vollkatalysatorformkörpers B1 ($[Bi_2W_4O_{15}]_{0,24}[Bi_{0,02}Mo_{12}Fe_{3,0}Co_{5,5}Si_{1,6}K_{0,08}O_x]_1$)

Die Herstellung von B1 erfolgte wie unter 5) für V5 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 4,2 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von B1 gemäß Verfahrensschritt 1c) wurde das Mischungsverhältnis von Ausgangsmasse A1 zu Ausgangsmasse A2 so angepasst, dass bei der Kalzinierung die Stöchiometrie

$$([Bi_2W_4O_{15}]_{0,24}[Bi_{0,02}Mo_{12}Fe_{3,0}Co_{5,5}Si_{1,6}K_{0,08}O_x]_1)$$

resultierte. $n_{Bi}/n_{Mo}$ war 0,5/12. $d_{90}^{A2}$ war 59,5 μm.

18) Herstellung des ringförmigen Vollkatalysatorformkörpers B2 ($[Bi_2W_4O_{15}]_{0,24}[Bi_{0,02}Mo_{12}Fe_{3,0}Co_{5,5}Si_{1,6}K_{0,08}O_x]_1$)

Die Herstellung von B2 erfolgte wie unter 17) für B1 beschrieben, mit dem Unterschied, dass bei der Kalzination gemäß Verfahrensschritt 1c) eine Endkalzinationstemperatur von 470 ± 1 °C eingestellt wurde. $n_{Bi}/n_{Mo}$ war 0,5/12.

19) Herstellung des ringförmigen Vollkatalysatorformkörpers B3 ($[Bi_2W_4O_{15}]_{0,235}[Bi_{0,03}Mo_{12}Fe_{3,0}Co_{5,5}Si_{1,6}K_{0,08}O_x]_1$)

Die Herstellung von B3 erfolgte wie unter 5) für V5 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 6,3 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von B3 gemäß Verfahrensschritt 1c) wurde das Mischungsverhältnis von Ausgangsmasse A1 zu Ausgangsmasse A2 so angepasst, dass bei der Kalzinierung die Stöchiometrie

$$([Bi_2W_4O_{15}]_{0,235}[Bi_{0,03}Mo_{12}Fe_{3,0}Co_{5,5}Si_{1,6}K_{0,08}O_x]_1)$$

resultierte. $n_{Bi}/n_{Mo}$ war 0,5/12. $d_{90}^{A2}$ war 59,6 $\mu$m.

20. Herstellung des ringförmigen Vollkatalysatorformkörpers B4 ($[Bi_2W_4O_{15}]_{0,235}[Bi_{0,03}Mo_{12}Fe_{3,0}Co_{5,5}Si_{1,6}K_{0,08}O_x]_1$)

Die Herstellung von B4 erfolgte wie unter 19) für B3 beschrieben, mit dem Unterschied, dass bei der Kalzination gemäß Verfahrensschritt 1c) eine Endtemperatur von 470 $\pm$ 1 °C eingestellt wurde. $n_{Bi}/n_{Mo}$ war 0,5/12.

21. Herstellung des ringförmigen Vollkatalysatorformkörpers B5 ($[Bi_2W_4O_{15}]_{0,345}[Bi_{0,01}Mo_{12}Fe_{3,0}Co_{5,5}Si_{1,6}K_{0,08}O_x]_1$)

Die Herstellung von B5 erfolgte wie unter 4) für V4 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 2,1 kg der eine Temperatur von 60 °C aufweisenden wässrigen salpetersauren Wismutnitratlösung gemäß Verfahrensschritt 1a) eindosiert und anschließend für weitere 30 Minuten bei 60 °C gerührt.

ii) Bei der Herstellung von B5 gemäß Verfahrensschritt 1c) wurde das Mischungsverhältnis von Ausgangsmasse A1 zu Ausgangsmasse A2 so angepasst, dass bei der Kalzinierung die Stöchiometrie

$$[Bi_2W_4O_{15}]_{0,345}[Bi_{0,01}Mo_{12}Fe_{3,0}Co_{5,5}Si_{1,6}K_{0,08}O_x]_1$$

resultierte. $n_{Bi}/n_{Mo}$ war wie bei V4 0,7/12. $d_{90}^{A2}$ war 59,8 $\mu$m.

22. Herstellung des ringförmigen Vollkatalysatorformkörpers B6 ($[Bi_2W_4O_{15}]_{0,34}[Bi_{0,02}Mo_{12}Fe_{3,0}Co_{5,5}Si_{1,6}K_{0,08}O_x]_1$)

Die Herstellung von B6 erfolgte wie unter 4) für V4 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 4,2 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von B6 gemäß Verfahrensschritt 1c) wurde das Mischungsverhältnis von Ausgangsmasse A1 zu Ausgangsmasse A2 so angepasst, dass bei der Kalzinierung die Stöchiometrie

$$[Bi_2W_4O_{15}]_{0,34}[Bi_{0,02}Mo_{12}Fe_{3,0}Co_{5,5}Si_{1,6}K_{0,08}O_x]_1$$

resultierte. $n_{Bi}/n_{Mo}$ war wie bei V4 0,7/12. $d_{90}^{A2}$ war 60,0 $\mu$m.

23. Herstellung des ringförmigen Vollkatalysatorformkörpers B7 ($[Bi_2W_4O_{15}]_{0,325}[Bi_{0,05}Mo_{12}Fe_{3,0}Co_{5,5}Si_{1,6}K_{0,08}O_x]_1$)

Die Herstellung von B7 erfolgte wie unter 4) für V4 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 10,6 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von B7 gemäß Verfahrensschritt 1c) wurde das Mischungsverhältnis von Ausgangsmasse A1 zu Ausgangsmasse A2 so angepasst, dass bei der Kalzinierung die Stöchiometrie

$$([Bi_2W_4O_{15}]_{0,325}[Bi_{0,05}Mo_{12}Fe_{3,0}Co_{5,5}Si_{1,6}K_{0,08}O_x]_1)$$

resultierte. $n_{Bi}/n_{Mo}$ war wie bei V4 0,7/12. $d_{90}^{A2}$ war 59,6 μm.

24. Herstellung des ringförmigen Vollkatalysatorformkörpers B8 ([Bi$_2$W$_4$O$_{15}$]$_{0,30}$[Bi$_{0,1}$Mo$_{12}$Fe$_{3,0}$Co$_{5,5}$Si$_{1,6}$K$_{0,08}$O$_x$]$_1$)

Die Herstellung von B8 erfolgte wie unter 4) für V4 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 21,1 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von B8 gemäß Verfahrensschritt 1c) wurde das Mischungsverhältnis von Ausgangsmasse A1 zu Ausgangsmasse A2 so angepasst, dass bei der Kalzinierung die Stöchiometrie

$$[Bi_2W_4O_{15}]_{0,30}[Bi_{0,1}Mo_{12}Fe_{3,0}Co_{5,5}Si_{1,6}K_{0,08}O_x]_1$$

resultierte. $n_{Bi}/n_{Mo}$ war wie bei V4 0,7/12. $d_{90}^{A2}$ war 59,4 μm.

25. Herstellung des ringförmigen Vollkatalysatorformkörpers B9 ([Bi$_2$W$_4$O$_{15}$]$_{0,30}$[Bi$_{0,05}$Mo$_{12}$Fe$_{3,0}$Co$_{5,5}$Si$_{1,6}$K$_{0,08}$O$_x$]$_1$)

Die Herstellung von B9 erfolgte wie unter 24) für B8 beschrieben, mit dem Unterschied, dass bei der Kalzination gemäß Verfahrensschritt 1c) eine Endtemperatur von 457 ± 1 °C eingestellt wurde. $n_{Bi}/n_{Mo}$ war 0,7/12.

26) Herstellung des ringförmigen Vollkatalysatorformkörpers B10 ([Bi$_2$W$_4$O$_{15}$]$_{0,50}$[Bi$_{0,3}$Mo$_{12}$Fe$_{3,0}$Co$_{5,5}$Si$_{1,6}$K$_{0,08}$O$_x$]$_1$)

Die Herstellung von B10 erfolgte wie unter 1) für V1 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 61,7 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von B10 gemäß Verfahrensschritt 1c) wurde das Mischungsverhältnis von Ausgangsmasse A1 zu Ausgangsmasse A2 so angepasst, dass bei der Kalzinierung die Stöchiometrie

$$([Bi_2W_4O_{15}]_{0,50}[Bi_{0,3}Mo_{12}Fe_{3,0}Co_{5,5}Si_{1,6}K_{0,08}O_x]_1)$$

resultierte. $n_{Bi}/n_{Mo}$ war 1,3/12. $d_{90}^{A2}$ war 59,7 μm.

27) Herstellung des ringförmigen Vollkatalysatorformkörpers B11 ([Bi$_2$W$_4$O$_{15}$]$_{0,50}$[Bi$_{0,6}$Mo$_{12}$Fe$_{3,0}$Co$_{5,5}$Si$_{1,6}$K$_{0,08}$O$_x$]$_1$)

Die Herstellung von B11 erfolgte wie unter 1) für V1 beschrieben, mit den folgenden Unterschieden:

i) Bei der Herstellung der Ausgangsmasse A2 wurden vor dem Ablassen der Lösung B in die Lösung A unter Rühren (70 U/min) bei 60 °C in die Lösung B zusätzlich 123,4 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

ii) Bei der Herstellung von B11 gemäß Verfahrensschritt 1c) wurde das Mischungsverhältnis von Ausgangsmasse A1 zu Ausgangsmasse A2 so angepasst, dass bei der Kalzinierung die Stöchiometrie

$$([Bi_2W_4O_{15}]_{0,50}[Bi_{0,6}Mo_{12}Fe_{3,0}Co_{5,5}Si_{1,6}K_{0,08}O_x]_1)$$

resultierte. $n_{Bi}/n_{Mo}$ war 1,6/12. $d_{90}^{A2}$ war 59,9 μm.

28) Herstellung des ringförmigen Vollkatalysatorformkörpers B12 ($[Bi_2W_4O_{15}]_{0,25}[Bi_{0,6}Mo_{12}Fe_{3,0}Co_{7,0}Si_{1,6}K_{0,08}O_x]_1$)

Die Herstellung von B12 erfolgte wie unter 1) für V1 beschrieben, mit den folgenden Unterschieden:

i) Bei der Herstellung der Lösung B für die Ausgangsmasse A2 wurde die Menge der wässrigen Kobalt(-II)-nitratlösung auf 358,9 kg erhöht.

ii) Bei der Herstellung der Ausgangsmasse A2 wurden vor dem Ablassen der Lösung B in die Lösung A unter Rühren (70 U/min) bei 60 °C in die Lösung B zusätzlich 123,4 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

iii) Bei der Herstellung von B12 gemäß Verfahrensschritt 1c) wurde das Mischungsverhältnis von Ausgangsmasse A1 zu Ausgangsmasse A2 so angepasst, dass bei Kalzinierung die Stöchiometrie

$$([Bi_2W_4O_{15}]_{0,25}[Bi_{0,6}Mo_{12}Fe_{3,0}Co_{7,0}Si_{1,6}K_{0,08}O_x]_1)$$

resultierte. $n_{Bi}/n_{Mo}$ war 1,1/12. $d_{90}^{A2}$ war 59,4 $\mu$m.

29) Herstellung des ringförmigen Vollkatalysatorformkörpers B13 ($[Bi_2W_4O_{15}]_{0,50}[Bi_{0,6}Mo_{12}Fe_{3,0}Co_{7,0}Si_{1,6}K_{0,08}O_x]_1$)

Die Herstellung von B13 erfolgte wie unter 1) für V1 beschrieben, mit den folgenden Unterschieden:

i) Bei der Herstellung der Lösung B für die Ausgangsmasse A2 wurde die Menge der wässrigen Kobalt(-II)-nitratlösung auf 358,9 kg erhöht.

ii) Bei der Herstellung der Ausgangsmasse A2 wurden vor dem Ablassen der Lösung B in die Lösung A unter Rühren (70 U/min) bei 60 °C in die Lösung B zusätzlich 123,4 kg der eine Temperatur von 60 °C aufweisenden wässrigen salpetersauren Wismutnitratlösung aus Verfahrensschritt 1a) eindosiert und anschließend für weitere 30 Minuten bei 60 °C gerührt.

iii) Bei der Herstellung von B13 gemäß Verfahrensschritt 1c) wurde das Mischungsverhältnis von Ausgangsmasse A1 zu Ausgangsmasse A2 so angepasst, dass sich nach der Kalzinierung die Stöchiometrie

$$([Bi_2W_4O_{15}]_{0,50}[Bi_{0,6}Mo_{12}Fe_{3,0}Co_{7,0}Si_{1,6}K_{0,08}O_x]_1)$$

resultierte. $n_{Bi}/n_{Mo}$ war 1,6/12. $d_{90}^{A2}$ war 59,4 $\mu$m.

II) Ermittlung der Anfangsperformance der ringförmigen Vollkatalysatorformkörper B1 bis B13 und der ringförmigen Vergleichsvollkatalysatorformkörper V1 bis V16 in einer durch sie katalysierten Gasphasenpartialoxidation von Propen zu Acrolein.

Zur Ermittlung der Anfangsperformance der ringförmigen Vollkatalysatorformkörper B1 bis B13 und V1 bis V16 in einer durch sie katalysierten Gasphasenpartialoxidation von Propen zu Acrolein, wurden die ringförmigen Vollkatalysatorformkörper B1 bis B13 und V1 bis V16 der nachfolgend beschriebenen Versuchsdurchführung unterworfen.

Ein Reaktionsrohr (V2A Stahl; 21 mm Außendurchmesser, 3 mm Wandstärke, 15 mm Innendurchmesser, Länge 120 cm) wurde von oben nach unten in Strömungsrichtung des Reaktionsgasgemischs wie folgt beschickt:

Abschnitt 1: ca. 30 cm Länge
40 g Steatitkugeln (C220 Steatit der Fa. CeramTec) mit einem Durchmesser von 1,5 bis 2,0 mm als Vorschüttung (Aufheizzone).

Abschnitt 2: ca. 70 cm Länge
Katalysatorbeschickung mit 100 g des jeweiligen ringförmigen Vollkatalysators B1 bis B13 bzw. V1 bis V16

Die Temperierung des Reaktionsrohres erfolgte jeweils mittels eines mit molekularem Stickstoff durchperlten, die jeweils erforderliche Salzbadtemperatur $T^S$ (°C) aufweisenden Salzbades (53 Gew.-% Kaliumnitrat, 40 Gew.-% Natriumnitrit und 7 Gew.-% Natriumnitrat.

Während der Durchführung des Performancetests wurde das Reaktionsrohr kontinuierlich mit einem Reaktionsgasausgangsgemisch (Beschickungsgasgemisch aus Luft, polymer grade Propen und molekularem Stickstoff) der nachfolgenden Zusammensetzung beschickt:

| 5 Vol.-% | Propen (polymer grade), |
| 9,5 Vol.-% | Sauerstoff, und |
| 85,5 Vol.-% | $N_2$. |

Zum Zweck der Ermittlung der Anfangsperformance des frisch in das Reaktionsrohr eingebrachten Katalysatorfestbetts wurde dem Reaktionsrohr ein Volumenstrom des Reaktionsgasausgangsgemischs (dessen Eintrittstemperatur in das Reaktionsrohr ca. 30 °C betrug) von 100 Nl/h zugeführt, und die Salzbadtemperatur $T^S$ (°C) dabei stetig so einjustiert, dass der Propenumsatz $U^P$ (mol-%) beim einmaligen Durchgang des Beschickungsgasgemischs durch das Reaktionsrohr kontinuierlich etwa 95 mol-% betrug. Als Maßstab für die Anfangsperformance wurden nach einer Betriebsdauer von 60 h jeweils $T^S$ (reflektiert die Anfangsaktivität) und die Selektivität $S^{AC+AA}$ der Zielproduktgesamtbildung (Acrolein + Acrylsäure) bestimmt ($S^{AC+AA}$ in mol-% der beim Einmaldurchgang durch das Reaktionsrohr umgesetzten molaren Propenmenge). Der Druck am Eingang in das Reaktionsrohr betrug 1,2 bar absolut. Tabellen 2 und 3 zeigen die ermittelten Ergebnisse. Darüber hinaus zeigen sie zusätzlich die nach einer Betriebsdauer von 60 h jeweils resultierende Selektivität der Acroleinbildung ($S^{AC}$ (mol-%) sowie die bei der Herstellung der ringförmigen Vollkatalysatorformkörper angewandten $d_{50}^{41}$ (µm) und $d_{90}^{42}$ (µm). Die Tabellen 2,3 weisen ferner die bei der Herstellung der ringförmigen Vollkatalysatorformkörper angewandten Endkalzinationstemperaturen ($T_{Endkalzination}$ (°C)) aus und die Stöchiometrie der jeweiligen zugehörigen Multielementoxid-I-Aktivmasse.

Tabelle 2

| verwendeter ringförmiger Vergleichskatalysatorformkörper | $d_{50}^{A1}$ / μm | $d_{90}^{A2}$ / μm | $T_{Endkalzination}$ / °C | $T^S$ / °C | $S^{AC}$ / mol-% | $S^{AC+AA}$ / mol-% |
|---|---|---|---|---|---|---|
| V1 | $[Bi_2W_4O_{15}]_{0,50}[Mo_{12}Fe_{3,0}Co_{5,5}Si_{1,6}K_{0,08}O_x]_1$ | 2,45 | 59,6 | 462 ± 1 | 331 | 86,1 | 94,3 |
| V2 | $[Bi_2W_4O_{15}]_{0,60}[Mo_{12}Fe_{3,0}Co_{5,5}Si_{1,6}K_{0,08}O_x]_1$ | 2,45 | 59,6 | 462 ± 1 | 335 | 86,2 | 94,7 |
| V3 | $[Bi_2W_4O_{15}]_{0,45}[Mo_{12}Fe_{3,0}Co_{5,5}Si_{1,6}K_{0,08}O_x]_1$ | 2,45 | 59,6 | 462 ± 1 | 334 | 85,1 | 93,8 |
| V4 | $[Bi_2W_4O_{15}]_{0,35}[Mo_{12}Fe_{3,0}Co_{5,5}Si_{1,6}K_{0,08}O_x]_1$ | 2,45 | 59,6 | 462 ± 1 | 334 | 81,0 | 91,9 |
| V5 | $[Bi_2W_4O_{15}]_{0,25}[Mo_{12}Fe_{3,0}Co_{5,5}Si_{1,6}K_{0,08}O_x]_1$ | 2,45 | 59,6 | 462 ± 1 | 335 | 75,5 | 87,7 |
| V6 | $[Bi_2W_4O_{15}]_{0,50}[Mo_{12}Fe_{3,0}Co_{5,5}Si_{1,6}K_{0,08}O_x]_1$ | 2,45 | 47,1 | 462 ± 1 | 335 | 85,7 | 94,2 |
| V7 | $[Bi_2W_4O_{15}]_{0,50}[Mo_{12}Fe_{3,0}Co_{5,5}Si_{1,6}K_{0,08}O_x]_1$ | 3,0 | 47,1 | 462 ± 1 | 339 | 81,6 | 91,9 |
| V8 | $[Bi_2W_4O_{15}]_{0,50}[Mo_{12}Fe_{3,0}Co_{5,5}Si_{1,6}K_{0,08}O_x]_1$ | 2,10 | 47,1 | 462 ± 1 | 336 | 88,0 | 95,4 |
| V9 | $[Bi_2W_4O_{15}]_{0,50}[Mo_{12}Fe_{3,0}Co_{5,5}Si_{1,6}K_{0,08}O_x]_1$ | 1,60 | 47,1 | 462 ± 1 | 348 | 88,6 | 96,4 |
| V10 | $[Bi_2W_4O_{15}]_{0,50}[Mo_{12}Fe_{3,0}Co_{5,5}Si_{1,6}K_{0,08}O_x]_1$ | 1,60 | 80,3 | 457 ± 1 | 333 | 88,3 | 95,7 |
| V11 | $[Bi_2W_4O_{15}]_{0,50}[Mo_{12}Fe_{3,0}Co_{5,5}Si_{1,6}K_{0,08}O_x]_1$ | 1,60 | 80,3 | 462 ± 1 | 344 | 87,1 | 95,4 |
| V12 | $[Bi_2W_4O_{15}]_{0,10}[Mo_{12}Fe_{3,0}Co_{5,5}Si_{1,6}K_{0,08}O_x]_1$ | 1,60 | 80,3 | 462 ± 1 | 334 | 75,7 | 87,2 |
| V13 | $[Bi_2W_4O_{15}]_{0,10}[Mo_{12}Fe_{3,0}Co_{5,5}Si_{1,6}K_{0,08}O_x]_1$ | 1,60 | 80,3 | 470 ± 1 | 330 | 86,4 | 94,3 |
| V14 | $[Bi_2W_4O_{15}]_{0,50}[Mo_{12}Fe_{3,0}Co_{5,5}Si_{1,6}K_{0,08}O_x]_1$ | 2,10 | 80,3 | 462 ± 1 | 341 | 86,1 | 94,4 |
| V15 | $[Bi_2W_4O_{15}]_{0,50}[Bi_{1,0}Mo_{12}Fe_{3,0}Co_{5,5}Si_{1,6}K_{0,08}O_x]_1$ | 2,45 | 59,9 | 462 ± 1 | 315 | 86,7 | 92,7 |
| V16 | $Bi_{0,6}Mo_{12}Fe_{3,0}Co_{7,0}Si_{1,6}K_{0,08}O_x$ | - | 59,4 | 462 ± 1 | 306 | 90,9 | 93,3 |

Tabelle 3

| verwendeter ringförmiger Vollkatalysator | | $d_{50}^{A1}$ / $\mu$m | $d_{90}^{A2}$ / $\mu$m | $T_{Endkalzination}$ / °C | $T^S$ / °C | $S^{AC}$ / mol-% | $S^{AC+AA}$ / mol-% |
|---|---|---|---|---|---|---|---|
| B1 | $[Bi_2W_4O_{15}]_{0,24}$ $[Bi_{0,02}Mo_{12}Fe_{3,0}Co_{5,5}Si_{1,6}K_{0,08}O_x]_1$ | 2,45 | 59,5 | 462 ± 1 | 307 | 84,5 | 91,1 |
| B2 | $[Bi_2W_4O_{15}]_{0,24}$ $[Bi_{0,02}Mo_{12}Fe_{3,0}Co_{5,5}Si_{1,6}K_{0,08}O_x]_1$ | 2,45 | 59,5 | 470 ± 1 | 318 | 88,2 | 95,1 |
| B3 | $[Bi_2W_4O_{15}]_{0,235}$ $[Bi_{0,03}Mo_{12}Fe_{3,0}Co_{5,5}Si_{1,6}K_{0,08}O_x]_1$ | 2,45 | 59,6 | 462 ± 1 | 304 | 84,5 | 91,1 |
| B4 | $[Bi_2W_4O_{15}]_{0,235}$ $[Bi_{0,03}Mo_{12}Fe_{3,0}Co_{5,5}Si_{1,6}K_{0,08}O_x]_1$ | 2,45 | 59,6 | 470 ± 1 | 321 | 89,0 | 95,0 |
| B5 | $[Bi_2W_4O_{15}]_{0,345}$ $[Bi_{0,01}Mo_{12}Fe_{3,0}Co_{5,5}Si_{1,6}K_{0,08}O_x]_1$ | 2,45 | 59,8 | 462 ± 1 | 321 | 83,7 | 92,0 |
| B6 | $[Bi_2W_4O_{15}]_{0,34}$ $[Bi_{0,02}Mo_{12}Fe_{3,0}Co_{5,5}Si_{1,6}K_{0,08}O_x]_1$ | 2,45 | 60,0 | 462 ± 1 | 318 | 87,4 | 94,2 |
| B7 | $[Bi_2W_4O_{15}]_{0,325}$ $[Bi_{0,05}Mo_{12}Fe_{3,0}Co_{5,5}Si_{1,6}K_{0,08}O_x]_1$ | 2,45 | 59,6 | 462 ± 1 | 320 | 90,6 | 96,0 |
| B8 | $[Bi_2W_4O_{15}]_{0,30}$ $[Bi_{0,1}Mo_{12}Fe_{3,0}Co_{5,5}Si_{1,6}K_{0,08}O_x]_1$ | 2,45 | 59,4 | 462 ± 1 | 329 | 91,1 | 96,6 |
| B9 | $[Bi_2W_4O_{15}]_{0,30}$ $[Bi_{0,1}Mo_{12}Fe_{3,0}Co_{5,5}Si_{1,6}K_{0,08}O_x]_1$ | 2,45 | 59,4 | 457 ± 1 | 317 | 91,2 | 96,2 |
| B10 | $[Bi_2W_4O_{15}]_{0,50}$ $[Bi_{0,3}Mo_{12}Fe_{3,0}Co_{5,5}Si_{1,6}K_{0,08}O_x]_1$ | 2,45 | 59,7 | 462 ± 1 | 328 | 91,2 | 95,8 |
| B11 | $[Bi_2W_4O_{15}]_{0,50}$ $[Bi_{0,6}Mo_{12}Fe_{3,0}Co_{5,5}Si_{1,6}K_{0,08}O_x]_1$ | 2,45 | 59,9 | 462 ± 1 | 322 | 89,5 | 94,5 |
| B12 | $[Bi_2W_4O_{15}]_{0,25}$ $[Bi_{0,6}Mo_{12}Fe_{3,0}Co_{7,0}Si_{1,6}K_{0,08}O_x]_1$ | 2,45 | 59,4 | 462 ± 1 | 305 | 91,5 | 93,9 |
| B13 | $[Bi_2W_4O_{15}]_{0,50}$ $[Bi_{0,6}Mo_{12}Fe_{3,0}Co_{7,0}Si_{1,6}K_{0,08}O_x]_1$ | 2,45 | 59,4 | 462 ± 1 | 307 | 92,1 | 94,3 |

Die Gesamtmenge der Vergleichsbeispiele V1 bis V16 zeigt augenfällig, dass es, trotz umfangreicher Variationen von Stöchiometrie, Partikeldurchmesser der Ausgangsmassen A1, A2 und Endkalzinationstemperatur, ohne Wismutdotierung des Anteils T des Multielement-I-Aktivmassenoxids nicht gelingt, eine Anfangsaktivität des Katalysatorbetts zu erzeugen, die ein $T^S \leq 329$ °C ermöglicht. Im Unterscheid dazu liegt $T^S$ im Fall aller Ausführungsbeispiele B1 bis B13 homogen bei Werten $\leq 329$ °C.

Ein Vergleich von V4 mit B5, B6, B7 und B8 weist aus, dass bei Gleichheit der ringförmigen Katalysatorformkörper in im Wesentlichen allen Herstellungsgesichtspunkten diejenigen (neben einer erhöhten Anfangsaktivität) eine erhöhte Anfangsselektivität $S^{AC}$ aufweisen, deren Anteil T eine erfindungsgemäße Wismutdotierung aufweist. Das gleiche gilt hinsichtlich $S^{AC+AA}$. Ein Vergleich von V1 mit B11 und B10 sowie von V5 mit B1 und B3 bestätigt die vorstehende Feststellung ebenso.

V15 im Vergleich mit B10 und B11 zeigt, dass, bei Gleichheit der ringförmigen Katalysatorformkörper in im Wesentlichen allen Herstellungsgesichtspunkten, eine überhöhte Wismutdotierung im Anteil T die Anfangsselektivität der Zielproduktbildung ($S^{AC}$, $S^{AC+AA}$) mindert.

Ein Vergleich von B12 und B13 mit V16 belegt die Notwendigkeit eines Beiseins des Anteils $[Bi_aZ^1_bO_x]_p$ zu Erreichung der erfindungsgemäßen Zielsetzung nachdrücklich. Der Vergleich des Beispiels B2 mit B1, des Beispiels B4 mit B3 und des Beispiels B8 mit B9 weist aus, dass eine begrenzte Erhöhung von $T_{Endkalzination}$ bei ansonsten im Wesentlichen identischen Herstellbedingungen $S^{AC+AA}$ auch spürbar erhöht, im Unterschied zur erfindungsgemäßen Bi-Dotierung jedoch ohne gleichzeitig auch die Anfangsaktivität zu fördern.

Ein Vergleich von B13 mit B11 zeigt, dass eine Erhöhung des Kobaltgehalts im Anteil T sich bei ansonsten im Wesentlichen unveränderten Herstellbedingungen positiv auf die Anfangsaktivität auswirkt und $S^{AC}$ relativ zu $S^{AA}$ (Selektivität der Acrylsäurebildung) fördert.

Auf US Provisional Patent Application 61/096553 eingereicht am 12.09.2008 wird hingewiesen. Im Hinblick auf die obengenannten Lehren sind zahlreiche Änderungen und Abweichungen von der vorliegenden Erfindung möglich.

Man kann deshalb davon ausgehen, dass die Erfindung im Rahmen der beigefügten Ansprüche, anders als hierin spezifisch beschrieben, ausgeführt werden kann.

**Patentansprüche**

1. Verfahren zur Herstellung von geometrischen Katalysatorformkörpern K, die als Aktivmasse ein Multielementoxid I der allgemeinen Stöchiometrie I,

$$[Bi_aZ^1{}_bO_x]_p[Bi_cMo_{12}Fe_dZ^2{}_eZ^3{}_fZ^4{}_gZ^5{}_hZ^6{}_iO_y]_1 \qquad (I),$$

mit

$Z^1$ = Wolfram oder Wolfram und Molybdän, mit der Maßgabe, dass wenigstens 10 mol-% der molaren Gesamtmenge an $Z^1$ Wolfram ist,

$Z^2$ = ein Element oder mehr als ein Element aus der Gruppe bestehend aus Nickel und Kobalt,

$Z^3$ = ein Element oder mehr als ein Element aus der Gruppe bestehend aus den Alkalimetallen, den Erdalkalimetallen und Thallium,

$Z^4$ = ein Element oder mehr als ein Element aus der Gruppe bestehend aus Zink, Phosphor, Arsen, Bor, Antimon, Zinn, Cer, Vanadium und Chrom,

$Z^5$ = ein Element oder mehr als ein Element aus der Gruppe bestehend aus Silizium, Aluminium, Titan, Wolfram und Zirkonium,

$Z^6$ = ein Element oder mehr als ein Element aus der Gruppe bestehend aus Kupfer, Silber, Gold, Yttrium, Lanthan und den Lanthaniden,

a = 0,1 bis 3,
b = 0,1 bis 10,
d = 0,01 bis 5,
e = 1 bis 10,
f = 0,01 bis 2,
g = 0 bis 5,
h = 0 bis 10,
i = 0 bis 1,
p = 0,05 bis 6, und
x, y = Zahlen, die durch die Wertigkeit und Häufigkeit der von Sauerstoff verschiedenen Elemente in I bestimmt werden,

enthalten, bei dem man

- ein feinteiliges Mischoxid $Bi_aZ^1{}_bO_x$ mit einem Partikeldurchmesser $d_{50}^{A1}$ als Ausgangsmasse A1 mit der Maßgabe vorbildet, dass $1~\mu m \leq d_{50}^{A1} \leq 100~\mu m$ erfüllt ist;
- unter Verwendung von Quellen der von Sauerstoff verschiedenen Elemente des Anteils T = $[Bi_cMo_{12}Fe_dZ^2{}_eZ^3{}_fZ^4{}_gZ^5{}_hZ^6{}_iO_y]_1$ des Multielementoxids I in wässrigem Medium mit der Maßgabe eine innige wässrige Mischung M erzeugt, dass

- jede der verwendeten Quellen im Verlauf der Herstellung der wässrigen Mischung M einen Zerteilungsgrad Q durchläuft, der **dadurch gekennzeichnet ist, dass** sein Durchmesser $d_{90}^{Q} \leq 5~\mu m$ beträgt, und
- die wässrige Mischung M die Elemente Bi, Mo, Fe, $Z^2$, $Z^3$, $Z^4$, $Z^5$ und $Z^6$ in der Stöchiometrie I*,

$$Bi_cMo_{12}Fe_dZ^2{}_eZ^3{}_fZ^4{}_gZ^5{}_hZ^6{}_i \qquad (I^*),$$

enthält;

- aus der wässrigen Mischung M durch Trocknen und Einstellen des Zerteilungsgrades $d_{90}^{A2}$ eine feinteilige Ausgangsmasse A2 mit einem Partikeldurchmesser $d_{90}^{A2}$ unter der Maßgabe erzeugt, dass $400~\mu m \geq d_{90}^{A2} \geq 10~\mu m$ erfüllt ist;

- Ausgangsmasse A1 und Ausgangsmasse A2, oder Ausgangsmasse A1, Ausgangsmasse A2 und feinteilige Formgebungshilfsmittel zu einer feinteiligen Ausgangsmasse A3 mit der Maßgabe miteinander vermischt, dass die Ausgangsmasse A3 die über die Ausgangsmassen A1 und A2 in die Ausgangsmasse A3 eingebrachten, von Sauerstoff verschiedenen, Elemente des Multielementoxids I in der Stöchiometrie I\*\*

$$[Bi_a Z^1{}_b]_p [Bi_c Mo_{12} Fe_d Z^2{}_e Z^3{}_f Z^4{}_g Z^5{}_h Z^6{}_i]_1 \qquad (I^{**}),$$

enthält,
- mit feinteiliger Ausgangsmasse A3 geometrische Formkörper V formt, und
- die Formkörper V bei erhöhter Temperatur unter Erhalt der geometrischen Katalysatorformkörper K thermisch behandelt,

**dadurch gekennzeichnet, dass** der stöchiometrische Koeffizient c die Bedingung $0 < c \leq 0,8$ erfüllt,

wobei die Partikeldurchmesser $d_{50}^{A1}$, $d_{90}^{Q}$ und $d_{90}^{A2}$ aus der mittels Laserbeugung nach ISO 13320 bei einem Dispergierdruck von 2 bar absolut bestimmten volumenbezogenen Partikeldurchmesserverteilung ermittelt werden.

2. Verfahren nach Anspruch 1, **dadurch gekennzeichnet, dass** $c \geq 0,001$ beträgt.

3. Verfahren nach Anspruch 1, **dadurch gekennzeichnet, dass** $c \geq 0,002$ beträgt.

4. Verfahren nach Anspruch 1, **dadurch gekennzeichnet, dass** $c \geq 0,005$ beträgt.

5. Verfahren nach einem der Ansprüche 1 bis 4, **dadurch gekennzeichnet, dass** $c \leq 0,7$ beträgt.

6. Verfahren nach einem der Ansprüche 1 bis 4, **dadurch gekennzeichnet, dass** $c \leq 0,6$ beträgt.

7. Verfahren nach Anspruch 1, **dadurch gekennzeichnet, dass** $0,007 \leq c \leq 0,5$ beträgt.

8. Verfahren nach Anspruch 1, **dadurch gekennzeichnet, dass** $0,01 \leq c \leq 0,4$ beträgt.

9. Verfahren nach Anspruch 1, **dadurch gekennzeichnet, dass** $0,02 \leq c \leq 0,3$ beträgt.

10. Verfahren nach Anspruch 1, **dadurch gekennzeichnet, dass** $0,03 \leq c \leq 0,2$ beträgt.

11. Verfahren nach Anspruch 1, **dadurch gekennzeichnet, dass** $0,04 \leq c \leq 0,1$ beträgt.

12. Verfahren nach Anspruch 1, **dadurch gekennzeichnet, dass** $0,005 \leq c \leq 0,08$ beträgt.

13. Verfahren nach Anspruch 1, **dadurch gekennzeichnet, dass** $0,01 \leq c \leq 0,06$ beträgt.

14. Verfahren nach einem der Ansprüche 1 bis 13, **dadurch gekennzeichnet, dass** p 0,1 bis 4 beträgt.

15. Verfahren nach einem der Ansprüche 1 bis 13, **dadurch gekennzeichnet, dass** p 0,2 bis 3 beträgt.

16. Verfahren nach einem der Ansprüche 1 bis 13, **dadurch gekennzeichnet, dass** p 0,3 bis 1 beträgt.

17. Verfahren nach einem der Ansprüche 1 bis 16, **dadurch gekennzeichnet, dass** das Verhältnis $n_{Bi} : n_{Mo}$ der im Multielementoxid I insgesamt enthaltenen molaren Menge an Bi, $n_{Bi}$, zur im Multielementoxid I insgesamt enthaltenen molaren Menge an Mo, $n_{Mo}$, 0,3 bis 2 zu 12 beträgt.

18. Verfahren nach Anspruch 17, **dadurch gekennzeichnet, dass** $n_{Bi} : n_{Mo}$ 0,4 bis 1,8 zu 12 beträgt.

19. Verfahren nach Anspruch 17, **dadurch gekennzeichnet, dass** $n_{Bi} : n_{Mo}$ 0,5 bis 1,6 zu 12 beträgt.

20. Verfahren nach einem der Ansprüche 1 bis 19, **dadurch gekennzeichnet, dass** $1 \ \mu m \leq d_{50}^{A1} \leq 50 \ \mu m$ beträgt.

21.

Verfahren nach einem der Ansprüche 1 bis 19, **dadurch gekennzeichnet, dass** $1\ \mu m \leq d_{50}^{A1} \leq 10\ \mu m$ beträgt.

22. Verfahren nach einem der Ansprüche 1 bis 19, **dadurch gekennzeichnet, dass** $1,5\ \mu m \leq d_{50}^{A1} \leq 6\ \mu m$ beträgt.

23. Verfahren nach einem der Ansprüche 1 bis 19, **dadurch gekennzeichnet, dass** $2\ \mu m \leq d_{50}^{A1} \leq 3\ \mu m$ beträgt.

24. Verfahren nach einem der Ansprüche 1 bis 23, **dadurch gekennzeichnet, dass** $200\ \mu m \geq d_{90}^{A2} \geq 20\ \mu m$ beträgt.

25. Verfahren nach einem der Ansprüche 1 bis 23, **dadurch gekennzeichnet, dass** $150\ \mu m \geq d_{90}^{A2} \geq 40\ \mu m$ beträgt.

26. Verfahren nach einem der Ansprüche 1 bis 23, **dadurch gekennzeichnet, dass** $100\ \mu m \geq d_{90}^{A2} \geq 50\ \mu m$ beträgt.

27. Verfahren nach einem der Ansprüche 1 bis 26, **dadurch gekennzeichnet, dass** der Partikeldurchmesser $d_{50}^{A2}$ der feinteiligen Ausgangsmasse A2 die Bedingung $10\ \mu m \leq d_{50}^{A2} \leq 100\ \mu m$ erfüllt.

28. Verfahren nach einem der Ansprüche 1 bis 26, **dadurch gekennzeichnet, dass** der Partikeldurchmesser $d_{50}^{A2}$ der feinteiligen Ausgangsmasse A2 die Bedingung $20\ \mu m \leq d_{50}^{A2} \leq 60\ \mu m$ erfüllt.

29. Verfahren nach einem der Ansprüche 1 bis 28, **dadurch gekennzeichnet, dass** das Verhältnis des Partikeldurchmessers $d_{90}^{A2}$ der feinteiligen Ausgangsmasse A2 zum Partikeldurchmesser $d_{10}^{A2}$ der feinteiligen Ausgangsmasse A2 im Bereich von 5 bis 20 liegt.

30. Verfahren nach einem der Ansprüche 1 bis 29, **dadurch gekennzeichnet, dass** $d_{90}^{Q}$ für jede der verwendeten Quellen $\leq 3\ \mu m$ ist.

31. Verfahren nach einem der Ansprüche 1 bis 29, **dadurch gekennzeichnet, dass** $d_{90}^{Q}$ für jede der verwendeten Quellen $\leq 1\ \mu m$ ist.

32. Verfahren nach einem der Ansprüche 1 bis 29, **dadurch gekennzeichnet, dass** $d_{90}^{Q}$ für jede der verwendeten Quellen $\leq 0,5\ \mu m$ ist.

33. Verfahren nach einem der Ansprüche 1 bis 32, **dadurch gekennzeichnet, dass** jede der verwendeten Quellen im Verlauf der Herstellung der wässrigen Mischung M den Zustand einer kolloidalen oder einer echten Lösung durchläuft.

34. Verfahren nach einem der Ansprüche 1 bis 32, **dadurch gekennzeichnet, dass** der Anteil T das Element Si enthält und jede der verwendeten Quellen der von Silizium verschiedenen Elemente im Verlauf der Herstellung der wässrigen Mischung M den Zustand einer echten Lösung durchläuft und als Quelle des Elements Si ein Kieselsol verwendet wird.

35. Verfahren nach einem der Ansprüche 1 bis 34, **dadurch gekennzeichnet, dass** die feinteilige Ausgangsmasse A2 durch Sprühtrocknung der wässrigen Mischung M erzeugt wird.

36. Verfahren nach einem der Ansprüche 1 bis 35, **dadurch gekennzeichnet, dass** $Z^2$ = Co ist.

37. Verfahren nach einem der Ansprüche 1 bis 36, **dadurch gekennzeichnet, dass** $Z^3$ = K, Cs und/oder Sr ist.

38. Verfahren nach einem der Ansprüche 1 bis 37, **dadurch gekennzeichnet, dass** $Z^5$ = Si ist.

39. Verfahren nach einem der Ansprüche 1 bis 38, **dadurch gekennzeichnet, dass** a 0,5 bis 3 ist.

**40.** Verfahren nach einem der Ansprüche 1 bis 38, **dadurch gekennzeichnet, dass** a 1,5 bis 2,5 ist.

**41.** Verfahren nach einem der Ansprüche 1 bis 40, **dadurch gekennzeichnet, dass** b 2 bis 5 ist.

**42.** Verfahren nach einem der Ansprüche 1 bis 40, **dadurch gekennzeichnet, dass** b 3 bis 5 ist.

**43.** Verfahren nach einem der Ansprüche 1 bis 42, **dadurch gekennzeichnet, dass** wenigstens 20 mol-% der molaren Gesamtmenge an $Z^1$ Wolfram ist.

**44.** Verfahren nach einem der Ansprüche 1 bis 42, **dadurch gekennzeichnet, dass** wenigstens 80 mol-% der molaren Gesamtmenge an $Z^1$ Wolfram ist.

**45.** Verfahren nach einem der Ansprüche 1 bis 42, **dadurch gekennzeichnet, dass** die molare Gesamtmenge an $Z^1$ Wolfram ist.

**46.** Verfahren nach einem der Ansprüche 1 bis 45, **dadurch gekennzeichnet, dass** e 3 bis 8 ist.

**47.** Verfahren nach einem der Ansprüche 1 bis 45, **dadurch gekennzeichnet, dass** e 4 bis 7 ist.

**48.** Verfahren nach einem der Ansprüche 1 bis 47, **dadurch gekennzeichnet, dass** f 0,03 bis 1 ist.

**49.** Verfahren nach einem der Ansprüche 1 bis 48, **dadurch gekennzeichnet, dass** d 1 bis 4 ist.

**50.** Verfahren nach einem der Ansprüche 1 bis 49, **dadurch gekennzeichnet, dass** h 0,1 bis 8 ist.

**51.** Verfahren nach einem der Ansprüche 1 bis 49, **dadurch gekennzeichnet, dass** h 0,5 bis 3 ist.

**52.** Verfahren nach einem der Ansprüche 1 bis 51, **dadurch gekennzeichnet, dass** das feinteilige Mischoxid $Bi_aZ^1{}_bO_x$ das Mischoxid $Bi_2W_4O_{15}$ ist.

**53.** Verfahren nach einem der Ansprüche 1 bis 52, **dadurch gekennzeichnet, dass** der Betrag F des Produktes

$$(d_{50}^{A1})^{0,7} \quad \bullet \quad (d_{90}^{A2})^{1,5} \quad \bullet \quad (p/2)^{-1}$$

$\geq 820$ beträgt, wobei die beiden Partikeldurchmesser $d_{50}^{A1}$ und $d_{90}^{A2}$ in der Längeneinheit $\mu$m anzugeben sind.

**54.** Verfahren nach Anspruch 53, **dadurch gekennzeichnet, dass** $1000 \leq F \leq 5000$ ist.

**55.** Verfahren nach Anspruch 53, **dadurch gekennzeichnet, dass** $2000 \leq F \leq 4000$ ist.

**56.** Verfahren nach einem der Ansprüche 1 bis 55, **dadurch gekennzeichnet, dass** der Betrag F* des Produktes

$$(d_{50}^{A1})^{0,7} \quad \bullet \quad (d_{50}^{A2})^{0,7} \quad \bullet \quad (p/2)^{-1}$$

$\geq 15$ beträgt, wobei die beiden Partikeldurchmesser $d_{50}^{A1}$ und $d_{50}^{A2}$ in der Längeneinheit $\mu$m anzugeben sind.

**57.** Verfahren nach Anspruch 56, **dadurch gekennzeichnet, dass** $35 \geq F* \geq 18$ ist.

**58.** Verfahren nach einem der Ansprüche 1 bis 57, **dadurch gekennzeichnet, dass** feinteilige Ausgangsmasse A1, feinteilige Ausgangsmasse A2 und feinteilige hydrophobisierte Kieselsäure umfassende Formgebungshilfsmittel zu der feinteiligen Ausgangsmasse A3 miteinander vermischt werden.

**59.** Verfahren nach einem der Ansprüche 1 bis 58, **dadurch gekennzeichnet, dass** feinteilige Ausgangsmasse A1, feinteilige Ausgangsmasse A2 und feinteiligen Graphit umfassende Formgebungshilfsmittel zu der feinteiligen Ausgangsmasse A3 miteinander vermischt werden.

60. Verfahren nach einem der Ansprüche 1 bis 59, **dadurch gekennzeichnet, dass** die Formung von geometrischen Formkörpern V mit feinteiliger Ausgangsmasse A3 durch Verdichten der feinteiligen Ausgangsmasse A3 erfolgt.

61. Verfahren nach Anspruch 60, **dadurch gekennzeichnet, dass** das Verdichten durch Strangpressen, Extrudieren oder Tablettieren erfolgt.

62. Verfahren nach einem der Ansprüche 1 bis 61, **dadurch gekennzeichnet, dass** der geometrische Formkörper V ein Ring ist.

63. Verfahren nach Anspruch 62, **dadurch gekennzeichnet, dass** die Seitendruckfestigkeit SD des ringförmigen Formkörpers V die Bedingung 12 N ≤ SD ≤ 25 N erfüllt.

64. Verfahren nach einem der Ansprüche 1 bis 61, **dadurch gekennzeichnet, dass** der geometrische Formkörper V eine Kugel ist.

65. Verfahren nach einem der Ansprüche 1 bis 61, **dadurch gekennzeichnet, dass** der geometrische Formkörper V ein Vollzylinder ist.

66. Verfahren nach Anspruch 62 oder 63, **dadurch gekennzeichnet, dass** der Außendurchmesser = 2 bis 10 mm, die Höhe = 2 bis 10 mm und die Wandstärke des Rings 1 bis 3 mm betragen.

67. Verfahren nach Anspruch 64, **dadurch gekennzeichnet, dass** der Kugeldurchmesser 2 bis 10 mm beträgt.

68. Verfahren nach Anspruch 65, **dadurch gekennzeichnet, dass** der Außendurchmesser = 1 bis 10 mm und die Höhe des Vollzylinders 2 bis 10 mm betragen.

69. Verfahren nach einem der Ansprüche 1 bis 59, **dadurch gekennzeichnet, dass** die Formung von geometrischen Formkörpern V mit feinteiliger Ausgangsmasse A3 dadurch erfolgt, dass man die feinteilige Ausgangsmasse A3 mit Hilfe eines flüssigen Bindemittels auf die Oberfläche eines geometrischen Trägerformkörpers aufbringt.

70. Verfahren nach einem der Ansprüche 1 bis 69, **dadurch gekennzeichnet, dass** im Rahmen der thermischen Behandlung der Formkörper V die Temperatur 350 °C überschritten und die Temperatur 600 °C nicht überschritten wird.

71. Verfahren nach einem der Ansprüche 1 bis 69, **dadurch gekennzeichnet, dass** im Rahmen der thermischen Behandlung der Formkörper V die Temperatur 420 °C überschritten und die Temperatur 500 °C nicht überschritten wird.

72. Verfahren nach einem der Ansprüche 1 bis 71, **dadurch gekennzeichnet, dass** die thermische Behandlung im Beisein von Luft erfolgt.

73. Verfahren nach einem der Ansprüche 1 bis 68 oder nach einem der Ansprüche 70 bis 72, **dadurch gekennzeichnet, dass** der Katalysatorformkörper K ein Vollkatalysatorformkörper K ist und sich an das Verfahren seiner Herstellung ein Verfahren der Mahlung zu feinteiligem Material anschließt und von dem feinteiligen Material mit Hilfe eines flüssigen Bindemittels auf die Oberfläche eines geometrischen Trägerformkörpers aufgebracht wird.

74. Katalysatorformkörper erhältlich nach einem Verfahren gemäß einem der Ansprüche 1 bis 73.

75. Katalysator erhältlich durch Mahlen eines Katalysatorformkörpers der ein Vollkatalysatorformkörper und nach einem Verfahren gemäß einem der Ansprüche 1 bis 68 erhältlich ist.

76. Verfahren der heterogen katalysierten partiellen Gasphasenoxidation eines 3 bis 6 C-Atome enthaltenden Alkans, Alkanols, Alkanals, Alkens und/oder Alkenals an einem Katalysatorbett, **dadurch gekennzeichnet, dass** das Katalysatorbett einen Katalysatorformkörper gemäß Anspruch 74 oder einen Katalysator gemäß Anspruch 75 umfasst.

77. Verfahren nach Anspruch 76, **dadurch gekennzeichnet, dass** es ein Verfahren der heterogen katalysierten partiellen Gasphasenoxidation von Propen zu Acrolein ist.

**78.** Verfahren nach Anspruch 76 **dadurch gekennzeichnet, dass** es ein Verfahren der heterogen katalysierten partiellen Gasphasenoxidation von iso-Buten zu Methacrolein ist.

**79.** Verfahren nach Anspruch 76, **dadurch gekennzeichnet, dass** es ein Verfahren der Ammoxidation von Propen zu Acrylnitril oder ein Verfahren der Ammoxidation von iso-Buten zu Methacrylnitril ist.

**80.** Verwendung eines Katalysatorformkörpers gemäß Anspruch 74 oder eines Katalysators gemäß Anspruch 75 als Katalysator in einem Verfahren der heterogen katalysierten partiellen Gasphasenoxidation eines 3 bis 6 C-Atome enthaltenden Alkans, Alkanols, Alkanals, Alkens und/oder Alkenals.

**81.** Verwendung nach Anspruch 80, **dadurch gekennzeichnet, dass** sie in einem Verfahren der heterogen katalysierten partiellen Gasphasenoxidation von Propen zu Acrolein, von iso-Buten zu Methacrolein, oder in einem Verfahren der Ammoxidation von Propen zu Acrylnitril oder von iso-Buten zu Methacrylnitril erfolgt.


**Claims**

**1.** A process for producing geometric shaped catalyst bodies K which comprise, as an active material, a multielement oxide I of the general stoichiometry I

$$[Bi_aZ^1_bO_x]_p[Bi_cMo_{12}Fe_dZ^2_eZ^3_fZ^4_gZ^5_hZ^6_iO_y]_1 \qquad (I),$$

where

$Z^1$ = tungsten or tungsten and molybdenum, with the proviso that at least 10 mol% of the molar total amount of $Z^1$ is tungsten,
$Z^2$ = one element or more than one element from the group consisting of nickel and cobalt,
$Z^3$ = one element or more than one element from the group consisting of the alkali metals, the alkaline earth metals and thallium,
$Z^4$ = one element or more than one element from the group consisting of zinc, phosphorus, arsenic, boron, antimony, tin, cerium, vanadium and chromium,
$Z^5$ = one element or more than one element from the group consisting of silicon, aluminum, titanium, tungsten and zirconium,
$Z^6$ = one element or more than one element from the group consisting of copper, silver, gold, yttrium, lanthanum and the lanthanides,
a = 0.1 to 3,
b = 0.1 to 10,
d = 0.01 to 5,
e = 1 to 10,
f = 0.01 to 2,
g = 0 to 5,
h = 0 to 10,
i = 0 to 1,
p = 0.05 to 6, and
x, y = numbers determined by the valency and frequency of the elements in I other than oxygen,

in which

- a finely divided mixed oxide $Bi_aZ^1_bO_x$ with a particle diameter $d_{50}^{A1}$, as starting material A1, is preformed with the proviso that $1 \ \mu m \ \leq \ d_{50}^{A1} \ \leq 100 \ \mu m$;
- sources of the elements other than oxygen in the component T = $[Bi_cMo_{12}Fe_dZ^2_eZ^3_fZ^4_gZ^5_hZ^6_iO_y]_1$ of the multielement oxide I are used in an aqueous medium to obtain an intimate aqueous mixture M, with the proviso that

- each of the sources used, in the course of preparation of the aqueous mixture M, passes through a degree of division Q for which its diameter $d_{90}^{Q}$ is $\leq 5 \, \mu m$,

and

- the aqueous mixture M comprises the elements Bi, Mo, Fe, $Z^2$, $Z^3$, $Z^4$, $Z^5$ and $Z^6$ in the stoichiometry I*

$$Bi_c Mo_{12} Fe_d Z^2_e Z^3_f Z^4_g Z^5_h Z^6_i \qquad (I^*);$$

- the aqueous mixture M, by means of drying and adjusting the degree of division $d_{90}^{A2}$, is used to obtain a finely divided starting material A2 with a particle diameter $d_{90}^{A2}$, with the proviso that

$$400 \; \mu m \geq d_{90}^{A2} \geq 10 \; \mu m;$$

- starting material A1 and starting material A2, or starting material A1, starting material A2 and finely divided shaping assistant, are mixed with one another to form a finely divided starting material A3, with the proviso that the starting material A3 comprises the elements other than oxygen introduced into the starting material A3 via starting materials A1 and A2 in the multielement oxide I in the stoichiometry I**

$$[Bi_a Z^1_b]_p [Bi_c Mo_{12} Fe_d Z^2_e Z^3_f Z^4_g Z^5_h Z^6_i]_1 \qquad (I^{**}),$$

- finely divided starting material A3 is used to form geometric shaped bodies V, and
- the shaped bodies V are treated thermally at elevated temperature to obtain the geometric shaped catalyst bodies K,

wherein the stoichiometric coefficient c satisfies the condition $0 < c \leq 0.8$,

wherein the particle diameters $d_{50}^{A1}$, $d_{90}^{Q}$ and $d_{90}^{A2}$ are ascertained from the volume-based particle diameter distribution determined by means of laser diffraction according to ISO 13320 at a dispersion pressure of 2 bar absolute.

2. The process according to claim 1, wherein c is $\geq 0.001$.

3. The process according to claim 1, wherein c is $\geq 0.002$.

4. The process according to claim 1, wherein c is $\geq 0.005$.

5. The process according to any one of claims 1 to 4, wherein c is $\leq 0.7$.

6. The process according to any one of claims 1 to 4, wherein c is $\leq 0.6$.

7. The process according to claim 1, wherein $0.007 \leq c \leq 0.5$.

8. The process according to claim 1, wherein $0.01 \leq c \leq 0.4$.

9. The process according to claim 1, wherein $0.02 \leq c \leq 0.3$.

10. The process according to claim 1, wherein $0.03 \leq c \leq 0.2$.

11. The process according to claim 1, wherein $0.04 \leq c \leq 0.1$.

12. The process according to claim 1, wherein $0.005 \leq c \leq 0.08$.

13. The process according to claim 1, wherein $0.01 \leq c \leq 0.06$.

14. The process according to any one of claims 1 to 13, wherein p is 0.1 to 4.

15. The process according to any one of claims 1 to 13, wherein p is 0.2 to 3.

16. The process according to any one of claims 1 to 13, wherein p is 0.3 to 1.

**17.** The process according to any one of claims 1 to 16, wherein the $n_{Bi} : n_{Mo}$ ratio of the total molar amount of Bi present in the multielement oxide I, $n_{Bi}$, to the total molar amount of Mo present in the multielement oxide I, $n_{Mo}$, is 0.3 to 2:12.

**18.** The process according to claim 17, wherein $n_{Bi} : n_{Mo}$ is 0.4 to 1.8:12.

**19.** The process according to claim 17, wherein $n_{Bi} : n_{Mo}$ is 0.5 to 1.6:12.

**20.** The process according to any one of claims 1 to 19, wherein $1 \ \mu m \ \leq \ d_{50}^{A1} \ \leq \ 50 \ \mu m$.

**21.** The process according to any one of claims 1 to 19, wherein $1 \ \mu m \ \leq \ d_{50}^{A1} \ \leq \ 10 \ \mu m.$

**22.** The process according to any one of claims 1 to 19, wherein $1.5 \ \mu m \ \leq \ d_{50}^{A1} \ \leq \ 6 \ \mu m.$

**23.** The process according to any one of claims 1 to 19, wherein $2 \ \mu m \ \leq \ d_{50}^{A1} \ \leq \ 3 \ \mu m.$

**24.** The process according to any one of claims 1 to 23, wherein $200 \ \mu m \ \geq \ d_{90}^{A2} \ \geq \ 20 \ \mu m.$

**25.** The process according to any one of claims 1 to 23, wherein $150 \ \mu m \ \geq \ d_{90}^{A2} \ \geq \ 40 \ \mu m.$

**26.** The process according to any one of claims 1 to 23, wherein $100 \ \mu m \ \geq \ d_{90}^{A2} \ \geq \ 50 \ \mu m.$

**27.** The process according to any one of claims 1 to 26, wherein the particle diameter $d_{50}^{A2}$ of the finely divided starting material A2 satisfies the condition $10 \ \mu m \ \leq \ d_{50}^{A2} \ \leq \ 100 \ \mu m.$

**28.** The process according to any one of claims 1 to 26, wherein the particle diameter $d_{50}^{A2}$ of the finely divided starting material A2 satisfies the condition $20 \ \mu m \ \leq \ d_{50}^{A2} \ \leq \ 60 \ \mu m.$

**29.** The process according to any one of claims 1 to 28, wherein the ratio of the particle diameter $d_{90}^{A2}$ of the finely divided starting material A2 to the particle diameter $d_{10}^{A2}$ of the finely divided starting material A2 is in the range from 5 to 20.

**30.** The process according to any one of claims 1 to 29, wherein $d_{90}^{Q}$ for each of the sources used is $\leq 3 \ \mu m$.

**31.** The process according to any one of claims 1 to 29, wherein $d_{90}^{Q}$ for each of the sources used is $\leq 1 \ \mu m$.

**32.** The process according to any one of claims 1 to 29, wherein $d_{90}^{Q}$ for each of the sources used is $\leq 0.5 \ \mu m$.

**33.** The process according to any one of claims 1 to 32, wherein each of the sources used, in the course of preparation of the aqueous mixture M, passes through the state of a colloidal solution or of a true solution.

**34.** The process according to any one of claims 1 to 32, wherein the component T comprises the element Si and each of the sources of the elements other than silicon used, in the course of preparation of the aqueous mixture M, passes through the state of a true solution and the source of the element Si used is a silica sol.

**35.** The process according to any one of claims 1 to 34, wherein the finely divided starting material A2 is obtained by spray-drying the aqueous mixture M.

**36.** The process according to any one of claims 1 to 35, wherein $Z^2$ = Co.

**37.** The process according to any one of claims 1 to 36, wherein $Z^3$ = K, Cs and/or Sr.

**38.** The process according to any one of claims 1 to 37, wherein $Z^5$ = Si.

**39.** The process according to any one of claims 1 to 38, wherein a is from 0.5 to 3.

**40.** The process according to any one of claims 1 to 38, wherein a is from 1.5 to 2.5.

**41.** The process according to any one of claims 1 to 40, wherein b is from 2 to 5.

**42.** The process according to any one of claims 1 to 40, wherein b is from 3 to 5.

**43.** The process according to any one of claims 1 to 42, wherein at least 20 mol% of the molar total amount of $Z^1$ is tungsten.

**44.** The process according to any one of claims 1 to 42, wherein at least 80 mol% of the molar total amount of $Z^1$ is tungsten.

**45.** The process according to any one of claims 1 to 42, wherein the molar total amount of $Z^1$ is tungsten.

**46.** The process according to any one of claims 1 to 45, wherein e is from 3 to 8.

**47.** The process according to any one of claims 1 to 45, wherein e is from 4 to 7.

**48.** The process according to any one of claims 1 to 47, wherein f is from 0.03 to 1.

**49.** The process according to any one of claims 1 to 48, wherein d is from 1 to 4.

**50.** The process according to any one of claims 1 to 49, wherein h is from 0.1 to 8.

**51.** The process according to any one of claims 1 to 49, wherein h is from 0.5 to 3.

**52.** The process according to any one of claims 1 to 51, wherein the finely divided mixed oxide $Bi_a Z^1_b O_x$ is the mixed oxide $Bi_2 W_4 O_{15}$.

**53.** The process according to any one of claims 1 to 52, wherein the value F of the product

$$(d_{50}^{A1})^{0.7} \bullet (d_{90}^{A2})^{1.5} \bullet (p/2)^{-1}$$

is $\geq 820$, where the two particle diameters $d_{50}^{A1}$ and $d_{90}^{A2}$ should be reported in the length unit $\mu$m.

**54.** The process according to claim 53, wherein $1000 \leq F \leq 5000$.

**55.** The process according to claim 53, wherein $2000 \leq F \leq 4000$.

**56.** The process according to any one of claims 1 to 55, wherein the magnitude F* of the product

$$(d_{50}^{A1})^{0.7} \bullet (d_{50}^{A2})^{0.7} \bullet (p/2)^{-1}$$

is $\geq 15$, where the two particle diameters $d_{50}^{A1}$ and $d_{50}^{A2}$ should be reported in the length unit $\mu$m.

**57.** The process according to claim 56, wherein $35 \geq F^* \geq 18$.

**58.** The process according to any one of claims 1 to 57, wherein finely divided starting material A1, finely divided starting material A2 and shaping assistant comprising finely divided hydrophobized silica are mixed with one another to give the finely divided starting material A3.

**59.** The process according to any one of claims 1 to 58, wherein finely divided starting material A1, finely divided starting material A2 and shaping assistant comprising finely divided graphite are mixed with one another to give the finely divided starting material A3.

60. The process according to any one of claims 1 to 59, wherein geometric shaped bodies V are formed with finely divided starting material A3 by compacting the finely divided starting material A3.

61. The process according to claim 60, wherein the compaction is effected by extrusion or tableting.

62. The process according to any one of claims 1 to 61, wherein the geometric shaped body V is a ring.

63. The process according to claim 62, wherein the side crushing strength SCS of the annular shaped body V satisfies the condition 12 N ≤ SCS ≤ 25 N.

64. The process according to any one of claims 1 to 61, wherein the geometric shaped body V is a sphere.

65. The process according to any one of claims 1 to 61, wherein the geometric shaped body V is a solid cylinder.

66. The process according to claim 62 or 63, wherein the external diameter = from 2 to 10 mm, the height = from 2 to 10 mm and the wall thickness of the ring is from 1 to 3 mm.

67. The process according to claim 64, wherein the sphere diameter is from 2 to 10 mm.

68. The process according to claim 65, wherein the external diameter = from 1 to 10 mm and the height of the solid cylinder is from 2 to 10 mm.

69. The process according to any one of claims 1 to 59, wherein geometric shaped bodies V are formed with finely divided starting material A3 by applying the finely divided starting material A3 to the surface of a geometric shaped support body with the aid of a liquid binder.

70. The process according to any one of claims 1 to 69, wherein the temperature of 350°C is exceeded and the temperature of 600°C is not exceeded in the course of thermal treatment of the shaped bodies V.

71. The process according to any one of claims 1 to 69, wherein the temperature of 420°C is exceeded and the temperature of 500°C is not exceeded in the course of thermal treatment of the shaped bodies V.

72. The process according to any one of claims 1 to 71, wherein the thermal treatment is effected in the presence of air.

73. The process according to any one of claims 1 to 68 or according to any one of claims 70 to 72, wherein the shaped catalyst body K is a shaped unsupported catalyst body K and the process for preparing it is followed by a process for grinding to give finely divided material and the finely divided material is applied to the surface of a geometric shaped support body with the aid of a liquid binder.

74. A shaped catalyst body obtainable by a process according to any one of claims 1 to 73.

75. A catalyst obtainable by grinding a shaped catalyst body which is a shaped unsupported catalyst body and is obtainable by a process according to any one of claims 1 to 68.

76. A process for heterogeneously catalyzed partial gas phase oxidation of an alkane, alkanol, alkanal, alkene and/or alkenal which comprises from 3 to 6 carbon atoms over a catalyst bed, wherein said catalyst bed comprises a shaped catalyst body according to claim 74 or a catalyst according to claim 75.

77. The process according to claim 76, which is a process for heterogeneously catalyzed partial gas phase oxidation of propene to acrolein.

78. The process according to claim 76, which is a process for heterogeneously catalyzed partial gas phase oxidation of isobutene to methacrolein.

79. The process according to claim 76, which is a process for ammoxidation of propene to acrylonitrile or a process for ammoxidation of isobutene to methacrylonitrile.

**80.** The use of a shaped catalyst body according to claim 74 or of a catalyst according to claim 75 as a catalyst in a process for heterogeneously catalyzed partial gas phase oxidation of an alkane, alkanol, alkanal, alkene and/or alkenal comprising from 3 to 6 carbon atoms.

**81.** The use according to claim 80 in a process for heterogeneously catalyzed partial gas phase oxidation of propene to acrolein, of isobutene to methacrolein, or in a process for ammoxidation of propene to acrylonitrile or of isobutene to methacrylonitrile.

**Revendications**

**1.** Procédé de fabrication de corps moulés catalytiques géométriques K, qui contiennent en tant que masse active un oxyde de plusieurs éléments I de la stœchiométrie générale I,

$$[Bi_aZ^1_bO_x]_p[Bi_cMo_{12}Fe_dZ^2_eZ^3_fZ^4_gZ^5_hZ^6_iO_y]_1 \qquad (I),$$

avec

$Z^1$ = le tungstène ou le tungstène et le molybdène, à condition qu'au moins 10 % en moles de la quantité molaire totale de $Z^1$ soit du tungstène,
$Z^2$ = un élément ou plus d'un élément du groupe constitué par le nickel et le cobalt,
$Z^3$ = un élément ou plus d'un élément du groupe constitué par les métaux alcalins, les métaux alcalino-terreux et le thallium,
$Z^4$ = un élément ou plus d'un élément du groupe constitué par le zinc, le phosphore, l'arsenic, le bore, l'antimoine, l'étain, le cérium, le vanadium et le chrome,
$Z^5$ = un élément ou plus d'un élément du groupe constitué par le silicium, l'aluminium, le titane, le tungstène et le zirconium,
$Z^6$ = un élément ou plus d'un élément du groupe constitué par le cuivre, l'argent, l'or, l'yttrium, le lanthane et les lanthanides,
a = 0,1 à 3,
b = 0,1 à 10,
d = 0,01 à 5,
e = 1 à 10,
f = 0,01 à 2,
g = 0 à 5,
h = 0 à 10,
i = 0 à 1,
p = 0,05 à 6, et
x, y = nombres qui sont déterminés par la valence et la fréquence des éléments différents de l'oxygène dans I,

selon lequel

- un oxyde mixte finement divisé $Bi_aZ^1_bO_x$ ayant un diamètre de particule $d_{50}^{A1}$ est préformé en tant que masse initiale A1, à condition que $1~\mu m \leq d_{50}^{A1} \leq 100~\mu m$ soit satisfait ;

- en utilisant des sources des éléments différents de l'oxygène de la fraction $T = [Bi_cMo_{12}Fe_dZ^2_eZ^3_fZ^4_gZ^5_hZ^6_iO_y]_1$ de l'oxyde de plusieurs éléments I dans un milieu aqueux, un mélange aqueux intime M est généré, à condition que

- chacune des sources utilisées passe au cours de la fabrication du mélange aqueux M par un degré de fragmentation Q qui est **caractérisé en ce que** son diamètre $d_{90}^{Q}$ est $\leq 5~\mu m$, et

- le mélange aqueux M contienne les éléments Bi, $M_o$, $F_e$, $Z^2$, $Z^3$, $Z^4$, $Z^5$ et $Z^6$ en la stœchiométrie I*,

$$Bi_cMo_{12}Fe_dZ^2_eZ^3_fZ^4_gZ^5_hZ^6_i \qquad (I^*),$$

- à partir du mélange aqueux M, par séchage et ajustement du degré de fragmentation $d_{90}^{A2}$, une masse initiale finement divisée A2 ayant un diamètre de particule $d_{90}^{A2}$ est générée, à condition que $400 \ \mu m \geq d_{90}^{A2} \geq 10 \ \mu m$ soit satisfait ;

- la masse initiale A1 et la masse initiale A2, ou la masse initiale A1, la masse initiale A2 et des adjuvants de façonnage finement divisés sont mélangés les uns avec les autres en une masse initiale finement divisée A3, à condition que la masse initiale A3 contienne les éléments différents de l'oxygène de l'oxyde de plusieurs éléments I, introduits par les masses initiales A1 et A2 dans la masse initiale A3, en la stœchiométrie I**

$$[Bi_aZ^1_b]_p[Bi_cMo_{12}Fe_dZ^2_eZ^3_fZ^4_gZ^5_hZ^6_i]_1 \qquad (I^{**}),$$

- des corps moulés géométriques V sont façonnés avec la masse initiale finement divisée A3, et
- les corps moulés V sont traités thermiquement à température élevée pour obtenir les corps moulés catalytiques géométriques K,
**caractérisé en ce que** le coefficient stœchiométrique c satisfait la condition 0 < c ≤ 0,8,

les diamètres de particules $d_{50}^{A1}$, $d_{90}^{Q}$ et $d_{90}^{A2}$ étant calculés à partir de la distribution des diamètres de particules relative au volume déterminée à une pression de dispersion de 2 bar absolu par diffraction laser selon ISO 13320.

2. Procédé selon la revendication 1, **caractérisé en ce que** c ≥ 0,001.

3. Procédé selon la revendication 1, **caractérisé en ce que** c ≥ 0,002.

4. Procédé selon la revendication 1, **caractérisé en ce que** c ≥ 0,005.

5. Procédé selon l'une quelconque des revendications 1 à 4, **caractérisé en ce que** c ≤ 0,7.

6. Procédé selon l'une quelconque des revendications 1 à 4, **caractérisé en ce que** c ≤ 0,6.

7. Procédé selon la revendication 1, **caractérisé en ce que** 0,007 ≤ c ≤ 0,5.

8. Procédé selon la revendication 1, **caractérisé en ce que** 0,01 ≤ c ≤ 0,4.

9. Procédé selon la revendication 1, **caractérisé en ce que** 0,02 ≤ c ≤ 0,3.

10. Procédé selon la revendication 1, **caractérisé en ce que** 0,03 ≤ c ≤ 0,2.

11. Procédé selon la revendication 1, **caractérisé en ce que** 0,04 ≤ c ≤ 0,1.

12. Procédé selon la revendication 1, **caractérisé en ce que** 0,005 ≤ c ≤ 0,08.

13. Procédé selon la revendication 1, **caractérisé en ce que** 0,01 ≤ c ≤ 0,06.

14. Procédé selon l'une quelconque des revendications 1 à 13, **caractérisé en ce que** p vaut 0,1 à 4.

15. Procédé selon l'une quelconque des revendications 1 à 13, **caractérisé en ce que** p vaut 0,2 à 3.

16. Procédé selon l'une quelconque des revendications 1 à 13, **caractérisé en ce que** p vaut 0,3 à 1.

17. Procédé selon l'une quelconque des revendications 1 à 16, **caractérisé en ce que** le rapport $n_{Bi} : n_{Mo}$ entre la quantité molaire de Bi, $n_{Bi}$, contenue au total dans l'oxyde de plusieurs éléments I et la quantité molaire de Mo, $n_{Mo}$, contenue au total dans l'oxyde de plusieurs éléments I est de 0,3 à 2 sur 12.

18. Procédé selon la revendication 17, **caractérisé en ce que** $n_{Bi} : n_{Mo}$ vaut 0,4 à 1,8 sur 12.

19. Procédé selon la revendication 17, **caractérisé en ce que** $n_{Bi} : n_{Mo}$ vaut 0,5 à 1,6 sur 12.

**20.**

Procédé selon l'une quelconque des revendications 1 à 19, **caractérisé en ce que** $1 \ \mu m \le d_{50}^{A1} \le 50 \ \mu m$.

**21.** Procédé selon l'une quelconque des revendications 1 à 19, **caractérisé en ce que** $1 \ \mu m \le d_{50}^{A1} \le 10 \ \mu m$.

**22.** Procédé selon l'une quelconque des revendications 1 à 19, **caractérisé en ce que** $1,5 \ \mu m \le d_{50}^{A1} \le 6 \ \mu m$.

**23.** Procédé selon l'une quelconque des revendications 1 à 19, **caractérisé en ce que** $2 \ \mu m \le d_{50}^{A1} \le 3 \ \mu m$.

**24.** Procédé selon l'une quelconque des revendications 1 à 23, **caractérisé en ce que** $200 \ \mu m \ge d_{90}^{A2} \ge 20 \ \mu m$.

**25.** Procédé selon l'une quelconque des revendications 1 à 23, **caractérisé en ce que** $150 \ \mu m \ge d_{90}^{A2} \ge 40 \ \mu m$.

**26.** Procédé selon l'une quelconque des revendications 1 à 23, **caractérisé en ce que** $100 \ \mu m \ge d_{90}^{A2} \ge 50 \ \mu m$.

**27.** Procédé selon l'une quelconque des revendications 1 à 26, **caractérisé en ce que** le diamètre de particule $d_{50}^{A2}$ de la masse initiale finement divisée A2 satisfait la condition $10 \ \mu m \le d_{50}^{A2} \le 100 \ \mu m$.

**28.** Procédé selon l'une quelconque des revendications 1 à 26, **caractérisé en ce que** le diamètre de particule $d_{50}^{A2}$ de la masse initiale finement divisée A2 satisfait la condition $20 \ \mu m \le d_{50}^{A2} \le 60 \ \mu m$.

**29.** Procédé selon l'une quelconque des revendications 1 à 28, **caractérisé en ce que** le rapport entre le diamètre de particule $d_{90}^{A2}$ de la masse initiale finement divisée A2 et le diamètre de particule $d_{10}^{A2}$ de la masse initiale finement divisée A2 se situe dans la plage allant de 5 à 20.

**30.** Procédé selon l'une quelconque des revendications 1 à 29, **caractérisé en ce que** $d_{90}^{Q}$ pour chacune des sources utilisées est $\le 3 \ \mu m$.

**31.** Procédé selon l'une quelconque des revendications 1 à 29, **caractérisé en ce que** $d_{90}^{Q}$ pour chacune des sources utilisées est $\le 1 \ \mu m$.

**32.** Procédé selon l'une quelconque des revendications 1 à 29, **caractérisé en ce que** $d_{90}^{Q}$ pour chacune des sources utilisées est $\le 0,5 \ \mu m$.

**33.** Procédé selon l'une quelconque des revendications 1 à 32, **caractérisé en ce que** chacune des sources utilisées passe par l'état d'une solution colloïdale ou véritable au cours de la fabrication du mélange aqueux M.

**34.** Procédé selon l'une quelconque des revendications 1 à 32, **caractérisé en ce que** la fraction T contient l'élément Si et chacune des sources utilisées des éléments différents du silicium passe par l'état d'une solution véritable au cours de la fabrication du mélange aqueux M, et un sol de silice est utilisé en tant que source de l'élément Si.

**35.** Procédé selon l'une quelconque des revendications 1 à 34, **caractérisé en ce que** la masse initiale finement divisée A2 est générée par séchage par pulvérisation du mélange aqueux M.

**36.** Procédé selon l'une quelconque des revendications 1 à 35, **caractérisé en ce que** $Z^2$ = Co.

**37.** Procédé selon l'une quelconque des revendications 1 à 36, **caractérisé en ce que** $Z^3$ = K, Cs et/ou Sr.

**38.** Procédé selon l'une quelconque des revendications 1 à 37, **caractérisé en ce que** $Z^5$ = Si.

**39.** Procédé selon l'une quelconque des revendications 1 à 38, **caractérisé en ce que** a vaut 0,5 à 3.

**40.** Procédé selon l'une quelconque des revendications 1 à 38, **caractérisé en ce que** a vaut 1,5 à 2,5.

**41.** Procédé selon l'une quelconque des revendications 1 à 40, **caractérisé en ce que** b vaut 2 à 5.

**42.** Procédé selon l'une quelconque des revendications 1 à 40, **caractérisé en ce que** b vaut 3 à 5.

**43.** Procédé selon l'une quelconque des revendications 1 à 42, **caractérisé en ce qu'**au moins 20 % en moles de la quantité molaire totale de $Z^1$ est du tungstène.

**44.** Procédé selon l'une quelconque des revendications 1 à 42, **caractérisé en ce qu'**au moins 80 % en moles de la quantité molaire totale de $Z^1$ est du tungstène.

**45.** Procédé selon l'une quelconque des revendications 1 à 42, **caractérisé en ce que** la quantité molaire totale de $Z^1$ est du tungstène.

**46.** Procédé selon l'une quelconque des revendications 1 à 45, **caractérisé en ce que** e vaut 3 à 8.

**47.** Procédé selon l'une quelconque des revendications 1 à 45, **caractérisé en ce que** e vaut 4 à 7.

**48.** Procédé selon l'une quelconque des revendications 1 à 47, **caractérisé en ce que** f vaut 0,03 à 1.

**49.** Procédé selon l'une quelconque des revendications 1 à 48, **caractérisé en ce que** d vaut 1 à 4.

**50.** Procédé selon l'une quelconque des revendications 1 à 49, **caractérisé en ce que** h vaut 0,1 à 8.

**51.** Procédé selon l'une quelconque des revendications 1 à 49, **caractérisé en ce que** h vaut 0,5 à 3.

**52.** Procédé selon l'une quelconque des revendications 1 à 51, **caractérisé en ce que** l'oxyde mixte finement divisé $Bi_a Z^1_b O_x$ est l'oxyde mixte $Bi_2 W_4 O_{15}$.

**53.** Procédé selon l'une quelconque des revendications 1 à 52, **caractérisé en ce que** le montant F du produit

$$( d^{A1}_{50} )^{0,7} \qquad \bullet \qquad ( d^{A2}_{90} )^{1,5} \qquad \bullet \qquad (p/2)^{-1}$$

est $\geq$ 820, les deux diamètres de particules $d^{A1}_{50}$ et $d^{A2}_{90}$ étant donnés dans l'unité de longueur $\mu$m.

**54.** Procédé selon la revendication 53, **caractérisé en ce que** 1 000 $\leq$ F $\leq$ 5 000.

**55.** Procédé selon la revendication 53, **caractérisé en ce que** 2 000 $\leq$ F $\leq$ 4 000.

**56.** Procédé selon l'une quelconque des revendications 1 à 55, **caractérisé en ce que** le montant F* du produit

$$( d^{A1}_{50} )^{0,7} \qquad \bullet \qquad ( d^{A2}_{50} )^{0,7} \qquad \bullet \qquad (p/2)^{-1}$$

est $\geq$ 15, les deux diamètres de particules $d^{A1}_{50}$ et $d^{A2}_{50}$ étant donnés dans l'unité de longueur $\mu$m.

**57.** Procédé selon la revendication 56, **caractérisé en ce que** $35 \geq F^* \geq 18$.

**58.** Procédé selon l'une quelconque des revendications 1 à 57, **caractérisé en ce que** la masse initiale finement divisée A1, la masse initiale finement divisée A2 et des adjuvants de façonnage comprenant de la silice hydrophobée finement divisée sont mélangés les uns avec les autres en la masse initiale finement divisée A3.

**59.** Procédé selon l'une quelconque des revendications 1 à 58, **caractérisé en ce que** la masse initiale finement divisée A1, la masse initiale finement divisée A2 et des adjuvants de façonnage comprenant du graphite finement divisé sont mélangés les uns avec les autres en la masse initiale finement divisée A3.

**60.** Procédé selon l'une quelconque des revendications 1 à 59, **caractérisé en ce que** le façonnage de corps moulés géométriques V avec la masse initiale finement divisée A3 a lieu par compactage de la masse initiale finement divisée A3.

**61.** Procédé selon la revendication 60, **caractérisé en ce que** le compactage a lieu par filage à la presse, extrusion ou pastillage.

**62.** Procédé selon l'une quelconque des revendications 1 à 61, **caractérisé en ce que** le corps moulé géométrique V est un anneau.

**63.** Procédé selon la revendication 62, **caractérisé en ce que** la résistance à la pression latérale SD du corps moulé annulaire V satisfait la condition $12\,N \leq SD \leq 25\,N$.

**64.** Procédé selon l'une quelconque des revendications 1 à 61, **caractérisé en ce que** le corps moulé géométrique V est une sphère.

**65.** Procédé selon l'une quelconque des revendications 1 à 61, **caractérisé en ce que** le corps moulé géométrique V est un cylindre plein.

**66.** Procédé selon la revendication 62 ou 63, **caractérisé en ce que** le diamètre extérieur = 2 à 10 mm, la hauteur = 2 à 10 mm et l'épaisseur de paroi de l'anneau est de 1 à 3 mm.

**67.** Procédé selon la revendication 64, **caractérisé en ce que** le diamètre de sphère est de 2 à 10 mm.

**68.** Procédé selon la revendication 65, **caractérisé en ce que** le diamètre extérieur = 1 à 10 mm et la hauteur du cylindre plein est de 2 à 10 mm.

**69.** Procédé selon l'une quelconque des revendications 1 à 59, **caractérisé en ce que** le façonnage de corps moulés géométriques V avec la masse initiale finement divisée A3 a lieu par application de la masse initiale finement divisée A3 à l'aide d'un liant liquide sur la surface d'un corps moulé géométrique support.

**70.** Procédé selon l'une quelconque des revendications 1 à 69, **caractérisé en ce que**, dans le cadre du traitement thermique des corps moulés V, la température de 350 °C est dépassée et la température de 600 °C n'est pas dépassée.

**71.** Procédé selon l'une quelconque des revendications 1 à 69, **caractérisé en ce que**, dans le cadre du traitement thermique des corps moulés V, la température de 420 °C est dépassée et la température de 500 °C n'est pas dépassée.

**72.** Procédé selon l'une quelconque des revendications 1 à 71, **caractérisé en ce que** le traitement thermique a lieu en présence d'air.

**73.** Procédé selon l'une quelconque des revendications 1 à 68 ou selon l'une quelconque des revendications 70 à 72, **caractérisé en ce que** le corps moulé catalytique K est un corps moulé catalytique plein K et son procédé de fabrication est suivi par un procédé de broyage en un matériau finement divisé, et le matériau finement divisé est appliqué à l'aide d'un liant liquide sur la surface d'un corps moulé géométrique support.

**74.** Corps moulé catalytique pouvant être obtenu par un procédé selon l'une quelconque des revendications 1 à 73.

**75.** Catalyseur pouvant être obtenu par broyage d'un corps moulé catalytique qui est un corps moulé catalytique plein et peut être obtenu par un procédé selon l'une quelconque des revendications 1 à 68.

**76.** Procédé d'oxydation partielle en phase gazeuse sous catalyse hétérogène d'un alcane, alcanol, alcanal, alcène et/ou alcénal contenant 3 à 6 atomes C sur un lit catalytique, **caractérisé en ce que** le lit catalytique comprend un corps moulé catalytique selon la revendication 74 ou un catalyseur selon la revendication 75.

**77.** Procédé selon la revendication 76, **caractérisé en ce qu'**il s'agit d'un procédé d'oxydation partielle en phase gazeuse sous catalyse hétérogène de propène en acroléine.

**78.** Procédé selon la revendication 76, **caractérisé en ce qu'**il s'agit d'un procédé d'oxydation partielle en phase gazeuse sous catalyse hétérogène d'iso-butène en méthacroléine.

**79.** Procédé selon la revendication 76, **caractérisé en ce qu'**il s'agit d'un procédé d'ammoxydation de propène en acrylonitrile ou d'un procédé d'ammoxydation d'iso-butène en méthacrylonitrile.

**80.** Utilisation d'un corps moulé catalytique selon la revendication 74 ou d'un catalyseur selon la revendication 75 en tant que catalyseur dans un procédé d'oxydation partielle en phase gazeuse sous catalyse hétérogène d'un alcane, alcanol, alcanal, alcène et/ou alcénal contenant 3 à 6 atomes C.

**81.** Utilisation selon la revendication 80, **caractérisée en ce qu'**elle a lieu dans un procédé d'oxydation partielle en phase gazeuse sous catalyse hétérogène de propène en acroléine, d'iso-butène en méthacroléine, ou dans un procédé d'ammoxydation de propène en acrylonitrile ou d'iso-butène en méthacrylonitrile.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007003778
- EP 575897 A
- EP 1005908 A
- WO 2007017431 A
- WO 0224620 A
- WO 200542459 A
- WO 200547224 A
- WO 200549200 A
- WO 2005113127 A
- DE 102008040093
- DE 102008040094
- DE 102007005606 A
- DE 102007004961 A
- DE 19835247 A
- DE 10051419 A
- DE 10046672 A
- DE 10122027 A
- DE 3338380 A
- WO 2005030393 A
- DE 10325487 A
- US 20050131253 A
- WO 02062737 A
- EP 184790 A
- DE 10046957 A
- DE 2909671 A
- WO 0249757 A
- DE 4442346 A
- WO 2007082827 A
- WO 2005047224 A
- WO 2005042459 A
- EP 990636 A
- EP 1106598 A
- DE 10246119 A
- DE 10245585 A
- DE 4407020 A
- DE 10232748 A
- DE 4431957 A
- EP 700714 A
- EP 700893 A
- EP 468290 B
- DE 19910506 A
- DE 10313213 A
- DE 10313208 A
- DE 19948523 A
- DE 19948248 A
- DE 10313209 A
- WO 0053557 A
- WO 0053558 A
- WO 0136364 A
- DE 10337788 A
- US 61096553

**In der Beschreibung aufgeführte Nicht-Patentliteratur**

- **H. BRUNNER ; D. SCHUTTE.** *Chem. Ing. Techn.,* 1965, vol. 89, 437
- **W.A. RITSCHEL ; A. BAUER-BRANDL.** Handbuch der Entwicklung, Herstellung und Qualitätssicherung. 2002